# EUROPEAN PATENT APPLICATION

(11) **EP 4 394 270 A1**
(43) Date of publication of application: **03.07.2024**
(21) Application number: 22860902.0
(22) Date of filing: 16.05.2022
(51) Int. Cl.: F24F 6/00, F24F 6/12, F24F 6/16, A61L 9/01, F24F 7/007, F24F 8/133, F24F 8/24

(54) **SPACE PURIFICATION SYSTEM**

(30) Priority: 25.08.2021 JP 2021136973; 07.09.2021 JP 2021145172; 14.09.2021 JP 2021149062
(71) Applicant: Panasonic Intellectual Property Management Co., Ltd., Kadoma-shi, Osaka 571-0057 (JP)
(72) Inventor: HAYASHI, Tomohiro, Kadoma-shi, Osaka 571-0057 (JP); MIZUNO, Yuki, Kadoma-shi, Osaka 571-0057 (JP); YOSHIDA, Shinji, Kadoma-shi, Osaka 571-0057 (JP); FUKUMOTO, Masahide, Kadoma-shi, Osaka 571-0057 (JP)
(74) Representative: Grünecker Patent- und Rechtsanwälte PartG mbB
(86) International application number: PCT/JP2022/020377
(87) International publication number: WO 2023/026605

(57) **Abstract**

Space purification system (100) of the present disclosure includes: hypochlorous acid water supplier (36) that supplies hypochlorous acid water to mixing tank (92); water supplier (50) that supplies water to mixing tank (92); water level sensor (90) that detects a water level of mixing tank (92); air purifier (11) that micronizes and releases, into air, mixed water of the hypochlorous acid water and the water stored in mixing tank (92); and air purification control unit (41) that controls supply treatment and drainage treatment of the mixed water. Air purification control unit (41) (i) executes first control of causing hypochlorous acid water supplier (36) to perform supply of the hypochlorous acid water at predetermined time intervals, and second control of causing water supplier (50) to perform supply of water based on information regarding the water level of mixing tank (92) from water level sensor (90), and (ii) executes, as the control of the drainage treatment, third control of causing the mixing tank (92) to draini the mixed water stored in mixing tank (92) based on an integrated humidification amount in air purifier (11).

## Description

### TECHNICAL FIELD

The present disclosure relates to a space purification device that micronizes water, blows out suctioned air containing the micronized water, and releases the micronized water containing a purification component.

### BACKGROUND ART

Conventionally, as this type of space purification device, an air-conditioning system that disinfects a space by bringing air to be supplied indoors into contact with a gas-liquid contact member containing a purification component and releasing the air is known (see, for example, PTL 1).

In such conventional space purification device, generally, in addition to release of micronized water, water stored in the device (water containing a purification component) contains a part of the purification component along with micronization behavior, and the water and the purification component are vaporized and released into a space.

### Citation List

### Patent Literature

PTL 1: Unexamined Japanese Patent Publication No. 2009-133521

### SUMMARY OF THE INVENTION

With the conventional space purification system, however, in a situation where a humidification amount required for an indoor space is small, for example, in summer season in Japan (rainy season in particular), when air dehumidified by an air-conditioner or the like and having high relative humidity (e.g., 12 °C 95%) is ventilated, water (hypochlorous acid water) containing a micronized purification component is hardly vaporized, therefore the purification component (hypochlorous acid) is not vaporized, and the purification component is hardly released into the indoor space. On the other hand, in a situation where the required humidification amount is large, for example, in winter in Japan, when heated air having low relative humidity (e.g., 20 °C 30%) is ventilated, water containing the micronized purification component is easily vaporized, and therefore a large amount of purification component is released into the indoor space. In other words, the conventional space purification device has a problem that it is not easy to adjust the amount of the purification component released into the indoor space (into the air).

An object of the present disclosure is to provide a technique that can easily adjust the amount of a purification component released into air.

A space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank based on an integrated humidification amount in the humidifying purifier.

The space purification system according to the present disclosure can easily adjust the amount of the purification component released into air.

### BRIEF DESCRIPTION OF THE DRAWINGS

Fig. 1 is a view illustrating a configuration of a space purification system according to a first exemplary embodiment of the present disclosure.
Fig. 2 is a block diagram illustrating a configuration of a control unit of the space purification system according to the first exemplary embodiment of the present disclosure.
Fig. 3 is a schematic diagram illustrating temporal changes (winter: first example) of a water amount, a hypochlorous acid water concentration, and a hypochlorous acid concentration in the space purification system according to the first exemplary embodiment of the present disclosure.
Fig. 4 is a schematic diagram illustrating temporal changes (summer: second example) of a water amount, a hypochlorous acid water concentration, and a hypochlorous acid concentration in the space purification system according to the first exemplary embodiment of the present disclosure.
Fig. 5 is a schematic diagram illustrating temporal changes (summer: third example) of a water amount, a hypochlorous acid water concentration, and a hypochlorous acid concentration in the space purification system according to the first exemplary embodiment of the present disclosure.
Fig. 6 is a view illustrating a configuration of a space purification system according to a second exemplary embodiment of the present disclosure.
Fig. 7 is a block diagram illustrating a configuration of a control unit of the space purification system according to the second exemplary embodiment of the present disclosure.
Fig. 8 is a schematic diagram illustrating temporal changes (winter: first example) of a water amount, a hypochlorous acid water concentration, and a hypochlorous acid concentration in the space purification system according to the second exemplary embodiment of the present disclosure.
Fig. 9 is a schematic diagram illustrating temporal changes (summer: second example) of a water amount, a hypochlorous acid water concentration, and a hypochlorous acid concentration in the space purification system according to the second exemplary embodiment of the present disclosure.
Fig. 10 is a view illustrating a configuration of a space purification system according to a third exemplary embodiment of the present disclosure.
Fig. 11 is a block diagram illustrating a configuration of a control unit of the space purification system according to the third exemplary embodiment of the present disclosure.
Fig. 12 is a schematic diagram illustrating temporal changes (winter: first example) of a water amount, a hypochlorous acid water concentration, and a hypochlorous acid concentration in the space purification system according to the third exemplary embodiment of the present disclosure.
Fig. 13 is a schematic diagram illustrating temporal changes (summer: second example) of a water amount, a hypochlorous acid water concentration, and a hypochlorous acid concentration in the space purification system according to the third exemplary embodiment of the present disclosure.
Fig. 14 is a schematic diagram illustrating temporal changes (summer: third example) of a water amount, a hypochlorous acid water concentration, and a hypochlorous acid concentration in the space purification system according to the third exemplary embodiment of the present disclosure.

### DESCRIPTION OF EMBODIMENT

A space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank based on an integrated humidification amount in the humidifying purifier.

By doing this, when air having high relative humidity is ventilated as in summer in Japan, since the consumption amount of mixed water stored in the mixing tank is small, the supply frequency (the number of times of performing the first control) of the hypochlorous acid water to the mixing tank increases, and the mixed water is micronized and released into the air in a state where the hypochlorous acid concentration of the mixed water in the mixing tank is high. Since the consumption amount of the mixed water stored in the mixing tank is small, the frequency of drainage of the mixed water (the number of times of performing the third control) decreases, and the hypochlorous acid concentration of the mixed water in the mixing tank is maintained in a high state. As a result, even in a situation where the micronized hypochlorous acid water hardly vaporize, hypochlorous acid raised to a predetermined concentration contained in the air can be released into the indoor space. On the other hand, when air having low relative humidity is ventilated as in winter in Japan, since the consumption amount of the mixed water stored in the mixing tank is large, the supply frequency of water to the mixing tank (the number of times of performing the second control) increases, and the mixed water is micronized and released into the air in a state where the hypochlorous acid concentration of the mixed water in the mixing tank is low. Since the consumption amount of the mixed water stored in the mixing tank is large, the frequency of drainage of the mixed water in the mixing tank (the number of times of performing the third control) increases, and it is possible to suppress an excessive increase in the hypochlorous acid concentration of the mixed water. As a result, even in a situation where the micronized hypochlorous acid water easily vaporizes, hypochlorous acid attenuated to a predetermined concentration contained in the air can be released into the indoor space. That is, the space purification system can easily adjust the amount of hypochlorous acid to be released into the air.

In the space purification system according to the present disclosure, the control unit preferably executes the third control when the integrated humidification amount becomes greater than or equal to a reference amount. This enables the space purification system to easily adjust the concentration of the hypochlorous acid water stored in the mixing tank based on the humidification amount in the humidifying purifier.

In the space purification system according to the present disclosure, the integrated humidification amount is preferably calculated based on the number of times of execution of the first control and the second control. This enables the space purification system to simply calculate the integrated humidification amount, and improve the controllability of the third control.

In the space purification system according to the present disclosure, the control unit preferably executes the third control when the number of times of performing the first control becomes a reference number of times. This enables the space purification system to return the state in the mixing tank to the operation initial state by executing the third control of draining the mixed water stored in the mixing tank before the hypochlorous acid water concentration in the mixing tank excessively increases even when a long time operation (e.g., 24 hours) is performed. That is, the space purification system can easily adjust the amount of hypochlorous acid to be released into the air.

In the space purification system according to the present disclosure, the control unit preferably executes the third control immediately before executing the first control or the second control. Due to this, in the space purification system, since drainage by the third control is no longer performed immediately after the hypochlorous acid is supplied to the mixing tank by the first control or immediately after the water is supplied by the second control, the hypochlorous acid water supplied by the first control or the water supplied by the second control can be continuously used maximally and waste due to the drainage by the third control can be reduced.

In the space purification system according to the present disclosure, in the control of the supply treatment, the control unit preferably (i) performs the first control and the second control such that the number of times of performing the first control becomes smaller than the number of times of performing the second control when the humidification requirement amount required for the humidifying purifier is greater than or equal to a first reference value, and (ii) performs the first control and the second control such that the number of times of performing the first control becomes larger than the number of times of performing the second control when the humidification requirement amount is less than the first reference value. This enables the space purification system can micronize and release, into the air, the mixed water in a state where the hypochlorous acid concentration in the mixing tank is high when the humidification requirement amount is less than the first reference value in the supply treatment. On the other hand, the mixed water can be micronized and released into the air in a state where the hypochlorous acid concentration in the mixing tank is low when the humidification requirement amount is greater than or equal to the first reference value. In other words, based on the humidification requirement amount, the space purification device can give hypochlorous acid to the air released from the humidifying purifier under a condition suitable for the environment of the indoor space.

Modes for carrying out the present disclosure will now be described with reference to the accompanying drawings. Note that the following exemplary embodiments are examples embodying the present disclosure, and do not limit the technical scope of the present disclosure in any way. Throughout the drawings, identical parts are denoted by the identical reference marks, and descriptions thereof will be omitted. Furthermore, details of each part not directly related to the present disclosure are not described for each drawing to avoid redundancy.

### (First exemplary embodiment)

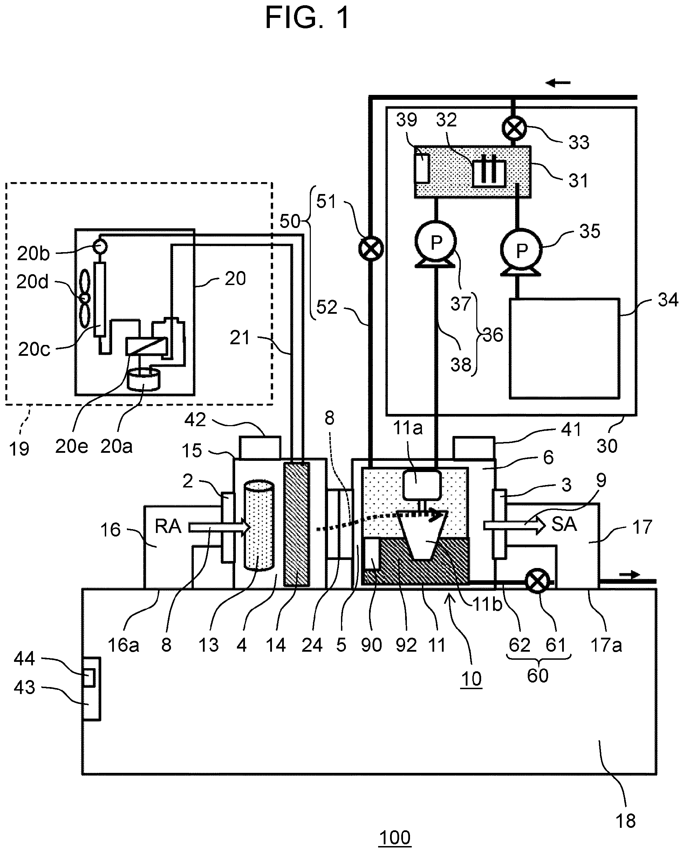

Fig. 1 is a view illustrating the configuration of space purification system 100 according to the first exemplary embodiment of the present disclosure. Space purification system 100 is a device that performs cooling treatment (dehumidification treatment) or heating treatment on air 8 (RA) from indoor space 18 as necessary when circulating the air of indoor space 18, and causes air 8 flowing inside to contain a component (hereinafter, simply called "air purification component") that purifies air together with micronized water. Space purification system 100 performs sterilization and deodorization of indoor space 18 by supplying indoor space 18 with air 9 (SA) having circulated inside. Here, hypochlorous acid is used as the air purification component, and the water containing the air purification component is hypochlorous acid water.

As illustrated in Fig. 1, space purification system 100 is mainly configured to include space purification device 10, air-conditioning device 15, and hypochlorous acid water generator 30.

Space purification device 10 includes outlet 3, air purifier 11, and air purification control unit 41. Air-conditioning device 15 includes inlet 2, air blower 13, refrigerant coil 14, and air-conditioning control unit 42. Each of space purification device 10 and air-conditioning device 15 has a housing constituting an outer frame of the respective device, and space purification device 10 and air-conditioning device 15 are connected via duct 24. Inlet 2 is formed on a side surface of air-conditioning device 15, and outlet 3 is formed on a side surface of space purification device 10.

Inlet 2 is an intake port for taking in air 8 from indoor space 18 into air-conditioning device 15. Inlet 2 is communicated via duct 16 with indoor inlet 16a provided on a ceiling or the like of indoor space 18. Thus, inlet 2 enables the air of indoor space 18 to be suctioned into air-conditioning device 15 via indoor inlet 16a.

Outlet 3 is a discharge port through which air 9 (SA) having circulated in space purification device 10 is discharged to indoor space 18. Outlet 3 is communicated via duct 17 with indoor outlet 17a provided on a ceiling or the like of indoor space 18. Thus, outlet 3 enables air 9 having circulated in space purification device 10 to be blown out toward indoor space 18 from indoor outlet 17a.

Inside air-conditioning device 15 and space purification device 10 is provided with an air passage (prior stage air passage 4, middle stage air passage 5, and subsequent stage air passage 6) causing inlet 2 and outlet 3 to be communicated each other via duct 24. Prior stage air passage 4 is an air passage adjacent to inlet 2. Prior stage air passage 4 is provided with air blower 13 and refrigerant coil 14.

Middle stage air passage 5 is an air passage through which air 8 having circulated through prior stage air passage 4 circulates, at a position adjacent to prior stage air passage 4 (duct 24). Inside middle stage air passage 5 is provided with air purifier 11.

Subsequent stage air passage 6 is an air passage adjacent to outlet 3, and in subsequent stage air passage 6, air 8 having circulated through middle stage air passage 5 circulates through air purifier 11 and becomes air 9 containing hypochlorous acid together with micronized water.

In air-conditioning device 15 and space purification device 10, air 8 having been suctioned in from inlet 2 circulates through prior stage air passage 4, circulates through middle stage air passage 5 and subsequent stage air passage 6, and is blown out from outlet 3 as air 9.

Air blower 13 of air-conditioning device 15 is a device for conveying air 8 (RA) in indoor space 18 from inlet 2 into air-conditioning device 15. Air blower 13 is installed upstream of refrigerant coil 14 in prior stage air passage 4. In air blower 13, on/off of operation behavior is controlled in accordance with blower output information from air-conditioning control unit 42. Due to operation behavior of air blower 13, air 8 in indoor space 18 is taken in to air-conditioning device 15, and directed to refrigerant coil 14.

Refrigerant coil 14 is a member that is disposed downstream of air blower 13 in prior stage air passage 4 and cools or heats air 8 to be introduced. Refrigerant coil 14 changes an output state (cooling, heating, or off) in response to an output signal from air-conditioning control unit 42, and adjusts the cooling capacity (cooling amount) or the heating capacity (heating amount) for air 8 to be introduced. When refrigerant coil 14 cools air 8 to be introduced, air 8 having been introduced is dehumidified, and therefore, the cooling capacity (cooling amount) for air 8 can be deemed also as the dehumidifying capacity (dehumidification amount) for air 8.

Refrigerant coil 14 functions as a heat absorber or a heat radiator in a refrigeration cycle configured to include a compressor, a heat radiator, an expander, and a heat absorber, and is configured to absorb heat (cooling) or radiate heat (heating) when the refrigerant introduced from condenser unit 20 circulates inside. More specifically, refrigerant coil 14 is connected to condenser unit 20 via refrigerant circuit 21 through which a refrigerant flows. Condenser unit 20 is an outdoor unit installed in outdoor space 19, and includes compressor 20a, expander 20b, outdoor heat exchanger 20c, blower fan 20d, and four-way valve 20e. Since condenser unit 20 with a general configuration is used, detailed descriptions of these devices (compressor 20a, expander 20b, outdoor heat exchanger 20c, blower fan 20d, and four-way valve 20e) will be omitted.

Since four-way valve 20e is connected to the refrigeration cycle including refrigerant coil 14, air-conditioning device 15 can switch between a state of a cooling mode (dehumidification mode) in which the refrigerant circulates in a first direction by four-way valve 20e and cools and dehumidifies the air (air 8) and a state of a heating mode in which the refrigerant circulates in a second direction by four-way valve 20e and heats the air (air 8).

Here, the first direction is a direction in which the refrigerant circulates through compressor 20a, outdoor heat exchanger 20c, expander 20b, and refrigerant coil 14 in this order. The second direction is a direction in which the refrigerant circulates through compressor 20a, refrigerant coil 14, expander 20b, and outdoor heat exchanger 20c in this order. Refrigerant coil 14 can cool or heat the air (air 8) to be introduced.

Air purifier 11 of space purification device 10 is a unit for humidifying air 8 taken therein, and causes hypochlorous acid together with micronized water to be contained the air during humidification. More specifically, air purifier 11 includes mixing tank 92, water level sensor 90, humidifying motor 11a, and humidifying nozzle 11b. Air purifier 11 has a centrifugal crushing type configuration in which humidifying nozzle 1 1b is rotated using humidifying motor 11a, the hypochlorous acid water stored in mixing tank 92 of air purifier 11 is suctioned by a centrifugal force, scattered, collided, and crushed in the surroundings (centrifugal direction), and moisture is contained in the passing air. Air purifier 11 adjusts the humidifying capacity (humidification amount) by changing the rotation speed (hereinafter, rotation output value) of humidifying motor 11a in response to an output signal from air purification control unit 41. The humidification amount can be deemed also to be an addition amount by which the hypochlorous acid is added to the air. Note that air purifier 11 corresponds to the "humidifying purifier" in the claims.

Water level sensor 90 measures the water level of the hypochlorous acid water stored in mixing tank 92, and outputs a measurement value to air purification control unit 41. More specifically, water level sensor 90 measures, as the water level of the hypochlorous acid water stored in mixing tank 92, each of the water level at which mixing tank 92 is brought into a water shortage state, the water level at which mixing tank 92 is brought into a full state, and the water level at which mixing tank 92 has a reference water amount, and outputs the measurement values to air purification control unit 41 as water level information. Note that the reference water amount is a water amount in a state of about 5/6 of the capacity of mixing tank 92. Mixing tank 92 is a tank in which hypochlorous acid water is stored in air purifier 11, and can be deemed also to be a water storage. In mixing tank 92, hypochlorous acid water with a predetermined concentration supplied from hypochlorous acid water supplier 36 described later is mixed with the water supplied from water supplier 50 described later, and is stored as mixed water including diluted hypochlorous acid water. Note that the hypochlorous acid water (mixed water) stored in mixing tank 92 can be drained from mixing tank 92 to the outside by drainage 60 that behaves in response to an output signal from air purification control unit 41.

Hypochlorous acid water generator 30 includes electrolytic tank 31, electrodes 32, electromagnetic valve 33, brine tank 34, brine conveyance pump 35, water level sensor 39, and hypochlorous acid water supplier 36.

Brine tank 34 stores brine, and supplies the brine to electrolytic tank 31 via brine conveyance pump 35 in response to an output signal from air purification control unit 41. Electrolytic tank 31 stores the brine that is an electrolysis target supplied from brine tank 34. Electrolytic tank 31 is also supplied with tap water from a water supply pipe such as water supply via electromagnetic valve 33 in response to an output signal from air purification control unit 41, the supplied tap water and brine are mixed, and brine having a predetermined concentration is stored therein. Electrodes 32 are disposed in electrolytic tank 31, performs electrolysis of brine for a predetermined time by energization in response to an output signal from air purification control unit 41, and generates hypochlorous acid water having a predetermined concentration. That is, electrolytic tank 31 generates hypochlorous acid water by performing electrolysis of an aqueous chloride solution (e.g., sodium chloride solution) as an electrolyte between a pair of electrodes. Since a general device is used as electrolytic tank 31, a detailed description will be omitted. Here, the electrolyte is an electrolyte from which hypochlorous acid water can be generated, and is not particularly limited as long as it contains chloride ions even in a small amount, and examples of the electrolyte include an aqueous solution in which sodium chloride, calcium chloride, magnesium chloride, or the like is dissolved as a solute. Hydrochloric acid is also acceptable. In the present exemplary embodiment, an aqueous sodium chloride solution (brine) in which sodium chloride is added to water is used as the electrolyte.

Water level sensor 39 measures the water level in electrolytic tank 31, and outputs the measurement value to air purification control unit 41.

Hypochlorous acid water supplier 36 supplies hypochlorous acid water from electrolytic tank 31 into mixing tank 92 of air purifier 11 in response to an output signal from air purification control unit 41. Hypochlorous acid water supplier 36 includes hypochlorous acid water conveyance pump 37 and water conveyance pipe 38. Hypochlorous acid water conveyance pump 37 feeds the hypochlorous acid water in electrolytic tank 31 to water conveyance pipe 38 in response to an output signal from air purification control unit 41. Water conveyance pipe 38 is connected between hypochlorous acid water conveyance pump 37 and mixing tank 92, and conveys the hypochlorous acid water to mixing tank 92.

Water supplier 50 supplies water to mixing tank 92 in response to an output signal from air purification control unit 41. Water supplier 50 includes electromagnetic valve 51 and water conveyance pipe 52. Electromagnetic valve 51 controls as to whether or not to flow, to water conveyance pipe 52, the water supplied from a water supply pipe external of space purification device 10 in response to an output signal from air purification control unit 41. Water conveyance pipe 52 is connected between electromagnetic valve 51 and mixing tank 92, through which water is conveyed to mixing tank 92.

Drainage 60 is connected to a bottom of mixing tank 92, and drains the mixed water stored in mixing tank 92 to the outside in response to an output signal from air purification control unit 41. Drainage 60 includes electromagnetic valve 61 and water conveyance pipe 62. Electromagnetic valve 61 controls whether or not to flow the mixed water stored in mixing tank 92 to an external drainage pipe in response to an output signal from air purification control unit 41. Water conveyance pipe 62 is connected between mixing tank 92 and electromagnetic valve 61, and supplies mixed water to the external drainage pipe.

In air purifier 11, the hypochlorous acid water from hypochlorous acid water supplier 36 and the water from water supplier 50 are supplied to mixing tank 92. The hypochlorous acid water and the water are mixed in mixing tank 92 of air purifier 11. Mixed water of hypochlorous acid water and water can also be called hypochlorous acid water. More specifically, in mixing tank 92 of air purifier 11, the hypochlorous acid water from hypochlorous acid water supplier 36 or the water from water supplier 50 is supplied to and mixed with the hypochlorous acid water remaining in mixing tank 92. Air purifier 11 releases the hypochlorous acid water to indoor space 18 by centrifugally crushing the mixed water of the hypochlorous acid water and the water stored in mixing tank 92. The micronized hypochlorous acid water is released to indoor space 18 in a state where the liquid component is evaporated.

Operation device 43 is installed on a wall surface of indoor space 18. Operation device 43 includes a user interface that a user can operate, and receives a temperature setting value and a humidity setting value from the user. Operation device 43 includes temperature and humidity sensor 44, and temperature and humidity sensor 44 measures the temperature and humidity of air in indoor space 18. It is sufficient to use a known technology for temperature and humidity sensor 44 to measure the temperature and humidity, and therefore, a description is omitted here.

Operation device 43 is connected to air purification control unit 41 and air-conditioning control unit 42 in a wired or wireless manner, and transmits a temperature setting value, a humidity setting value, a temperature measurement value, and a humidity measurement value to air purification control unit 41 and air-conditioning control unit 42. These pieces of information may all be transmitted together, any two or more may be transmitted together, or each may be transmitted. Operation device 43 may transmit information to air purification control unit 41, and air purification control unit 41 may transfer information to air-conditioning control unit 42.

Air-conditioning control unit 42 of air-conditioning device 15 receives the temperature setting value and the temperature measurement value, and controls refrigerant coil 14 and condenser unit 20 so that the temperature measurement value approaches the temperature setting value. Air-conditioning control unit 42 increases the degree of heating as a difference between the temperature measurement value and the temperature setting value increases when the temperature measurement value is lower than the temperature setting value in the heating mode.

Next, air purification control unit 41 of space purification device 10 will be explained.

As treatment operations of hypochlorous acid water generator 30 and space purification device 10, air purification control unit 41 controls each of a behavior regarding the electrolysis treatment in electrolytic tank 31, a behavior regarding the supply treatment of hypochlorous acid water to air purifier 11, a behavior regarding the supply treatment of water to air purifier 11, a behavior regarding the humidifying purification treatment in air purifier 11, and a behavior regarding the drainage treatment of the mixed water in air purifier 11. Note that air purification control unit 41 includes a computer system including a processor and a memory. The computer system functions as a control unit by the processor executing a program stored in the memory. The program to be executed by the processor is assumed here to be recorded in advance in the memory of the computer system, but may be provided in a manner being recorded in a non-transitory recording medium such as a memory card, or provided through a telecommunication line such as the Internet. Air purification control unit 41 corresponds to the "control unit" in the claims.

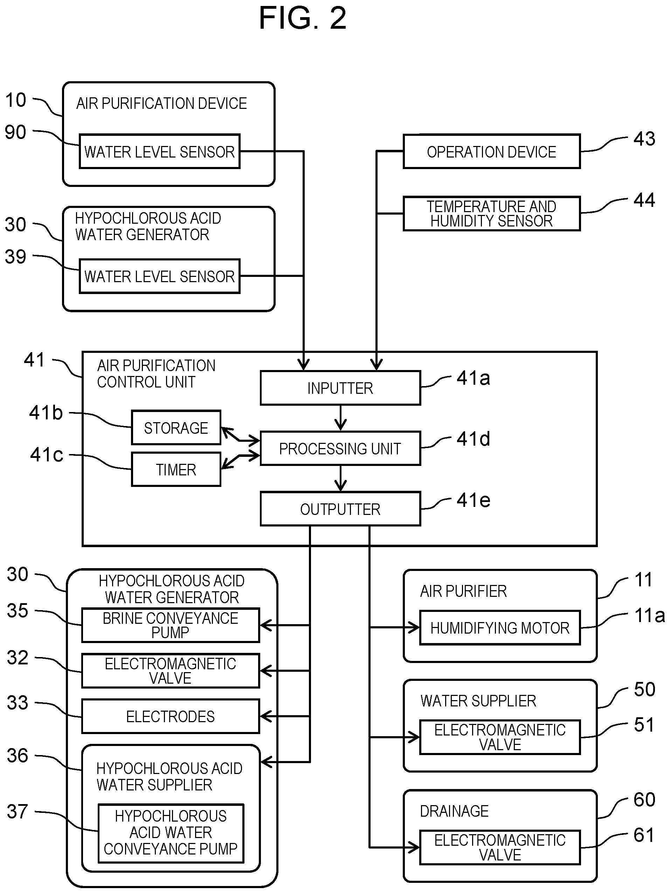

Fig. 2 is a block diagram illustrating the configuration of air purification control unit 41 of space purification system 100 according to the first exemplary embodiment. Specifically, as illustrated in Fig. 2, air purification control unit 41 includes inputter 41a, storage 41b, timer 41c, processing unit 41d, and outputter 41e.

### <Behavior regarding electrolysis treatment in electrolytic tank>

Air purification control unit 41 executes the following treatment as a behavior regarding the electrolysis treatment in electrolytic tank 31.

As a trigger of the electrolysis treatment of electrolytic tank 31, air purification control unit 41 receives and outputs, to processing unit 41d, water level information (water shortage signal) from water level sensor 39 and information regarding time (time information) from timer 41c.

Processing unit 41d specifies and outputs, to outputter 41e, control information based on the water level information from water level sensor 39, the time information from timer 41c, and the setting information from storage 41b. Here, the setting information includes information regarding start time or end time of hypochlorous acid water generation, information regarding a supply amount of tap water to be introduced into electrolytic tank 31, information regarding an input amount of a liquid containing chloride ions in brine conveyance pump 35, information regarding electrolysis conditions (such as time, current value, and voltage) in electrodes 32, information regarding opening/closing timing of electromagnetic valve 33, and information regarding on/off behavior of hypochlorous acid water conveyance pump 37.

The electrolysis conditions in electrodes 32 can be determined based on the water amount of tap water in electrolytic tank 31, the chloride ion concentration, the electrolysis time, and a deterioration degree of electrodes 32, are set with an algorithm created, and stored in storage 41b.

Outputter 41e outputs a signal (control signal) to each device (brine conveyance pump 35, electromagnetic valve 33, and hypochlorous acid water conveyance pump 37) based on the received control information.

More specifically, brine conveyance pump 35 first maintains a state of stopping based on a signal from outputter 41e, and hypochlorous acid water conveyance pump 37 maintains a state of stopping based on a signal from outputter 41e.

Then, electromagnetic valve 33 is opened based on a signal from outputter 41e. Due to this, supply of tap water from the water supply pipe to electrolytic tank 31 starts. Thereafter, electromagnetic valve 33 is closed based on a signal from outputter 41e having received the water level information (full) from water level sensor 39. Due to this, electrolytic tank 31 is brought into a state where tap water is supplied with a set supply amount.

Next, brine conveyance pump 35 starts behavior based on a signal from outputter 41e, conveys, to electrolytic tank 31, a liquid containing a predetermined amount of chloride ions, and is stopped. Due to this, chloride ions dissolve in the tap water, and electrolytic tank 31 is brought into a state where an aqueous solution (aqueous chloride solution) containing a predetermined amount of chloride ions is generated.

Then, electrodes 32 start electrolysis of the aqueous chloride solution based on a signal from outputter 41e, generates the hypochlorous acid water with the set conditions, and is stopped. The hypochlorous acid water generated by electrodes 32 is brought into a state where, for example, the hypochlorous acid concentration is 100 ppm to 150 ppm (e.g., 120 ppm) and pH is 7 to 8.5 (e.g., 8.0).

As described above, air purification control unit 41 executes the electrolysis treatment in electrolytic tank 31, and hypochlorous acid water of a predetermined concentration and amount is generated.

### <Behavior regarding supply treatment of hypochlorous acid water to air purifier>

Air purification control unit 41 executes the following treatment as a behavior regarding the supply treatment of hypochlorous acid water to air purifier 11.

As a trigger of the supply treatment of hypochlorous acid water to air purifier 11, air purification control unit 41 outputs a hypochlorous acid water supply request to hypochlorous acid water generator 30 (hypochlorous acid water supplier 36) every time timer 41c measures the operating time of humidifying motor 11a and the operating time elapses a predetermined time (e.g., 60 minutes). Here, the predetermined time is time estimated in advance through an experimental evaluation, given the fact that hypochlorous acid in hypochlorous acid water vaporizes and decreases over time.

Specifically, processing unit 41d specifies and outputs, to outputter 41e, control information based on the information regarding time (time information) from timer 41c and the setting information from storage 41b. Here, the setting information includes information regarding the supply interval of the hypochlorous acid water (e.g., 60 minutes) and information regarding on/off behavior of hypochlorous acid water conveyance pump 37.

Then, outputter 41e outputs a signal (control signal) to hypochlorous acid water conveyance pump 37 of hypochlorous acid water supplier 36 based on the received control information.

Hypochlorous acid water conveyance pump 37 operates based on a signal from outputter 41e. At this time, if the water amount of mixing tank 92 is greater than or equal to a reference water amount, hypochlorous acid water conveyance pump 37 waits until the water amount of mixing tank 92 becomes less than the reference water amount, and starts operation at timing when the hypochlorous acid water stored in mixing tank 92 is consumed and the water amount of mixing tank 92 becomes less than the reference water amount. In the present exemplary embodiment, the reference water amount is about 5/6 of the capacity of mixing tank 92. Due to this, hypochlorous acid water generator 30 starts supply of the hypochlorous acid water from electrolytic tank 31 to air purifier 11 (mixing tank 92). Note that in order to ensure the concentration of the hypochlorous acid water stored in electrolytic tank 31, when hypochlorous acid water is supplied from hypochlorous acid water generator 30 to mixing tank 92, the entire amount of hypochlorous acid water generated in electrolytic tank 31 is supplied. Therefore, after the hypochlorous acid water is supplied, electrolytic tank 31 is in an empty state, and generation of hypochlorous acid water is not started from a state where the hypochlorous acid water remains in electrolytic tank 31. When the hypochlorous acid water in electrolytic tank 31 is brought into a state where the entire amount has been supplied, water level sensor 39 outputs a water shortage signal as water level information.

Thereafter, hypochlorous acid water conveyance pump 37 is stopped based on a signal from outputter 41e having received the information regarding the time (time required for supplying a prescribed amount) from timer 41c. Due to this, hypochlorous acid water generator 30 supplies the hypochlorous acid water from electrolytic tank 31 to air purifier 11 (mixing tank 92) at a set supply amount.

As described above, air purification control unit 41 executes the supply treatment of hypochlorous acid water from hypochlorous acid water generator 30 (electrolytic tank 31) to air purifier 11. Note that the control by which air purification control unit 41 performs supply of the hypochlorous acid water by hypochlorous acid water supplier 36 at predetermined time intervals is "first control".

### <Behavior regarding supply treatment of water to air purifier>

Air purification control unit 41 executes the following treatment as a behavior regarding the supply treatment of water to air purifier 11.

As a trigger of the supply treatment of water to air purifier 11, air purification control unit 41 receives water level information (water shortage signal) from water level sensor 90 of space purification device 10, and outputs a water supply request to water supplier 50.

Specifically, inputter 41a receives and outputs, to processing unit 41d, water level information (water shortage signal) from water level sensor 90 of space purification device 10.

Processing unit 41d specifies and outputs, to outputter 41e, control information based on the water level information (water shortage signal) from inputter 41a, the information regarding time (time information) from timer 41c, and the setting information from storage 41b. Here, the setting information includes information regarding on/off behavior of electromagnetic valve 51 of water supplier 50.

Then, outputter 41e then outputs a signal (control signal) to electromagnetic valve 51 based on the received control information.

Electromagnetic valve 51 operates based on a signal from outputter 41e. Due to this, water supplier 50 starts supply of water from an external water supply pipe to air purifier 11 (mixing tank 92), via water conveyance pipe 52.

Thereafter, electromagnetic valve 51 is stopped based on a signal from outputter 41e having received the water level information (full signal) from water level sensor 90 of space purification device 10. Due to this, water supplier 50 supplies water from the external water supply pipe to air purifier 11 (mixing tank 92) until the water reaches the set amount.

As described above, air purification control unit 41 executes the supply treatment of water from water supplier 50 to air purifier 11. Note that the control by which air purification control unit 41 supplies water by water supplier 50 based on information

(water shortage information) regarding the water level of mixing tank 92 from water level sensor 90 is "second control".

### <Behavior regarding humidifying purification treatment in air purifier>

Next, the behavior regarding the humidifying purification treatment in air purifier 11 of air purification control unit 41 will be explained.

Inputter 41a receives user input information from operation device 43, temperature and humidity information of the air of indoor space 18 from temperature and humidity sensor 44, and water level information of the hypochlorous acid water (mixed water) in mixing tank 92 from water level sensor 90. Inputter 41a outputs each piece of the received information to processing unit 41d.

Here, operation device 43 is a terminal to which user input information (e.g., air volume, target temperature, target humidity, presence or absence of addition of hypochlorous acid, target supply amount level of hypochlorous acid, and the like) regarding space purification device 10 is input, and is communicably connected to air purification control unit 41 in a wireless or wired manner.

Temperature and humidity sensor 44 is a sensor provided in target indoor space 18, and senses the temperature and humidity of the air in indoor space 18.

Storage 41b stores the user input information received by inputter 41a, and the supply setting information in supply behavior of hypochlorous acid to the air being circulated in the device. Storage 41b outputs, to processing unit 41d, the supply setting information having been stored. Note that the supply setting information in supply behavior of hypochlorous acid can also be deemed as humidification setting information in humidifying purification operation of air purifier 11.

Timer 41c outputs time information regarding current time to processing unit 41d.

Processing unit 41d receives various types of information (user input information, temperature and humidity information, and water level information) from inputter 41a, the time information from timer 41c, and the supply setting information from storage 41b. Using the received user input information, time information, and supply setting information, processing unit 41d specifies control information regarding humidifying purification operation behavior.

Specifically, processing unit 41d specifies the humidification requirement amount necessary for indoor space 18 based on a humidity difference between the target humidity stored in storage 41b and the temperature and humidity information of the air in indoor space 18 from temperature and humidity sensor 44 at a regular interval by time information from timer 41c. Then, processing unit 41d specifies the control information regarding the humidifying purification operation behavior based on the specified humidification requirement amount and the supply setting information stored in storage 41b. Then, processing unit 41d outputs the specified control information to outputter 41e.

In processing unit 41d, when the water level information from water level sensor 90 includes information (water shortage signal) regarding the water level indicating water shortage of the hypochlorous acid water (mixed water) in mixing tank 92, outputter 41e outputs, to outputter 41e, a signal of water supply request for water supplier 50. Furthermore, in processing unit 41d, when the operating time of air purifier 11 (humidifying motor 11a) reaches a predetermined time (e.g., 60 minutes) based on the time information from timer 41c, outputter 41e outputs, to outputter 41e, a signal of hypochlorous acid water supply request for hypochlorous acid water generator 30. Note that in the present exemplary embodiment, the water level at which the hypochlorous acid water (mixed water) in mixing tank 92 indicates water shortage is set to a water level in a state where the hypochlorous acid water amount has dropped to about 1/3 from the state where the hypochlorous acid water (mixed water) is full in mixing tank 92.

Then, outputter 41e outputs the received signals to air purifier 11, hypochlorous acid water generator 30 (hypochlorous acid water supplier 36), and water supplier 50, respectively.

Then, air purifier 11 receives a signal from outputter 41e and executes control of the operation behavior based on the received signal. At this time, hypochlorous acid water generator 30 (hypochlorous acid water supplier 36) receives a signal (signal for hypochlorous acid water supply request) from outputter 41e, and executes behavior (first control) regarding the supply treatment of hypochlorous acid water to air purifier 11 described above, based on the received signal. Water supplier 50 receives a signal (signal for water supply request) from outputter 41e, and executes behavior (second control) regarding the supply treatment of water to air purifier 11 described above, based on the received signal.

As described above, air purification control unit 41 executes, as the supply treatment, first control of performing supply of the hypochlorous acid water by hypochlorous acid water generator 30 (hypochlorous acid water supplier 36) at predetermined time intervals, and second control of performing supply of water by water supplier 50 based on information (water shortage information) regarding a water level of mixing tank 92 from water level sensor 90, and stores the mixed water in mixing tank 92. Then, when supplying hypochlorous acid water and water to and storing mixed water in mixing tank 92, air purification control unit 41 executes the humidifying purification treatment to the air circulating through space purification device 10 (air purifier 11) with supply cycle of the hypochlorous acid water (predetermined time intervals) and supply cycle of the water (every water shortage detection) being made different from each other.

### <Behavior regarding drainage treatment of mixed water of air purifier>

Air purification control unit 41 executes the following treatment as a behavior regarding the drainage treatment of the mixed water stored in mixing tank 92 of air purifier 11.

As a trigger of the drainage treatment of the mixed water stored in mixing tank 92, air purification control unit 41 determines the presence or absence of implementation of the drainage treatment based on the information regarding an integrated value of the humidification amount (integrated humidification amount) in air purifier 11 or the information regarding the number of times of execution of the first control in hypochlorous acid water supplier 36.

Specifically, storage 41b stores the number of times of execution of the first control in hypochlorous acid water supplier 36 and the number of times of execution of the second control by water supplier 50. Here, the number of times of execution is the number of times of each control executed after the start of the humidifying purification treatment behavior (hereinafter, also called "after the operation start") with the initial state (e.g., a state where mixing tank 92 becomes full with supply of water and supply of hypochlorous acid water executed after the drainage treatment) of mixing tank 92 as a starting point.

Processing unit 41d specifies an integrated value (integrated humidification amount) of the humidification amount in air purifier 11 based on the information regarding the number of times of execution of the first control in hypochlorous acid water supplier 36 and the information regarding the number of times of execution of the second control by water supplier 50 from storage 41b.

Here, the integrated humidification amount is the total of the water supply amount (total of the hypochlorous acid water supply amount by the first control and the water supply amount by the second control) to mixing tank 92 after the operation start, and corresponds to the water amount of mixed water consumed and reduced by air purifier 11 after the operation start. Note that the integrated humidification amount is also called a cumulative humidification amount.

Processing unit 41d determines whether or not the specified integrated humidification amount is greater than or equal to the reference amount and whether or not the number of times of execution of the first control is the reference number of times.

Here, the supply amount of hypochlorous acid water by the first control is set to about 1/6 of the capacity of mixing tank 92, the supply amount of water by the second control is set to about 2/3 of the capacity of mixing tank 92, and the reference amount is set to about 2 times the capacity of mixing tank 92. The reference number of times is set to 11 times, which is immediately before the reference amount is reached only by the supply of hypochlorous acid water by the first control.

As a result of the determination, when the specified integrated humidification amount is greater than or equal to the reference amount, or when the number of times of execution of the first control is the reference number of times, processing unit 41d specifies and outputs, to outputter 41e, control information based on the information regarding time (time information) from timer 41c and the setting information from storage 41b. Here, the setting information includes information regarding on/off behavior of electromagnetic valve 61 of drainage 60.

Then, outputter 41e then outputs a signal (control signal) to electromagnetic valve 61 based on the received control information.

Electromagnetic valve 61 operates based on a signal from outputter 41e. Due to this, drainage 60 starts drainage of the mixed water from mixing tank 92 to the external drainage pipe through water conveyance pipe 62.

Thereafter, electromagnetic valve 61 is stopped after a lapse of a predetermined time (e.g., 1 minute) based on a signal from outputter 41e having received the time information from timer 41c. Due to this, mixing tank 92 is brought into an empty state with the stored mixed water drained entirely.

As described above, air purification control unit 41 executes drainage treatment of the mixed water from mixing tank 92 to the outside. Note that the control by which air purification control unit 41 performs drainage of mixed water by drainage 60 based on information regarding the integrated humidification amount in air purifier 11 or information regarding the number of times of execution of the first control in hypochlorous acid water supplier 36 is called "third control".

Here, the third control is preferably performed immediately before execution of the first control by hypochlorous acid water supplier 36 or immediately before execution of the second control by water supplier 50. Due to this, for example, since drainage by the third control is no longer performed immediately after the hypochlorous acid water is supplied to mixing tank 92 by the first control or immediately after the water is supplied by the second control, the mixed water stored in mixing tank 92 can be continuously used maximally and waste due to the drainage by the third control can be reduced. Note that in the following example, from the viewpoint of reducing waste of hypochlorous acid water as an active ingredient of disinfection, the third control is performed immediately before the execution of the first control.

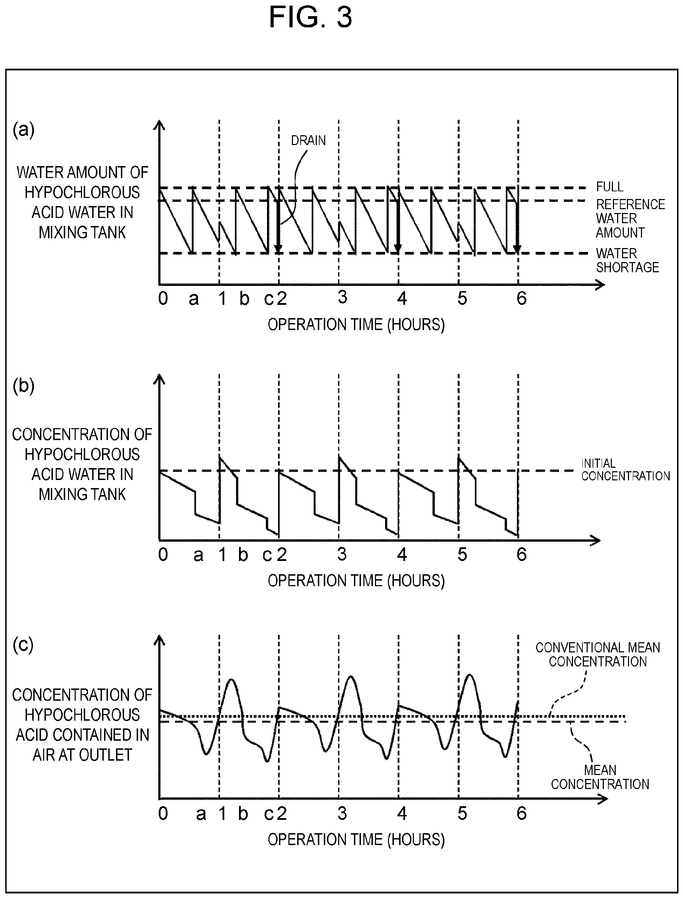

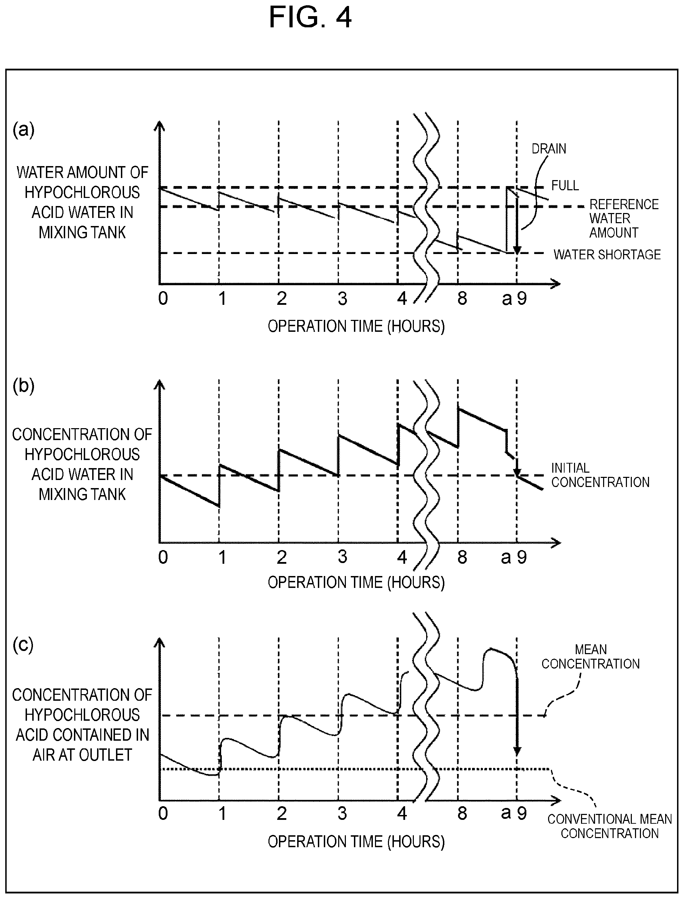

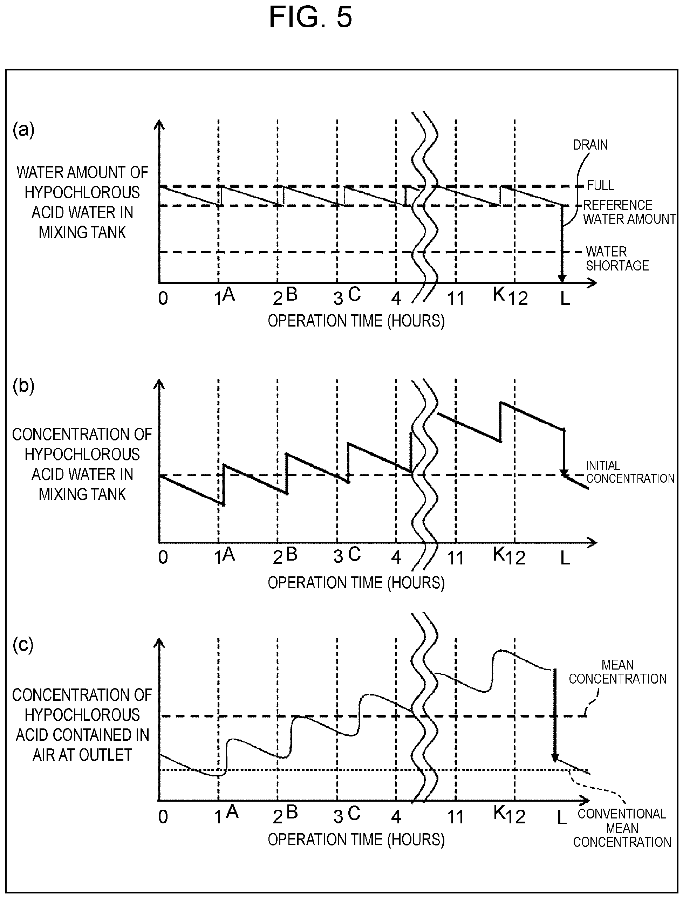

Next, with reference to Figs. 3 to 5, mixed water (mixed water mixed by performing the first control or the second control) in mixing tank 92 of space purification device 10 (air purifier 11) in space purification system 100 will be described. Fig. 3 is a schematic diagram illustrating temporal changes (winter: first example) of the water amount, the hypochlorous acid water concentration, and the hypochlorous acid concentration in space purification system 100. More specifically, (a) of Fig. 3 illustrates a temporal change in the water amount of hypochlorous acid water (mixed water) in mixing tank 92. (b) of Fig. 3 illustrates a history change of the concentration of hypochlorous acid water (mixed water) in mixing tank 92. (c) of Fig. 3 illustrates a temporal change in the concentration of the hypochlorous acid contained in the air at outlet 3. Fig. 4 is a schematic diagram illustrating temporal changes (summer: second example) of the water amount, the hypochlorous acid water concentration, and the hypochlorous acid concentration in space purification system 100. More specifically, (a) of Fig. 4 illustrates a temporal change in the water amount of hypochlorous acid water (mixed water) in mixing tank 92. (b) of Fig. 4 illustrates a history change of the concentration of hypochlorous acid water (mixed water) in mixing tank 92. (c) of Fig. 4 illustrates a temporal change in the concentration of the hypochlorous acid contained in the air at outlet 3. Fig. 5 is a schematic diagram illustrating temporal changes (summer: third example) of the water amount, the hypochlorous acid water concentration, and the hypochlorous acid concentration in space purification system 100. More specifically, (a) of Fig. 5 illustrates a temporal change in the water amount of hypochlorous acid water (mixed water) in mixing tank 92. (b) of Fig. 5 illustrates a history change of the concentration of hypochlorous acid water (mixed water) in mixing tank 92. (c) of Fig. 5 illustrates a temporal change in the concentration of the hypochlorous acid contained in the air at outlet 3.

Here, the supply of the hypochlorous acid water to mixing tank 92 is executed every predetermined time (1 hour), and the supply of the water to mixing tank 92 is executed every time water level sensor 90 detects the water level at which mixing tank 92 becomes water shortage. The drainage treatment is executed based on a determination result based on the integrated humidification amount or the number of times of execution of the first control performed immediately before the execution of the first control. More specifically, the drainage treatment is executed when the integrated humidification amount becomes greater than or equal to the reference amount (about 2 times the capacity of mixing tank 92) or when the number of times of execution of the first control becomes the reference number of times (11 times). Note that the drainage treatment may be performed not only immediately before the execution of the first control but also immediately before the execution of the second control.

Note that as described above, even when the hypochlorous acid water (mixed water) in mixing tank 92 reaches the water level of water shortage, hypochlorous acid water (mixed water) remains in mixing tank 92 by about 1/3 with respect to that at the time of full. In order to simplify the description, it is assumed that air purifier 11 behaves with a constant humidification requirement amount during the humidifying purification operating time. Hereinafter, a predetermined amount of hypochlorous acid water supplied to mixing tank 92 is also called "undiluted hypochlorous acid water".

First, a behavior situation in winter in Japan will be described. Note that in winter in Japan, since the outside air is dry, the humidification requirement amount to air purifier 11 is large, and supply of water is performed at a shorter interval than supply of hypochlorous acid water. In other words, the water level in mixing tank 92 becomes water shortage before the supply timing of the hypochlorous acid water.

Therefore, hereinafter, as the first example, the drainage treatment (third control) under a humidifying purification condition in which the supply of water (second control) is executed 3 times and the supply of undiluted hypochlorous acid water (first control) is executed once in a period of up to 2 hours of operating time after the operation start of air purifier 11 will be described.

Note that the humidifying purification condition described above is a condition set based on controlling air purifier 11 such that the number of times of performing the first control becomes smaller than the number of times of performing the second control when the humidification requirement amount for air purifier 11 is greater than or equal to the first reference value. Here, the first reference value is a value set to distinguish between a situation where the humidity of the air is low and the air is dry in winter in Japan and a situation where the humidity of the air is high and the air is moist in summer in Japan.

In the first example, as illustrated in (a) of Fig. 3, the supply of undiluted hypochlorous acid water (first control) to mixing tank 92 is executed at timings of 1 hour, 2 hours, 3 hours..., where the operation start is 0 hours. On the other hand, the supply of water (second control) to mixing tank 92 is executed at timings of a hours, b hours, and c hours.... At the time point of 0 hours when the operation is started, the supply of the hypochlorous acid water and supply of the water to mixing tank 92 are executed, and mixing tank 92 is in a state (initial state) of being full with the hypochlorous acid water (mixed water) having a predetermined concentration.

The drainage determination of the mixed water stored in mixing tank 92 is performed immediately before the execution of the first control at the timings of 1 hour, 2 hours, 3 hours....

Specifically, at the timing of 1 hour when the operating time after the operation start becomes a period up to 1 hour (period when the operating time is greater than or equal to 0 hours and less than 1 hour), the integrated humidification amount becomes the supply amount (about 0.67 times the capacity of mixing tank 92) based on one time of the second control, and it is determined that the integrated humidification amount is less than the reference amount (2 times the capacity of mixing tank 92). The number of times of execution of the first control is 0 times and is determined to have not reached the reference number of times (11 times). In response to the determination result, the first control is executed, and mixing tank 92 is supplied with the undiluted hypochlorous acid water.

Subsequently, at the timing of 2 hours when the operating time after the operation start becomes a period up to 2 hours (period when the operating time is greater than or equal to 0 hours and less than 2 hours), the integrated humidification amount becomes the supply amount (about 2.1 times the capacity of mixing tank 92) based on 1 time of the first control and 3 times of the second control, and it is determined that the integrated humidification amount is greater than or equal to the reference amount (2 times the capacity of mixing tank 92). The number of times of execution of the first control is 1 time and is determined to have not reached the reference number of times (11 times). In response to the determination result, the third control is executed, and the mixed water in mixing tank 92 is drained. Furthermore, after the third control is executed, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state.

Thereafter, the timing of 2 hours is regarded as the initial state (0 hours), and the same supply behavior and drainage operation are repeated every period of 2 hours.

This will be described in more detail.

First, with reference to (a) of Fig. 3, a description will be given focusing on a temporal change in the water level of the hypochlorous acid water (mixed water) in mixing tank 92.

At the early stage of the operation (0 hours), mixing tank 92 is filled with mixed water (this is also hypochlorous acid water) of undiluted hypochlorous acid solution and water up to full. The humidifying purification operation reduces the water amount of the mixed water at a constant speed and detects water shortage at a timing at which a hours elapses from the operation start, and water is supplied from water supplier 50 until mixing tank 92 becomes full. Thereafter, while the water level of the mixed water is reduced at a constant speed by the humidifying purification operation, 1 hour, which is the supply timing of the undiluted hypochlorous acid water, elapses, and the drainage determination of the mixed water is executed at this timing of 1 hour. In the humidifying purification operation so far, since the number of times of supply of water by the second control is 1 time and the number of times of supply of undiluted hypochlorous acid water by the first control is 0 times, the integrated humidification amount becomes about 0.67 times (≈ about 2/3 times = about 2/3 × 1 time) the capacity of mixing tank 92, and it is determined that the integrated humidification amount is less than the reference amount (2 times the capacity of mixing tank 92). Furthermore, the number of times of supply of the undiluted hypochlorous acid water by the first control is 0 times, and it is determined that the number of times of execution of the first control has not reached the reference number of times (11 times).

In response to the determination result, the first control is executed without the drainage of the mixed water stored in mixing tank 92 being executed, and the undiluted hypochlorous acid water is supplied from hypochlorous acid water generator 30 (hypochlorous acid water supplier 36) to mixing tank 92. Due to this, the water level in mixing tank 92 rises slightly. Also thereafter, the water level of the mixed water decreases by the humidifying purification operation, the water shortage occurs again at the timing of b hours and c hours from the operation start, and the water is supplied from water supplier 50 until mixing tank 92 becomes full.

Thereafter, the drainage determination is performed at a timing when the operating time after the operation start becomes 2 hours. In the humidifying purification operation so far, since the number of times of supply of water by the second control is 3 times and the number of times of supply of undiluted hypochlorous acid water by the first control is 1 time, the integrated humidification amount becomes about 2.1 times the capacity of mixing tank 92 (≈ about 13/6 times = about 2/3 × 3 times + about 1/6 × 1 time), and is determined to be greater than or equal to the reference amount (2 times the capacity of mixing tank 92). Furthermore, the number of times of supply of the undiluted hypochlorous acid water by the first control is 1 time, and it is determined that the number of times of execution of the first control has not reached the reference number of times (11 times). In response to the determination result, the third control is executed, and the mixed water in mixing tank 92 is drained. Furthermore, after the execution of drainage of the mixed water by the third control, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state (0 hours). Here, the integrated humidification amount (the number of times of execution of the first control and the second control) is reset, and the storage of the integrated humidification amount is started again.

Thereafter, the timing of 2 hours is regarded as the initial state (0 hours), and the same supply behavior and drainage operation are repeated every period of 2 hours. More specifically, same as ever, supply of water by the second control at the timing when the water shortage occurs, and supply of undiluted hypochlorous acid water by the first control at the supply timing of hypochlorous acid water are repeated. Immediately before the execution of the first control, the drainage determination of the mixed water by the third control is performed, and the third control is executed when the condition is satisfied.

Next, with reference to (b) of Fig. 3, a description will be given focusing on a temporal change in the concentration of the hypochlorous acid water (mixed water) in mixing tank 92.

At the beginning of the operation (0 hours), mixed water of undiluted hypochlorous acid water and water is mixed in mixing tank 92 so as to have a predetermined concentration (initial concentration). Then, when the humidifying purification operation is started, the concentration of the hypochlorous acid water (mixed water) in mixing tank 92 decreases with the elapse of time from the operation start up to a hours. This is because a constant proportion of hypochlorous acid with respect to the concentration of the hypochlorous acid water vaporizes and is given to the air due to the vapor pressure of hypochlorous acid being higher than that of water. Note that unless the hypochlorous acid vaporizes, the hypochlorous acid contained in the water merely consumed together with the water micronized by air purifier 11, and therefore, the hypochlorous acid water does not change in terms of the concentration of the hypochlorous acid water in mixing tank 92 although decreasing at a constant speed depending on the humidification amount. Even at a hours, which is the timing at which water level sensor 90 detects the water shortage, the concentration of the hypochlorous acid water is not zero, which is because the hypochlorous acid water (mixed water) remains in mixing tank 92 even in a state where a water shortage is detected, as mentioned earlier.

Then, when a hours elapses (water shortage detection) from the operation start, the hypochlorous acid water in mixing tank 92 is diluted with water along with supply of the water from water supplier 50, and therefore the concentration of the hypochlorous acid water in mixing tank 92 decreases. Thereafter, the concentration of the hypochlorous acid water (mixed water) slightly decreases by vaporization of the hypochlorous acid until 1 hour, which is the supply timing of the hypochlorous water, elapses.

Then, when 1 hour, which is the supply timing of the hypochlorous acid water, elapses from the operation start, the concentration of the hypochlorous acid water in mixing tank 92 rises to greater than or equal to the initial concentration along with supply of undiluted hypochlorous acid water from hypochlorous acid water generator 30 (hypochlorous acid water supplier 36). This is because a predetermined amount of hypochlorous acid water (undiluted hypochlorous acid water) supplied at the initial stage of operation is supplied to mixed water (water in a state of containing hypochlorous acid) having a water amount smaller than that of water supplied at the initial stage (0 hours) of the operation. Thereafter, the concentration of the hypochlorous acid water (mixed water) decreases by the vaporization of the hypochlorous acid up to b hours (water shortage detection) elapses from the operation start. Note that the decrease speed of the hypochlorous acid is higher than in the initial stage of operation because the content of the hypochlorous acid contained in the mixed water is large and accordingly, the vaporization amount of the hypochlorous acid becomes large.

Then, when b hours elapses (water shortage detection) from the operation start, the hypochlorous acid water in mixing tank 92 is diluted with water along with supply of the water from water supplier 50, and therefore the concentration of the hypochlorous acid water in mixing tank 92 decreases. Thereafter, similarly, the concentration decreases due to volatilization from b hours (water shortage detection) to c hours (water shortage detection), and when c hours elapses (water shortage detection) from the operation start, the hypochlorous acid water in mixing tank 92 is diluted with water along with supply of the water from water supplier 50, and therefore the concentration of the hypochlorous acid water in mixing tank 92 decreases.

Thereafter, the concentration of the hypochlorous acid water (mixed water) slightly decreases by vaporization of the hypochlorous acid until 2 hours, which is the supply timing of the hypochlorous water, elapses.

When 2 hours, which is the supply timing of the hypochlorous acid water, elapses from the operation start, the drainage timing comes based on the drainage determination, therefore, after the hypochlorous acid water (mixed water) in mixing tank 92 is drained entirely, water and undiluted hypochlorous acid water are supplied into mixing tank 92, and the concentration of the hypochlorous acid water in mixing tank 92 is brought into a similar state to that in the initial stage of operation (0 hours). Thereafter, the concentration change of hypochlorous acid water (mixed water) is repeated in the same manner as ever.

Next, with reference to (c) of Fig. 3, a description will be given focusing on a temporal change in the concentration of the hypochlorous acid contained in air 9 at outlet 3.

The concentration of the hypochlorous acid contained in air 9 released from outlet 3 is determined by the humidification amount in air purifier 11 and the concentration of the hypochlorous acid water in mixing tank 92, but in the first example, since the humidification amount is constant, the concentration of the hypochlorous acid water in mixing tank 92 is reflected. Therefore, as illustrated in (c) of Fig. 3, the concentration of the hypochlorous acid in air 9 at outlet 3 increases or decreases in accordance with an increase or a decrease in the concentration of the hypochlorous acid water in mixing tank 92 as illustrated in (b) of Fig. 3.

Here, as conventionally, in a case where undiluted hypochlorous acid water and water are supplied to make full every time water level sensor 90 detects water shortage, the state from the operation start (0 hours) to a hours is repeated until the timing of 2 hours. In this case, the mean concentration of the hypochlorous acid contained in air 9 at outlet 3 becomes a conventional mean concentration, for example. On the other hand, in the first example, the state from the operation start (0 hours) to a hours is the same as the conventional state, but the state in the period from a hours to 2 hours is different from the conventional state.

More specifically, in the period from a hours to 2 hours, as illustrated in (b) of Fig. 3, the period (period from 1 hour to b hours) in which the concentration of the hypochlorous acid water is higher than the initial concentration has become shorter than the period (period from a hours to 1 hour, from b hours to 2 hours) in which it is smaller than the initial concentration. Therefore, in the period from the operation start (0 hours) to 2 hours, the mean concentration of the hypochlorous acid contained in air 9 at outlet 3 becomes a mean concentration lower than the conventional mean concentration. Also for 2 hours and thereafter, the concentration change from 0 hours to 2 hours is repeated, and it is therefore possible to keep maintaining a mean concentration lower than that ever without a continuous increase in the concentration.

As described above, as in the first example, when undiluted hypochlorous acid water and water are supplied to mixing tank 92 and the mixed water is stored, the supply cycle (predetermined time intervals) of the undiluted hypochlorous acid water and the supply cycle (every water shortage detection) of the water are made different, and the drainage treatment of the mixed water is performed depending on the integrated humidification amount, whereby the concentration of the hypochlorous acid contained in air 9 at outlet 3, i.e., the air blown out into indoor space 18 can be reduced as compared with the case where the hypochlorous acid water and the water are supplied to mixing tank 92 in the conventional method.

Next, a behavior situation in summer in Japan will be described. Note that in summer in Japan, since the outside air is moist, the humidification requirement amount to air purifier 11 is small, and supply of water is performed at a longer interval than supply of the hypochlorous acid water. In other words, the water level in mixing tank 92 becomes water shortage after the supply timing of the hypochlorous acid water.

Therefore, hereinafter, as the second example, the drainage treatment (third control) under a humidifying purification condition in which the supply of water (second control) is executed 1 time and the supply of undiluted hypochlorous acid water (first control) is executed 8 times in a period in which the operating time after the operation start of air purifier 11 is up to 9 hours will be described.

Note that the humidifying purification condition described above is a condition set based on controlling air purifier 11 such that the number of times of performing the first control becomes larger than the number of times of performing the second control when the humidification requirement amount for air purifier 11 is less than the first reference value.

In the second example, as illustrated in (a) of Fig. 4, the supply of undiluted hypochlorous acid water (first control) to mixing tank 92 is executed at timings of 1 hour, 2 hours, 3 hours..., where the operation start is 0 hours. On the other hand, the supply of water (second control) to mixing tank 92 is executed at timings of a hours.... At the time point of 0 hours when the operation is started, the supply of the hypochlorous acid water and supply of the water to mixing tank 92 are executed, and mixing tank 92 is in a state (initial state) of being full with the hypochlorous acid water (mixed water) having a predetermined concentration.

The drainage determination of the mixed water stored in mixing tank 92 is performed immediately before the execution of the first control at the timings of 1 hour, 2 hours, 3 hours....

Specifically, at the timing of 1 hour when the operating time after the operation start becomes a period up to 1 hour (period when the operating time is greater than or equal to 0 hours and less than 1 hour), since neither the water nor the undiluted hypochlorous acid water is supplied to mixing tank 92, it is determined that the integrated humidification amount is less than the reference amount (2 times the capacity of mixing tank 92). Furthermore, the number of times of supply of the undiluted hypochlorous acid water by the first control is 0 times, and it is determined that the number of times of execution of the first control has not reached the reference number of times (11 times). In response to the determination result, the first control is executed, and mixing tank 92 is supplied with the undiluted hypochlorous acid water.

Next, the drainage determination of the mixed water is executed at the timing of 2 hours when the operating time after the operation start becomes a period of up to 2 hours (period in which the operating time is greater than or equal to 0 hours and less than 2 hours). In the humidifying purification operation so far, the integrated humidification amount becomes the supply amount (about 0.16 times the capacity of mixing tank 92) based on 1 time of the first control, and it is determined that the integrated humidification amount is less than the reference amount (2 times the capacity of mixing tank 92). Furthermore, the number of times of supply of the undiluted hypochlorous acid water by the first control is 1 time, and it is determined that the number of times of execution of the first control has not reached the reference number of times (11 times). In response to the determination result, the first control is executed, and mixing tank 92 is supplied with the undiluted hypochlorous acid water.

Thereafter, similar control is executed until the timing of 8 hours at which the operating time after the operation start becomes a period of up to 8 hours (period in which the operating time is from greater than or equal to 0 hours to less than 8 hours).

Subsequently, the drainage determination of mixed water is executed at the timing of 9 hours when the operating time after the operation start becomes a period up to 9 hours (period in which the operating time is greater than or equal to 0 hours and less than 9 hours). In the humidifying purification operation so far, the integrated humidification amount becomes the supply amount (about 2 times the capacity of mixing tank 92) based on 8 times of the first control and 1 time of the second control, and it is determined that the integrated humidification amount is greater than or equal to the reference amount (2 times the capacity of mixing tank 92). In response to the determination result, the third control is executed, and the mixed water in mixing tank 92 is drained. Furthermore, after the third control is executed, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state.

Thereafter, the timing of 9 hours is regarded as the initial state (0 hours), and the same supply behavior and drainage operation are repeated every period of 9 hours.

This will be described in more detail.

First, with reference to (a) of Fig. 4, a description will be given focusing on a temporal change in the water level of the hypochlorous acid water (mixed water) in mixing tank 92.

At the early stage of the operation (0 hours), mixing tank 92 is filled with mixed water (this is also hypochlorous acid water) of undiluted hypochlorous acid solution and water up to full. The humidifying purification operation reduces the water amount of the mixed water at a constant speed, and 1 hour, which is the supply timing of the hypochlorous acid water, elapses. Then, the drainage determination of the mixed water is executed at this timing of 1 hour.

In the humidifying purification operation so far, since neither the supply of water by the second control nor the supply of undiluted hypochlorous acid water by the first control is executed, it is determined that the integrated humidification amount is less than the reference amount (2 times the capacity of mixing tank 92). Furthermore, the number of times of supply of the undiluted hypochlorous acid water by the first control is 0 times, and it is determined that the number of times of execution of the first control has not reached the reference number of times (11 times). In response to the determination result, the first control is executed without the drainage of the mixed water stored in mixing tank 92 being executed, and the undiluted hypochlorous acid water is supplied from hypochlorous acid water generator 30 (hypochlorous acid water supplier 36) to mixing tank 92. Due to this, the water level in mixing tank 92 rises slightly. Also thereafter, the water level of the mixed water decreases by the humidifying purification operation, 2 hours, which is the supply timing of the hypochlorous acid water, elapses. Then, the drainage determination of the mixed water is executed at this timing of 2 hours.

In the humidifying purification operation so far, since the number of times of supply of undiluted hypochlorous acid water by the first control is 1 time, the integrated humidification amount becomes about 0.17 times (≈ about 1/6 times = about 1/6 × 1 time) the capacity of mixing tank 92, and it is determined that the integrated humidification amount is less than the reference amount (2 times the capacity of mixing tank 92). Furthermore, the number of times of supply of the undiluted hypochlorous acid water by the first control is 1 time, and it is determined that the number of times of execution of the first control has not reached the reference number of times (11 times). In response to the determination result, the first control is executed without the drainage of the mixed water stored in mixing tank 92 being executed, and the undiluted hypochlorous acid water is supplied from hypochlorous acid water generator 30 (hypochlorous acid water supplier 36) to mixing tank 92. Due to this, the water level in mixing tank 92 rises slightly. The humidifying purification operation reduces the water amount of the mixed water at a constant speed. When this is repeated 2 hours and thereafter, the water amount of the mixed water gradually decreases as a whole.

Thereafter, 8 hours is elapsed since the operation start, water shortage is detected at a timing when a hours has elapsed since the operation start, and water is supplied from water supplier 50 until mixing tank 92 becomes full.

Then, while the water level of the mixed water is reduced at a constant speed by the humidifying purification operation, 9 hours, which is the supply timing of the undiluted hypochlorous acid water, elapses. Then, the drainage determination of the mixed water is executed at this timing of 9 hours.

In the humidifying purification operation so far, since the number of times of supply of water by the second control is 1 time and the number of times of supply of undiluted hypochlorous acid water by the first control is 8 times, the integrated humidification amount becomes about 2 times the capacity of mixing tank 92 (= about 2/3 × 1 time + about 1/6 × 8 times), and it is determined that the integrated humidification amount is greater than or equal to the reference amount (2 times the capacity of mixing tank 92). Furthermore, the number of times of supply of the undiluted hypochlorous acid water by the first control is 8 times, and it is determined that the number of times of execution of the first control has not reached the reference number of times (11 times). In response to the determination result, the third control is executed, and the mixed water in mixing tank 92 is drained. Furthermore, after the execution of drainage of the mixed water by the third control, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state (0 hours). Here, the integrated humidification amount (the number of times of execution of the first control and the second control) is reset, and the storage of the integrated humidification amount is started again.

Thereafter, the timing of 9 hours is regarded as the initial state (0 hours), and the same supply behavior and drainage operation are repeated every period of 9 hours. More specifically, same as ever, supply of water by the second control at the timing when the water shortage occurs, and supply of undiluted hypochlorous acid water by the first control at the supply timing of hypochlorous acid water are repeated. Immediately before the execution of the first control, the drainage determination of the mixed water by the third control is performed, and the third control is executed when the condition is satisfied.

Next, with reference to (b) of Fig. 4, a description will be given focusing on a temporal change in the concentration of hypochlorous acid water (mixed water) in mixing tank 92.

At the beginning of the operation (0 hours), mixed water of undiluted hypochlorous acid water and water is mixed in mixing tank 92 so as to have a predetermined concentration (initial concentration). Then, when the humidifying purification operation is started, the concentration of the hypochlorous acid water (mixed water) in mixing tank 92 decreases with the elapse of time from the operation start up to 1 hour. This is because a constant proportion of hypochlorous acid with respect to the concentration of the hypochlorous acid water vaporizes and is given to the air due to the vapor pressure of hypochlorous acid being higher than that of water, as mentioned above.

Then, when 1 hour, which is the supply timing of the hypochlorous acid water, elapses from the operation start, the concentration of the hypochlorous acid water in mixing tank 92 rises to greater than or equal to the initial concentration along with supply of undiluted hypochlorous acid water from hypochlorous acid water generator 30 (hypochlorous acid water supplier 36). This is because, as described above, a predetermined amount of hypochlorous acid water (undiluted hypochlorous acid water) supplied at the initial stage of operation is supplied to mixed water (water in a state of containing hypochlorous acid) having a water amount smaller than that of the mixed water stored at the initial stage (0 hours) of the operation. Thereafter, the concentration of the hypochlorous acid water (mixed water) decreases by the vaporization of the hypochlorous acid up to 2 hours elapses from the operation start.

Then, when 2 hours, which is the supply timing of the hypochlorous acid water, elapses from the operation start, the concentration of the hypochlorous acid water in mixing tank 92 further rises to greater than or equal to the initial concentration along with supply of undiluted hypochlorous acid water from hypochlorous acid water generator 30 (hypochlorous acid water supplier 36). Thereafter, the concentration of hypochlorous acid water (mixed water) decreases by the vaporization of the hypochlorous acid until 3 hours elapses from the operation start. Similarly, until the timing of 8 hours thereafter, the concentration change of the hypochlorous acid water (mixed water) is repeated, and the concentration of the hypochlorous acid water (mixed water) gradually rises.

Then, when a hours elapses (water shortage detection) from the operation start, the hypochlorous acid water in mixing tank 92 is diluted with water along with supply of the water from water supplier 50, and therefore the concentration of the hypochlorous acid water in mixing tank 92 decreases. However, the concentration of the hypochlorous acid water in mixing tank 92 remains greater than or equal to the initial concentration. Thereafter, the concentration of the hypochlorous acid water (mixed water) slightly decreases by vaporization of the hypochlorous acid until 9 hours, which is the supply timing of the hypochlorous water, elapses.

When 9 hours, which is the supply timing of the undiluted hypochlorous acid water, elapses from the operation start, the drainage timing comes based on the drainage determination, therefore, after the hypochlorous acid water (mixed water) in mixing tank 92 is drained entirely, water and undiluted hypochlorous acid water are supplied into mixing tank 92, and the concentration of the hypochlorous acid water in mixing tank 92 is brought into a similar state to that in the initial stage of operation (0 hours). Thereafter, the concentration change of hypochlorous acid water (mixed water) is repeated in the same manner as ever.

Next, with reference to (c) of Fig. 4, a description will be given focusing on a temporal change in the concentration of the hypochlorous acid contained in air 9 at outlet 3.

Since the concentration of the hypochlorous acid contained in air 9 released from outlet 3 is determined by the humidification amount in air purifier 11 and the concentration of the hypochlorous acid water in mixing tank 92 as in winter in Japan, as illustrated in (c) of Fig. 4, the concentration of the hypochlorous acid contained in air 9 at outlet 3 increases or decreases in accordance with an increase or a decrease in the concentration of the hypochlorous acid water in mixing tank 92 illustrated in (b) of Fig. 4.

Here, as conventionally, in a case where undiluted hypochlorous acid water and water are supplied to make full every time water level sensor 90 detects water shortage, the concentration of the hypochlorous acid water continues to decrease from the operation start (0 hours) to 9 hours. In this case, the mean concentration of the hypochlorous acid contained in air 9 at outlet 3 becomes a conventional mean concentration, for example.

On the other hand, in the second example, the state from the operation start (0 hours) to 1 hour is the same as the conventional state, but the state in the period from 1 hour to 9 hours is different from the conventional state. More specifically, in the period from 1 hour to 9 hours, as illustrated in (b) of Fig. 4, the period in which the concentration of the hypochlorous acid water is higher than the initial concentration is far longer than the period in which it is lower than the initial concentration. Therefore, in the period from the operation start (0 hours) to 9 hours, the mean concentration of the hypochlorous acid contained in air 9 at outlet 3 becomes a mean concentration higher than the conventional mean concentration.

Next, the drainage treatment (third control) under a humidifying purification condition in which the humidification requirement amount is smaller than that in the second example in the behavior situation in summer in Japan will be described.

Therefore, hereinafter, as the third example, the drainage treatment (third control) under a humidifying purification condition in which the supply of water (second control) is not executed even 1 time in a period in which the operating time after the operation start of air purifier 11 is up to 12 hours will be described. That is, in the third example, only the supply of undiluted hypochlorous acid water (first control) is executed at predetermined time intervals.

In the third example, as illustrated in (a) of Fig. 5, the supply of undiluted hypochlorous acid water (first control) to mixing tank 92 is executed at timings of A hours, B hours, and C hours..., which are delayed from the timings of 1 hour, 2 hours, and 3 hours..., where the operation start is 0 hours. On the other hand, the supply of water (second control) to mixing tank 92 is not executed in a period of at least up to 12 hours. At the time point of 0 hours when the operation is started, the supply of the hypochlorous acid water and supply of the water to mixing tank 92 are executed, and mixing tank 92 is in a state (initial state) of being full with the hypochlorous acid water (mixed water) having a predetermined concentration.

Here, the reason why a delay occurs in the supply (first control) of the undiluted hypochlorous acid water to mixing tank 92 is that the humidification amount by air purifier 11 is small, the time during which the mixed water stored in mixing tank 92 is consumed has become longer than 1 hour, the mixed water is consumed by the humidifying purification treatment, and the supply is waited until the water amount in mixing tank 92 becomes less than the reference water amount (about 5/6 of the capacity of mixing tank 92). The reference water amount is set based on the supply amount (about 1/6 of the capacity of mixing tank 92) of the undiluted hypochlorous acid water. Note that the supply of the undiluted hypochlorous acid water in the third example is executed at the timings of A hours, B hours, and C hours..., which are delayed from the timings of 1 hour, 2 hours, and 3 hours..., and they shall be included in the "first control of performing supply of the hypochlorous acid water at predetermined time intervals" in the claims.

The drainage determination of the mixed water stored in mixing tank 92 is performed immediately before the execution of the first control at the timings of A hours, B hours, and C hours....

Specifically, at the timing of A hours when the operating time after the operation start becomes a period up to A hours (period when the operating time is greater than or equal to 0 hours and less than A hours) corresponding to 1 hour, since neither the water nor the undiluted hypochlorous acid water is supplied to mixing tank 92, it is determined that the integrated humidification amount is less than the reference amount (2 times the capacity of mixing tank 92). Furthermore, the number of times of supply of the undiluted hypochlorous acid water by the first control is 0 times" and it is determined that the number of times of execution of the first control has not reached the reference number of times (11 times). In response to the determination result, the first control is executed, and mixing tank 92 is supplied with the undiluted hypochlorous acid water. Note that since the undiluted hypochlorous acid water is supplied in a state of becoming substantially the reference water amount, mixing tank 92 is brought into a state of being full with supply of the undiluted hypochlorous acid water by the first control.

Next, the drainage determination of the mixed water is executed at the timing of B hours when the operating time after the operation start becomes a period of up to B hours (period in which the operating time is greater than or equal to 0 hours and less than B hours) corresponding to 2 hours. In the humidifying purification operation so far, the integrated humidification amount becomes the supply amount (about 0.16 times the capacity of mixing tank 92) based on 1 time of the first control, and it is determined that the integrated humidification amount is less than the reference amount (2 times the capacity of mixing tank 92). Furthermore, the number of times of supply of the undiluted hypochlorous acid water by the first control is 1 time, and it is determined that the number of times of execution of the first control has not reached the reference number of times (11 times). In response to the determination result, the first control is executed, and mixing tank 92 is supplied with the undiluted hypochlorous acid water.

Thereafter, similar control is executed until the timing of K hours at which the operating time after the operation start becomes a period of up to K hours (period in which the operating time is from greater than or equal to 0 hours to less than K hours) corresponding to 11 hours.

Subsequently, the drainage determination of mixed water is executed at the timing of L hours when the operating time after the operation start becomes a period up to L hours (period when the operating time is greater than or equal to 0 hours and less than L hours) corresponding to 12 hours. In the humidifying purification operation so far, the integrated humidification amount becomes the supply amount (about 1.8 times the capacity of mixing tank 92) based on 11 times of the first control, and it is determined that the integrated humidification amount is less than the reference amount (2 times the capacity of mixing tank 92). Furthermore, the number of times of supply of the undiluted hypochlorous acid water by the first control is 11 times, and it is determined that the number of times of execution of the first control is the reference number of times (11 times). In response to the determination result, the third control is executed, and the mixed water in mixing tank 92 is drained. Furthermore, after the third control is executed, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state.

Thereafter, the timing of L hours is regarded as the initial state (0 hours), and the same supply behavior and drainage operation are repeated every period of L hours.

This will be described in more detail.

First, with reference to (a) of Fig. 5, a description will be given focusing on a temporal change in the water level of the hypochlorous acid water (mixed water) in mixing tank 92.

At the early stage of the operation (0 hours), mixing tank 92 is filled with mixed water (this is also hypochlorous acid water) of undiluted hypochlorous acid solution and water up to full. The humidifying purification operation reduces the water amount of the mixed water at a constant speed, and A hours, which is the supply timing of the hypochlorous acid water, elapses. Then, the drainage determination of the mixed water is executed at this timing of A hours.

In the humidifying purification operation so far, since neither the supply of water by the second control nor the supply of undiluted hypochlorous acid water by the first control is executed, it is determined that the integrated humidification amount is less than the reference amount (2 times the capacity of mixing tank 92). Furthermore, the number of times of supply of the undiluted hypochlorous acid water by the first control is 0 times, and it is determined that the number of times of execution of the first control has not reached the reference number of times (11 times). In response to the determination result, the first control is executed without the drainage of the mixed water stored in mixing tank 92 being executed, and the undiluted hypochlorous acid water is supplied from hypochlorous acid water generator 30 (hypochlorous acid water supplier 36) to mixing tank 92. Due to this, the water level in mixing tank 92 rises to a full state. Also thereafter, the water level of the mixed water decreases by the humidifying purification operation, B hours, which is the supply timing of the hypochlorous acid water, elapses. Then, the drainage determination of the mixed water is executed at this timing of B hours.

In the humidifying purification operation so far, since the number of times of supply of undiluted hypochlorous acid water by the first control is 1 time, the integrated humidification amount becomes about 0.17 times (≈ about 1/6 times = about 1/6 × 1 time) the capacity of mixing tank 92, and it is determined that the integrated humidification amount is less than the reference amount (2 times the capacity of mixing tank 92). Furthermore, the number of times of supply of the undiluted hypochlorous acid water by the first control is 1 time, and it is determined that the number of times of execution of the first control has not reached the reference number of times (11 times). In response to the determination result, the first control is executed without the drainage of the mixed water stored in mixing tank 92 being executed, and the undiluted hypochlorous acid water is supplied from hypochlorous acid water generator 30 (hypochlorous acid water supplier 36) to mixing tank 92. Due to this, the water level in mixing tank 92 rises to a full state. Thus, in the third example, although the water amount of the mixed water decreases at a constant speed by the humidifying purification operation, the water amount of the mixed water only increases or decreases between the full state and the reference water amount.

Thereafter, L hours, which is the supply timing of the undiluted hypochlorous acid water, elapses, and the drainage determination of the mixed water is executed at this timing of L hours.

In the humidifying purification operation so far, since the number of times of supply of undiluted hypochlorous acid water by the first control is 11 times, the integrated humidification amount becomes about 1.8 times (≈ about 1/6 × 11 times) the capacity of mixing tank 92, and it is determined that the integrated humidification amount is less than the reference amount (2 times the capacity of mixing tank 92). Furthermore, it is determined that the number of times of supply of the undiluted hypochlorous acid water by the first control is 11, and the number of times of execution of the first control has reached the reference number of times (11 times). In response to the determination result, the third control is executed, and the mixed water in mixing tank 92 is drained. Furthermore, after the execution of drainage of the mixed water by the third control, the supply of undiluted hypochlorous acid water and the supply of water are newly executed to mixing tank 92, and mixing tank 92 is brought into a state of full with the hypochlorous acid water (mixed water) having a predetermined concentration that is the same as the initial state (0 hours). Here, the integrated humidification amount (the number of times of execution of the first control) is reset, and the storage of the integrated humidification amount is started again.

Thereafter, the timing of L hours is regarded as the initial state (0 hours), and the same supply behavior and drainage operation are repeated every period of L hours. More specifically, same as ever, supply of undiluted hypochlorous acid water by the first control at the supply timing of hypochlorous acid water is repeated. Immediately before the execution of the first control, the drainage determination of the mixed water by the third control is performed, and the third control is executed when the condition is satisfied. The water level of the hypochlorous acid water (mixed water) in mixing tank 92 increases or decreases corresponding to each behavior.

Next, with reference to (b) of Fig. 5, a description will be given focusing on a temporal change in the concentration of hypochlorous acid water (mixed water) in mixing tank 92.

At the beginning of the operation (0 hours), mixed water of undiluted hypochlorous acid water and water is mixed in mixing tank 92 so as to have a predetermined concentration (initial concentration). Then, when the humidifying purification operation is started, the concentration of the hypochlorous acid water (mixed water) in mixing tank 92 decreases with the elapse of time from the operation start up to A hours. This is because a constant proportion of hypochlorous acid with respect to the concentration of the hypochlorous acid water vaporizes and is given to the air due to the vapor pressure of hypochlorous acid being higher than that of water, as mentioned above.

Then, when A hours, which is the supply timing of the hypochlorous acid water, elapses from the operation start, the concentration of the hypochlorous acid water in mixing tank 92 rises to greater than or equal to the initial concentration along with supply of undiluted hypochlorous acid water from hypochlorous acid water generator 30 (hypochlorous acid water supplier 36). This is because, as described above, a predetermined amount of hypochlorous acid water (undiluted hypochlorous acid water) supplied at the initial stage of operation is supplied to mixed water (water in a state of containing hypochlorous acid) having a water amount smaller than that of the mixed water stored at the initial stage (0 hours) of the operation. Thereafter, the concentration of the hypochlorous acid water (mixed water) decreases by the vaporization of the hypochlorous acid up to B hours elapses from the operation start.

Then, when B hours, which is the supply timing of the hypochlorous acid water, elapses from the operation start, the concentration of the hypochlorous acid water in mixing tank 92 further rises to greater than or equal to the initial concentration along with supply of undiluted hypochlorous acid water from hypochlorous acid water generator 30 (hypochlorous acid water supplier 36). Thereafter, the concentration of hypochlorous acid water (mixed water) decreases by the vaporization of the hypochlorous acid until C hours elapses from the operation start. Similarly, until the timing of K hours thereafter, the concentration change of the hypochlorous acid water (mixed water) is repeated, and the concentration of the hypochlorous acid water (mixed water) gradually rises.

When L hours, which is the supply timing of the undiluted hypochlorous acid water, elapses from the operation start, the drainage timing comes based on the drainage determination, therefore, after the hypochlorous acid water (mixed water) in mixing tank 92 is drained entirely, water and undiluted hypochlorous acid water are supplied into mixing tank 92, and the concentration of the hypochlorous acid water in mixing tank 92 is brought into a similar state to that in the initial stage of operation (0 hours). Thereafter, the concentration change of hypochlorous acid water (mixed water) is repeated in the same manner as ever.

Next, with reference to (c) of Fig. 5, a description will be given focusing on a temporal change in the concentration of the hypochlorous acid contained in air 9 at outlet 3.

Since the concentration of the hypochlorous acid contained in air 9 released from outlet 3 is determined by the humidification amount in air purifier 11 and the concentration of the hypochlorous acid water in mixing tank 92 as in winter in Japan, as illustrated in (c) of Fig. 5, the concentration of the hypochlorous acid contained in air 9 at outlet 3 increases or decreases in accordance with an increase or a decrease in the concentration of the hypochlorous acid water in mixing tank 92 illustrated in (b) of Fig. 5.

Here, as conventionally, in a case where undiluted hypochlorous acid water and water are supplied to make full every time water level sensor 90 detects water shortage, the concentration of the hypochlorous acid water continues to decrease from the operation start (0 hours) to L hours. In this case, the mean concentration of the hypochlorous acid contained in air 9 at outlet 3 becomes a conventional mean concentration, for example.

On the other hand, in the third example, the state from the operation start (0 hours) to A hours is the same as the conventional state, but the state in the period from A hours to L hours is different from the conventional state. More specifically, in the period from A hours to L hours, as illustrated in (b) of Fig. 5, the period in which the concentration of the hypochlorous acid water is higher than the initial concentration is far longer than the period in which it is lower than the initial concentration. Therefore, in the period from the operation start (0 hours) to 12 hours, the mean concentration of the hypochlorous acid contained in air 9 at outlet 3 becomes a mean concentration higher than the conventional mean concentration.

Then, also for L hours and thereafter, the concentration change of the mixed water is repeated every L hours with L hours as 1 cycle, and it is therefore possible to keep adjusting the concentration of the hypochlorous acid water within a range of less than or equal to a certain concentration without the concentration of the hypochlorous acid water keeping rising. That is, when the humidifying purification operation is continued, the concentration of the hypochlorous acid water in mixing tank 92 may excessively rise, however, by providing drainage determination control in accordance with the number of times of supply of the undiluted hypochlorous acid water by the first control, it is possible to reset the concentration of the hypochlorous acid water in mixing tank 92 at regular intervals, and eventually the amount of hypochlorous acid contained in air 9 at outlet 3, and it is possible to control the supply amount of a hypochlorous acid gas to indoor space 18.

As described above, space purification system 100 supplies hypochlorous acid water into mixing tank 92 every preset time (e.g., 1 hour) as the first control, executes the treatment of supplying water based on the water level information (water shortage signal) from water level sensor 90 as the second control, and drains the mixed water in mixing tank 92 based on the integrated humidification amount or the number of times of execution of the first control as the third control. Furthermore, air purification control unit 41 of space purification system 100 makes the number of times of performing the first control within a predetermined period different from the number of times of performing the second control within a predetermined period based on the humidification requirement amount (a humidification requirement amount corresponding to winter in Japan or a humidification requirement amount corresponding to summer in Japan) required to air purifier 11. Due to this, in a state where the humidification requirement amount is high as in winter in Japan, air 9 in a state where the content of the hypochlorous acid amount is small can be released to indoor space 18 as compared with the conventional method, and in a state where the humidification requirement amount is low as in summer in Japan, air 9 in a state where the content of the hypochlorous acid amount is large can be released to indoor space 18 as compared with the conventional method. Furthermore, when the humidifying purification operation is continued for a long time, an excessive increase in the hypochlorous acid concentration released to indoor space 18 can be suppressed.

That is, by operating the supply of hypochlorous acid water, the supply of water, and the drainage of mixed water by triggers separate from one another, it is possible to adjust the concentration of the hypochlorous acid water in mixing tank 92 (concentration of the hypochlorous acid contained in air 9 blown out to indoor space 18) by simple control (first control, second control, and third control).

As described above, space purification system 100 according to the present first exemplary embodiment can achieve the following effects.
(1) Space purification system 100 includes hypochlorous acid water generator 30 that generates hypochlorous acid water, hypochlorous acid water supplier 36 that supplies hypochlorous acid water from hypochlorous acid water generator 30 to mixing tank 92, water supplier 50 that supplies water to mixing tank 92, water level sensor 90 for detecting a water level of mixing tank 92, air purifier 11 that micronizes and releases, into air, mixed water of the hypochlorous acid water and the water stored in mixing tank 92, and air purification control unit 41 that controls supply treatment in hypochlorous acid water supplier 36 and water supplier 50 and drainage treatment of the mixed water stored in mixing tank 92. Air purification control unit 41 (i) executes, as the control of the supply treatment, first control of causing the hypochlorous acid water supplier 36 to perform supply of the hypochlorous acid water at predetermined time intervals (e.g., 60 minutes), and second control of causing the water supplier 50 to perform supply of the water based on information (water shortage information) regarding the water level of mixing tank 92 from water level sensor 90, and (ii) executes, as the control of the drainage treatment, third control of causing the mixing tank to drain the mixed water stored in mixing tank 92 based on an integrated humidification amount in air purifier 11.

Due to this, when air having high relative humidity is ventilated as in summer in Japan, since the consumption amount of the mixed water stored in mixing tank 92 is small, the supply frequency (the number of times of performing the first control) of hypochlorous acid water to mixing tank 92 increases, and the mixed water is micronized and released into the air in a state where the hypochlorous acid concentration of the mixed water in mixing tank 92 is high. Since the consumption amount of the mixed water stored in mixing tank 92 is small, the frequency of drainage of the mixed water (the number of times of performing the third control) decreases, and the hypochlorous acid concentration of the mixed water in mixing tank 92 is maintained in a high state. As a result, even in a situation where the micronized hypochlorous acid water hardly vaporize, hypochlorous acid raised to a predetermined concentration contained in the air can be released into indoor space 18.

On the other hand, when air having low relative humidity is ventilated as in winter in Japan, since the consumption amount of the mixed water stored in mixing tank 92 is large, the supply frequency of water to mixing tank 92 (the number of times of performing the second control) increases, and the mixed water is micronized and released into the air in a state where the hypochlorous acid concentration of the mixed water in mixing tank 92 is low. Since the consumption amount of the mixed water stored in mixing tank 92 is large, the frequency of drainage of the mixed water in mixing tank 92 (the number of times of performing the third control) increases, and it is possible to suppress an excessive increase in the hypochlorous acid concentration of the mixed water. As a result, even in a situation where the micronized hypochlorous acid water easily vaporizes, hypochlorous acid attenuated to a predetermined concentration contained in the air can be released into indoor space 18.

That is, space purification system 100 can easily adjust the amount of hypochlorous acid to be released into the air.

(2) In space purification system 100, air purification control unit 41 executes the third control when the integrated humidification amount becomes greater than or equal to a reference amount. This enables space purification system 100 to easily adjust the concentration of the hypochlorous acid water stored in mixing tank 92 based on the humidification amount in air purifier 11.

(3) In space purification system 100, the integrated humidification amount is calculated based on the number of times of execution of the first control and the second control. This enables space purification system 100 to simply and accurately calculate the integrated humidification amount, and improve the controllability of the third control.

(4) In space purification system 100, air purification control unit 41 executes the third control when the number of times of performing the first control becomes a reference number of times. This enables space purification system 100 to return the state in mixing tank 92 to the operation initial state by executing the third control of draining the mixed water stored in mixing tank 92 before the hypochlorous acid water concentration in mixing tank 92 excessively increases even when a long time operation (e.g., 24 hours) is performed. That is, space purification system 100 can easily adjust the amount of hypochlorous acid to be released into the air.

(5) In space purification system 100, air purification control unit 41 executes the third control immediately before executing the first control. Due to this, in space purification system 100, since drainage by the third control is no longer performed immediately after the hypochlorous acid is supplied to mixing tank 92 by the first control, the hypochlorous acid water supplied by the first control can be continuously used maximally and waste due to the drainage by the third control can be reduced.

Here, air purification control unit 41 may execute the third control immediately before executing the second control in addition to the first control, or may execute the third control only immediately before executing the second control. Also by doing this, in space purification system 100, since drainage by the third control is no longer performed immediately after the hypochlorous acid is supplied to mixing tank 92 by the first control or immediately after the water is supplied by the second control, the hypochlorous acid water supplied by the first control or the water supplied by the second control can be continuously used maximally and waste due to the drainage by the third control can be reduced.

(6) In space purification system 100, in the control of the supply treatment, air purification control unit 41 (i) performs the first control and the second control such that the number of times of performing the first control becomes smaller than the number of times of performing the second control when the humidification requirement amount required for air purifier 11 is greater than or equal to a first reference value, and (ii) performs the first control and the second control such that the number of times of performing the first control becomes larger than the number of times of performing the second control when the humidification requirement amount is less than the first reference value. This enables space purification system 100 can micronize and release, into the air, the mixed water in a state where the hypochlorous acid concentration in mixing tank 92 is high when the humidification requirement amount is less than the first reference value in the supply treatment. On the other hand, the mixed water can be micronized and released into the air in a state where the hypochlorous acid concentration in mixing tank 92 is low when the humidification requirement amount is greater than or equal to the first reference value. In other words, based on the humidification requirement amount, space purification system 100 can give hypochlorous acid to air 9 released from air purifier 11 under a condition suitable for the environment of indoor space 18.

The present disclosure has been described above based on the exemplary embodiments. It is understood by those skilled in the art that these exemplary embodiments are merely examples, that the components or the treatment processes of those can be combined as various modifications, and that such modifications also fall within the scope of the present disclosure.

In the first example, the second example, and the third example in space purification system 100 according to the present first exemplary embodiment, it has been described that air purifier 11 behaves at a constant humidification requirement amount during the humidifying purification operating time, but actually, air purifier 11 behaves at a humidification requirement amount specified based on a humidity difference between the target humidity and the humidity of the air in indoor space 18 every constant time.

Space purification system 100 according to the present exemplary embodiment calculates the integrated humidification amount based on the number of times of execution of the first control and the number of times of execution of the second control, but the present invention is not limited to this. For example, by temperature and humidity sensors provided before and after the air passage of space purification device 10, the integrated humidification amount may be calculated from the change amount of temperature and humidity obtained from the temperature and humidity sensors.

In space purification system 100 according to the present exemplary embodiment, the reference number of times is set to 11 times, which is immediately before the reference amount is reached only by the supply of hypochlorous acid water by the first control, but the present invention is not limited to this. For example, the reference number of times may be set based on the concentration of the hypochlorous acid water supplied by the first control, that is, the concentration of the hypochlorous acid water generated in hypochlorous acid water generator 30. This can drain the mixed water stored in mixing tank 92 before the hypochlorous acid water concentration in mixing tank 92 excessively increases.

### (Second exemplary embodiment)

As a conventional space purification device, an air-conditioning system that disinfects a space by bringing air to be supplied indoors into contact with a gas-liquid contact member containing a purification component and releasing the air is known (see, for example, PTL 1).

In such conventional space purification device, generally, in addition to release of micronized water, water stored in the device (water containing a purification component) contains a part of the purification component along with micronization behavior, and the water and the purification component are vaporized and released into a space.

With the conventional space purification device, however, in a situation where a humidification amount required for an indoor space is small, for example, in summer season in Japan (rainy season in particular), when air dehumidified by an air-conditioner or the like and having high relative humidity (e.g., 12 °C 95%) is ventilated, water (hypochlorous acid water) containing a micronized purification component is hardly vaporized, therefore the purification component (hypochlorous acid) is not vaporized, and the purification component is hardly released into the indoor space. On the other hand, in a situation where the required humidification amount is large, for example, in winter in Japan, when heated air having low relative humidity (e.g., 20 °C 30%) is ventilated, water containing the micronized purification component is easily vaporized, and therefore a large amount of purification component is released into the indoor space. In other words, the conventional space purification device has a problem that it is not easy to adjust the amount of the purification component released into the indoor space (into the air).

Therefore, the present disclosure has been made to solve the above-described conventional problems, and an object of the present disclosure is to provide a technique that can easily adjust the amount of a purification component released into air.

In order to achieve this object, a space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank when the first control is continuously executed a predetermined number of times, thereby achieving the intended object.

The space purification system according to the present disclosure can easily adjust the amount of the purification component released into air.

Again, a space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank when the first control is continuously executed a predetermined number of times.

By doing this, when air having high relative humidity is ventilated as in summer in Japan, since the consumption amount of mixed water stored in the mixing tank is small, the supply frequency (the number of times of performing the first control) of the hypochlorous acid water to the mixing tank increases, and the mixed water is micronized and released into the air in a state where the hypochlorous acid concentration of the mixed water in the mixing tank is high. At this time, by executing the third control when continuously executing the first control a predetermined number of times, draining the mixed water stored in the mixing tank, and resetting the mixed water in the mixing tank, it is possible to suppress an excessive increase in the hypochlorous acid concentration in the mixing tank. As a result, even in a situation where the micronized hypochlorous acid water hardly vaporize, hypochlorous acid raised to a predetermined concentration contained in the air can be released into the indoor space. On the other hand, when air having low relative humidity is ventilated as in winter in Japan, since the consumption amount of the mixed water stored in the mixing tank is large, the supply frequency of water to the mixing tank (the number of times of performing the second control) increases, and the mixed water is micronized and released into the air in a state where the hypochlorous acid concentration of the mixed water in the mixing tank is low. As a result, even in a situation where the micronized hypochlorous acid water easily vaporizes, hypochlorous acid attenuated to a predetermined concentration contained in the air can be released into the indoor space. That is, the space purification system can easily adjust the amount of hypochlorous acid to be released into the air.

In the space purification system according to the present disclosure, the control unit preferably executes the third control immediately before executing the first control after continuously executing the first control a predetermined number of times. Due to this, in the space purification system, since drainage by the third control is no longer performed immediately after the hypochlorous acid is supplied to the mixing tank by the first control, the hypochlorous acid water supplied by the first control can be continuously used maximally and waste due to the drainage by the third control can be reduced.

In the space purification system according to the present disclosure, the control unit preferably sets the predetermined number of times in the third control based on the concentration of the hypochlorous acid water supplied by the first control. Due to this, for example, in the space purification system, when the concentration of the hypochlorous acid water supplied by the first control is high, the increase in the concentration of the hypochlorous acid water in the mixing tank becomes fast in a case where the first control is continuously executed a predetermined number of times, and therefore it is possible to more reliably suppress the excessive increase in the concentration of the hypochlorous acid water in the mixing tank by setting the predetermined number of times to be small.

Modes for carrying out the present disclosure will now be described with reference to the accompanying drawings. Note that the following exemplary embodiments are examples embodying the present disclosure, and do not limit the technical scope of the present disclosure in any way. Throughout the drawings, identical parts are denoted by the identical reference marks, and descriptions thereof will be omitted. Furthermore, details of each part not directly related to the present disclosure are not described for each drawing to avoid redundancy.

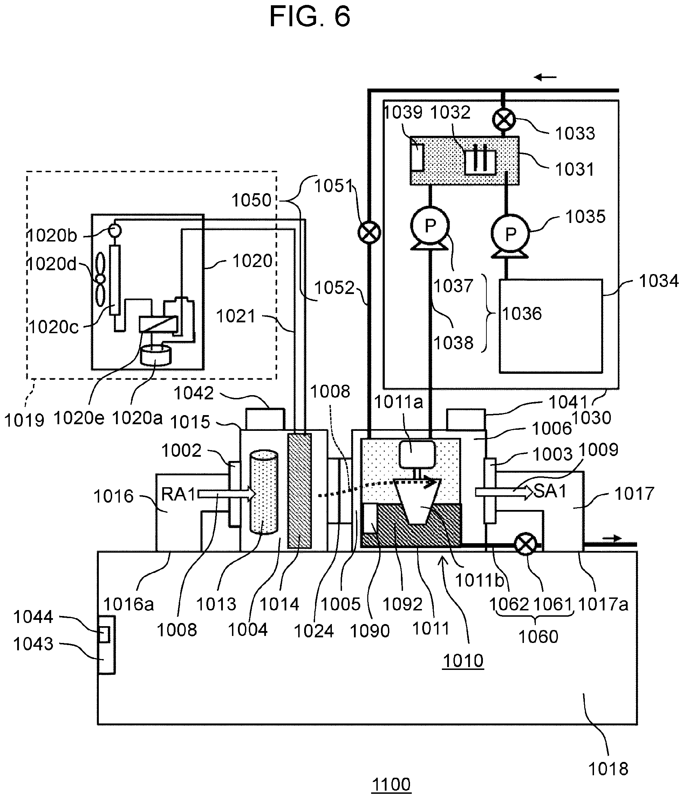

Fig. 6 is a view illustrating the configuration of space purification system 1100 according to the second exemplary embodiment of the present disclosure. Space purification system 1100 is a device that performs cooling treatment (dehumidification treatment) or heating treatment on air 1008 (RA1) from indoor space 1018 as necessary when circulating the air of indoor space 1018, and causes air 1008 flowing inside to contain a component (hereinafter, simply called "air purification component") that purifies air together with micronized water. Space purification system 1100 performs sterilization and deodorization of indoor space 1018 by supplying indoor space 1018 with air 1009 (SA1) having circulated inside. Here, hypochlorous acid is used as the air purification component, and the water containing the air purification component is hypochlorous acid water.

As illustrated in Fig. 6, space purification system 1100 is mainly configured to include space purification device 1010, air-conditioning device 1015, and hypochlorous acid water generator 1030.

Space purification device 1010 includes outlet 1003, air purifier 1011, and air purification control unit 1041. Air-conditioning device 1015 includes inlet 1002, air blower 1013, refrigerant coil 1014, and air-conditioning control unit 1042. Each of space purification device 1010 and air-conditioning device 1015 has a housing constituting an outer frame of the respective device, and space purification device 1010 and air-conditioning device 1015 are connected via duct 1024. Inlet 1002 is formed on a side surface of air-conditioning device 1015, and outlet 1003 is formed on a side surface of space purification device 1010.

Inlet 1002 is an intake port for taking in air 1008 from indoor space 1018 into air-conditioning device 1015. Inlet 1002 is communicated via duct 1016 with indoor inlet 1016a provided on a ceiling or the like of indoor space 1018. Thus, inlet 1002 enables the air of indoor space 1018 to be suctioned into air-conditioning device 1015 via indoor inlet 1016a.

Outlet 1003 is a discharge port through which air 1009 (SA1) having circulated in space purification device 1010 is discharged to indoor space 1018. Outlet 1003 is communicated via duct 1017 with indoor outlet 1017a provided on a ceiling or the like of indoor space 1018. Thus, outlet 1003 enables air 1009 having circulated in space purification device 1010 to be blown out toward indoor space 1018 from indoor outlet 1017a.

Inside air-conditioning device 1015 and space purification device 1010 is provided with an air passage (prior stage air passage 1004, middle stage air passage 1005, and subsequent stage air passage 1006) causing inlet 1002 and outlet 1003 to be communicated each other via duct 1024. Prior stage air passage 1004 is an air passage adjacent to inlet 1002. Prior stage air passage 1004 is provided with air blower 1013 and refrigerant coil 1014.

Middle stage air passage 1005 is an air passage through which air 1008 having circulated through prior stage air passage 1004 circulates, at a position adjacent to prior stage air passage 1004 (duct 1024). Inside middle stage air passage 1005 is provided with air purifier 1011.

Subsequent stage air passage 1006 is an air passage adjacent to outlet 1003, and in subsequent stage air passage 1006, air 1008 having circulated through middle stage air passage 1005 circulates through air purifier 1011 and becomes air 1009 containing hypochlorous acid together with micronized water.

In air-conditioning device 1015 and space purification device 1010, air 1008 having been suctioned in from inlet 1002 circulates through prior stage air passage 1004, circulates through middle stage air passage 1005 and subsequent stage air passage 1006, and is blown out from outlet 1003 as air 1009.

Air blower 1013 of air-conditioning device 1015 is a device for conveying air 1008 (RA1) in indoor space 1018 from inlet 1002 into air-conditioning device 1015. Air blower 1013 is installed upstream of refrigerant coil 1014 in prior stage air passage 1004. In air blower 1013, on/off of operation behavior is controlled in accordance with blower output information from air-conditioning control unit 1042. Due to operation behavior of air blower 1013, air 1008 in indoor space 1018 is taken in to air-conditioning device 1015, and directed to refrigerant coil 1014.

Refrigerant coil 1014 is a member that is disposed downstream of air blower 1013 in prior stage air passage 1004 and cools or heats air 1008 to be introduced. Refrigerant coil 1014 changes an output state (cooling, heating, or off) in response to an output signal from air-conditioning control unit 1042, and adjusts the cooling capacity (cooling amount) or the heating capacity (heating amount) for air 1008 to be introduced. When refrigerant coil 1014 cools air 1008 to be introduced, air 1008 having been introduced is dehumidified, and therefore, the cooling capacity (cooling amount) for air 1008 can be deemed also as the dehumidifying capacity for air 1008 (dehumidification amount).

Refrigerant coil 1014 functions as a heat absorber or a heat radiator in a refrigeration cycle configured to include a compressor, a heat radiator, an expander, and a heat absorber, and is configured to absorb heat (cooling) or radiate heat (heating) when the refrigerant introduced from condenser unit 1020 circulates inside. More specifically, refrigerant coil 1014 is connected to condenser unit 1020 via refrigerant circuit 1021 through which a refrigerant flows. Condenser unit 1020 is an outdoor unit installed in outdoor space 1019, and includes compressor 1020a, expander 1020b, outdoor heat exchanger 1020c, blower fan 1020d, and four-way valve 1020e. Since condenser unit 1020 with a general configuration is used, detailed descriptions of these devices (compressor 1020a, expander 1020b, outdoor heat exchanger 1020c, blower fan 1020d, and four-way valve 1020e) will be omitted.

Since four-way valve 1020e is connected to the refrigeration cycle including refrigerant coil 1014, air-conditioning device 1015 can switch between a state of a cooling mode (dehumidification mode) in which the refrigerant circulates in a first direction by four-way valve 1020e and cools and dehumidifies the air (air 1008) and a state of a heating mode in which the refrigerant circulates in a second direction by four-way valve 1020e and heats the air (air 1008).

Here, the first direction is a direction in which the refrigerant circulates through compressor 1020a, outdoor heat exchanger 1020c, expander 1020b, and refrigerant coil 1014 in this order. The second direction is a direction in which the refrigerant circulates through compressor 1020a, refrigerant coil 1014, expander 1020b, and outdoor heat exchanger 1020c in this order. Refrigerant coil 1014 can cool or heat the air (air 1008) to be introduced.

Air purifier 1011 of space purification device 1010 is a unit for humidifying air 1008 taken therein, and causes hypochlorous acid together with micronized water to be contained the air during humidification. More specifically, air purifier 1011 includes mixing tank 1092, water level sensor 1090, humidifying motor 1011a, and humidifying nozzle 1011b. Air purifier 1011 has a centrifugal crushing type configuration in which humidifying nozzle 1011b is rotated using humidifying motor 1011a, the hypochlorous acid water stored in mixing tank 1092 of air purifier 1011 is suctioned by a centrifugal force, scattered, collided, and crushed in the surroundings (centrifugal direction), and moisture is contained in the passing air. Air purifier 1011 adjusts the humidifying capacity (humidification amount) by changing the rotation speed (hereinafter, rotation output value) of humidifying motor 1011a in response to an output signal from air purification control unit 1041. The humidification amount can be deemed also to be an addition amount by which the hypochlorous acid is added to the air. Note that air purifier 1011 corresponds to the "humidifying purifier" in the claims.

Water level sensor 1090 measures the water level of the hypochlorous acid water stored in mixing tank 1092, and outputs a measurement value to air purification control unit 1041. More specifically, water level sensor 1090 measures, as the water level of the hypochlorous acid water stored in mixing tank 1092, each of the water level at which mixing tank 1092 is brought into a water shortage state and the water level at which mixing tank 1092 is brought into a full state, and outputs the measurement values to air purification control unit 1041 as water level information. Note that in the present exemplary embodiment, the water level at which mixing tank 1092 is brought into a water shortage state is set to a water level in a state where the hypochlorous acid water amount has dropped to about 1/3 from the state where the hypochlorous acid water (mixed water) is full in mixing tank 1092.

Mixing tank 1092 is a tank in which hypochlorous acid water is stored in air purifier 1011, and can be deemed also to be a water storage. In mixing tank 1092, hypochlorous acid water with a predetermined concentration supplied from hypochlorous acid water supplier 1036 described later is mixed with the water supplied from water supplier 1050 described later, and is stored as mixed water including diluted hypochlorous acid water. Note that the hypochlorous acid water (mixed water) stored in mixing tank 1092 can be drained from mixing tank 1092 to the outside by drainage 1060 that behaves in response to an output signal from air purification control unit 1041.

Hypochlorous acid water generator 1030 includes electrolytic tank 1031, electrodes 1032, electromagnetic valve 1033, brine tank 1034, brine conveyance pump 1035, water level sensor 1039, and hypochlorous acid water supplier 1036.

Brine tank 1034 stores brine, and supplies the brine to electrolytic tank 1031 via brine conveyance pump 1035 in response to an output signal from air purification control unit 1041. Electrolytic tank 1031 stores the brine that is an electrolysis target supplied from brine tank 1034. Electrolytic tank 1031 is also supplied with tap water from a water supply pipe such as water supply via electromagnetic valve 1033 in response to an output signal from air purification control unit 1041, the supplied tap water and brine are mixed, and brine having a predetermined concentration is stored therein. Electrodes 1032 are disposed in electrolytic tank 1031, performs electrolysis of brine for a predetermined time by energization in response to an output signal from air purification control unit 1041, and generates hypochlorous acid water having a predetermined concentration. That is, electrolytic tank 1031 generates hypochlorous acid water by performing electrolysis of an aqueous chloride solution (e.g., aqueous sodium chloride solution) as an electrolyte between a pair of electrodes. Since a general device is used as electrolytic tank 1031, a detailed description will be omitted. Here, the electrolyte is an electrolyte from which hypochlorous acid water can be generated, and is not particularly limited as long as it contains chloride ions even in a small amount, and examples of the electrolyte include an aqueous solution in which sodium chloride, calcium chloride, magnesium chloride, or the like is dissolved as a solute. Hydrochloric acid is also acceptable. In the present exemplary embodiment, an aqueous sodium chloride solution (brine) in which sodium chloride is added to water is used as the electrolyte.

Water level sensor 1039 measures the water level in electrolytic tank 1031, and outputs the measurement value to air purification control unit 1041.

Hypochlorous acid water supplier 1036 supplies hypochlorous acid water from electrolytic tank 1031 into mixing tank 1092 of air purifier 1011 in response to an output signal from air purification control unit 1041. Hypochlorous acid water supplier 1036 includes hypochlorous acid water conveyance pump 1037 and water conveyance pipe 1038. Hypochlorous acid water conveyance pump 1037 feeds the hypochlorous acid water in electrolytic tank 1031 to water conveyance pipe 1038 in response to an output signal from air purification control unit 1041. Water conveyance pipe 1038 is connected between hypochlorous acid water conveyance pump 1037 and mixing tank 1092, and conveys the hypochlorous acid water to mixing tank 1092.

Water supplier 1050 supplies water to mixing tank 1092 in response to an output signal from air purification control unit 1041. Water supplier 1050 includes electromagnetic valve 1051 and water conveyance pipe 1052. Electromagnetic valve 1051 controls as to whether or not to flow, to water conveyance pipe 1052, the water supplied from a water supply pipe external of space purification device 1010 in response to an output signal from air purification control unit 1041. Water conveyance pipe 1052 is connected between electromagnetic valve 1051 and mixing tank 1092, through which water is conveyed to mixing tank 1092.

Drainage 1060 is connected to a bottom of mixing tank 1092, and drains the mixed water stored in mixing tank 1092 to the outside in response to an output signal from air purification control unit 1041. Drainage 1060 includes electromagnetic valve 1061 and water conveyance pipe 1062. Electromagnetic valve 1061 controls whether or not to flow the mixed water stored in mixing tank 1092 to the external drainage pipe in response to an output signal from air purification control unit 1041. Water conveyance pipe 1062 is connected between mixing tank 1092 and electromagnetic valve 1061, and supplies mixed water to the external drainage pipe.

In air purifier 1011, the hypochlorous acid water from hypochlorous acid water supplier 1036 and the water from water supplier 1050 are supplied to mixing tank 1092. The hypochlorous acid water and the water are mixed in mixing tank 1092 of air purifier 1011. Mixed water of hypochlorous acid water and water can also be called hypochlorous acid water. More specifically, in mixing tank 1092 of air purifier 1011, the hypochlorous acid water from hypochlorous acid water supplier 1036 or the water from water supplier 1050 is supplied to and mixed with the hypochlorous acid water remaining in mixing tank 1092. Air purifier 1011 releases the hypochlorous acid water to indoor space 1018 by centrifugally crushing the mixed water of the hypochlorous acid water and the water stored in mixing tank 1092. The micronized hypochlorous acid water is released to indoor space 1018 in a state where the liquid component is evaporated.

Operation device 1043 is installed on a wall surface of indoor space 1018. Operation device 1043 includes a user interface that a user can operate, and receives a temperature setting value and a humidity setting value from the user. Operation device 1043 includes temperature and humidity sensor 1044, and temperature and humidity sensor 1044 measures the temperature and humidity of air in indoor space 1018. It is sufficient to use a known technology for temperature and humidity sensor 1044 to measure the temperature and humidity, and therefore, a description is omitted here.

Operation device 1043 is connected to air purification control unit 1041 and air-conditioning control unit 1042 in a wired or wireless manner, and transmits a temperature setting value, a humidity setting value, a temperature measurement value, and a humidity measurement value to air purification control unit 1041 and air-conditioning control unit 1042. These pieces of information may all be transmitted together, any two or more may be transmitted together, or each may be transmitted. Operation device 1043 may transmit information to air purification control unit 1041, and air purification control unit 1041 may transfer information to air-conditioning control unit 1042.

Air-conditioning control unit 1042 of air-conditioning device 1015 receives the temperature setting value and the temperature measurement value, and controls refrigerant coil 1014 and condenser unit 1020 so that the temperature measurement value approaches the temperature setting value. Air-conditioning control unit 1042 increases the degree of heating as a difference between the temperature measurement value and the temperature setting value increases when the temperature measurement value is lower than the temperature setting value in the heating mode.

Next, air purification control unit 1041 of space purification device 1010 will be explained.

As treatment operations of hypochlorous acid water generator 1030 and space purification device 1010, air purification control unit 1041 controls each of a behavior regarding the electrolysis treatment in electrolytic tank 1031, a behavior regarding the supply treatment of hypochlorous acid water to air purifier 1011, a behavior regarding the supply treatment of water to air purifier 1011, a behavior regarding the humidifying purification treatment in air purifier 1011, and a behavior regarding the drainage treatment of the mixed water in air purifier 1011. Note that air purification control unit 1041 includes a computer system including a processor and a memory. The computer system functions as a control unit by the processor executing a program stored in the memory. The program to be executed by the processor is assumed here to be recorded in advance in the memory of the computer system, but may be provided in a manner being recorded in a non-transitory recording medium such as a memory card, or provided through a telecommunication line such as the Internet. Air purification control unit 1041 corresponds to the "control unit" in the claims.

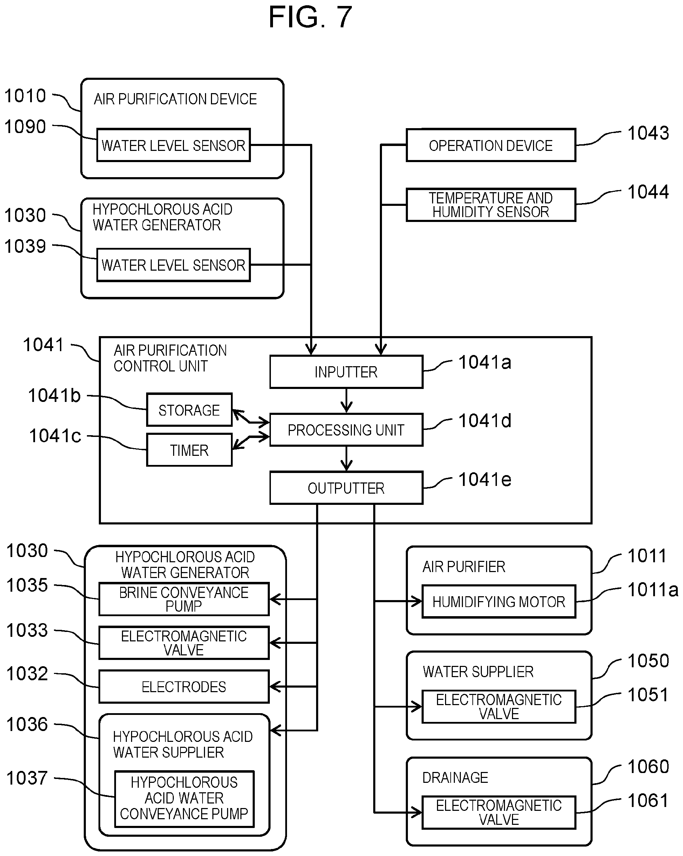

Fig. 7 is a block diagram illustrating the configuration of air purification control unit 1041 of space purification system 1100 according to the second exemplary embodiment. Specifically, as illustrated in Fig. 7, air purification control unit 1041 includes inputter 1041a, storage 1041b, timer 1041c, processing unit 1041d, and outputter 1041e.

### <Behavior regarding electrolysis treatment in electrolytic tank>

Air purification control unit 1041 executes the following treatment as a behavior regarding the electrolysis treatment in electrolytic tank 1031.

As a trigger of the electrolysis treatment of electrolytic tank 1031, air purification control unit 1041 receives and outputs, to processing unit 1041d, water level information (water shortage signal) from water level sensor 1039 and information regarding time (time information) from timer 1041c.

Processing unit 1041d specifies and outputs, to outputter 1041e, control information based on the water level information from water level sensor 1039, the time information from timer 1041c, and the setting information from storage 1041b. Here, the setting information includes information regarding start time or end time of hypochlorous acid water generation, information regarding a supply amount of tap water to be introduced into electrolytic tank 1031, information regarding an input amount of a liquid containing chloride ions in brine conveyance pump 1035, information regarding electrolysis conditions (such as time, current value, and voltage) in electrodes 1032, information regarding opening/closing timing of electromagnetic valve 1033, and information regarding on/off behavior of hypochlorous acid water conveyance pump 1037.

The electrolysis conditions in electrodes 1032 can be determined based on the water amount of tap water in electrolytic tank 1031, the chloride ion concentration, the electrolysis time, and a deterioration degree of electrodes 1032, are set with an algorithm created, and stored in storage 1041b.

Outputter 1041e outputs a signal (control signal) to each device (brine conveyance pump 1035, electromagnetic valve 1033, and hypochlorous acid water conveyance pump 1037) based on the received control information.

More specifically, brine conveyance pump 1035 first maintains a state of stopping based on a signal from outputter 1041e, and hypochlorous acid water conveyance pump 1037 maintains a state of stopping based on a signal from outputter 1041e.

Then, electromagnetic valve 1033 is opened based on a signal from outputter 1041e. Due to this, supply of tap water from the water supply pipe to electrolytic tank 1031 starts. Thereafter, electromagnetic valve 1033 is closed based on a signal from outputter 1041e having received the water level information (full) from water level sensor 1039. Due to this, electrolytic tank 1031 is brought into a state where tap water is supplied with a set supply amount.

Next, brine conveyance pump 1035 starts behavior based on a signal from outputter 1041e, conveys, to electrolytic tank 1031, a liquid containing a predetermined amount of chloride ions, and is stopped. Due to this, chloride ions dissolve in the tap water, and electrolytic tank 1031 is brought into a state where an aqueous solution (aqueous chloride solution) containing a predetermined amount of chloride ions is generated.

Then, electrodes 1032 start electrolysis of the aqueous chloride solution based on a signal from outputter 1041e, generates the hypochlorous acid water with the set conditions, and is stopped. The hypochlorous acid water generated by electrodes 1032 is brought into a state where, for example, the hypochlorous acid concentration is 100 ppm to 150 ppm (e.g., 120 ppm) and pH is 7.0 to 8.5 (e.g., 8.0).

As described above, air purification control unit 1041 executes the electrolysis treatment in electrolytic tank 1031, and hypochlorous acid water of a predetermined concentration and amount is generated.

### <Behavior regarding supply treatment of hypochlorous acid water to air purifier>

Air purification control unit 1041 executes the following treatment as a behavior regarding the supply treatment of hypochlorous acid water to air purifier 1011.

As a trigger of the supply treatment of hypochlorous acid water to air purifier 1011, air purification control unit 1041 outputs a hypochlorous acid water supply request to hypochlorous acid water generator 1030 (hypochlorous acid water supplier 1036) every time timer 1041c measures the operating time of humidifying motor 1011a and the operating time elapses a predetermined time (e.g., 60 minutes). Here, the predetermined time is time estimated in advance through an experimental evaluation, given the fact that hypochlorous acid in hypochlorous acid water vaporizes and decreases over time.

Specifically, processing unit 1041d specifies and outputs, to outputter 1041e, control information based on the information regarding time (time information) from timer 1041c and the setting information from storage 1041b. Here, the setting information includes information regarding the supply interval of the hypochlorous acid water (e.g., 60 minutes) and information regarding on/off behavior of hypochlorous acid water conveyance pump 1037.

Then, outputter 1041e outputs a signal (control signal) to hypochlorous acid water conveyance pump 1037 of hypochlorous acid water supplier 1036 based on the received control information.

Hypochlorous acid water conveyance pump 1037 operates based on a signal from outputter 1041e. Due to this, hypochlorous acid water generator 1030 starts supply of the hypochlorous acid water from electrolytic tank 1031 to air purifier 1011 (mixing tank 1092). Note that in order to ensure the concentration of the hypochlorous acid water stored in electrolytic tank 1031, when hypochlorous acid water is supplied from hypochlorous acid water generator 1030 to mixing tank 1092, the entire amount of hypochlorous acid water generated in electrolytic tank 1031 is supplied. Therefore, after the hypochlorous acid water is supplied, electrolytic tank 1031 is in an empty state, and generation of hypochlorous acid water is not started from a state where the hypochlorous acid water remains in electrolytic tank 1031. When the hypochlorous acid water in electrolytic tank 1031 is brought into a state where the entire amount has been supplied, water level sensor 1039 outputs a water shortage signal as water level information.

Thereafter, hypochlorous acid water conveyance pump 1037 is stopped based on a signal from outputter 1041e having received the information regarding the time (time required for supplying a prescribed amount) from timer 1041c. Due to this, hypochlorous acid water generator 1030 supplies the hypochlorous acid water from electrolytic tank 1031 to air purifier 1011 (mixing tank 1092) at a set supply amount.

As described above, air purification control unit 1041 executes the supply treatment of hypochlorous acid water from hypochlorous acid water generator 1030 (electrolytic tank 1031) to air purifier 1011. Note that the control by which air purification control unit 1041 performs supply of the hypochlorous acid water by hypochlorous acid water supplier 1036 at predetermined time intervals is "first control".

### <Behavior regarding supply treatment of water to air purifier>

Air purification control unit 1041 executes the following treatment as a behavior regarding the supply treatment of water to air purifier 1011.

As a trigger of the supply treatment of water to air purifier 1011, air purification control unit 1041 receives water level information (water shortage signal) from water level sensor 1090 of space purification device 1010, and outputs a water supply request to water supplier 1050.

Specifically, inputter 1041a receives and outputs, to processing unit 1041d, water level information (water shortage signal) from water level sensor 1090 of space purification device 1010.

Processing unit 1041d specifies and outputs, to outputter 1041e, control information based on the water level information (water shortage signal) from inputter 1041a, the information regarding time (time information) from timer 1041c, and the setting information from storage 1041b. Here, the setting information includes information regarding on/off behavior of electromagnetic valve 1051 of water supplier 1050.

Then, outputter 1041e then outputs a signal (control signal) to electromagnetic valve 1051 based on the received control information.

Electromagnetic valve 1051 operates based on a signal from outputter 1041e. Due to this, water supplier 1050 starts supply of water from an external water supply pipe to air purifier 1011 (mixing tank 1092), via water conveyance pipe 1052.

Thereafter, electromagnetic valve 1051 is stopped based on a signal from outputter 1041e having received the water level information (full signal) from water level sensor 1090 of space purification device 1010. Due to this, water supplier 1050 supplies water from the external water supply pipe to air purifier 1011 (mixing tank 1092) until the water reaches the set amount.

As described above, air purification control unit 1041 executes the supply treatment of water from water supplier 1050 to air purifier 1011. Note that the control by which air purification control unit 1041 supplies water by water supplier 1050 based on information (water shortage information) regarding the water level of mixing tank 1092 from water level sensor 1090 is "second control".

### <Behavior regarding humidifying purification treatment in air purifier>

Next, the behavior regarding the humidifying purification treatment in air purifier 1011 of air purification control unit 1041 will be explained.

Inputter 1041a receives user input information from operation device 1043, temperature and humidity information of the air of indoor space 1018 from temperature and humidity sensor 1044, and water level information of the hypochlorous acid water (mixed water) in mixing tank 1092 from water level sensor 1090. Inputter 1041a outputs each piece of the received information to processing unit 1041d.

Here, operation device 1043 is a terminal to which user input information (e.g., air volume, target temperature, target humidity, presence or absence of addition of hypochlorous acid, target supply amount level of hypochlorous acid, and the like) regarding space purification device 1010 is input, and is communicably connected to air purification control unit 1041 in a wireless or wired manner.

Temperature and humidity sensor 1044 is a sensor provided in target indoor space 1018, and senses the temperature and humidity of the air in indoor space 1018.

Storage 1041b stores the user input information received by inputter 1041a, and the supply setting information in supply behavior of hypochlorous acid to the air being circulated in the device. Storage 1041b outputs, to processing unit 1041d, the supply setting information having been stored. Note that the supply setting information in supply behavior of hypochlorous acid can also be deemed as humidification setting information in humidifying purification operation of air purifier 1011.

Timer 1041c outputs time information regarding current time to processing unit 1041d.

Processing unit 1041d receives various types of information (user input information, temperature and humidity information, and water level information) from inputter 1041a, the time information from timer 1041c, and the supply setting information from storage 1041b. Using the received user input information, time information, and supply setting information, processing unit 1041d specifies control information regarding humidifying purification operation behavior.

Specifically, processing unit 1041d specifies the humidification requirement amount necessary for indoor space 1018 based on a humidity difference between the target humidity stored in storage 1041b and the temperature and humidity information of the air in indoor space 1018 from temperature and humidity sensor 1044 at a regular interval by time information from timer 1041c.

Then, processing unit 1041d specifies the control information regarding the humidifying purification operation behavior based on the specified humidification requirement amount and the supply setting information stored in storage 1041b. Then, processing unit 1041d outputs the specified control information to outputter 1041e.

In processing unit 1041d, when the water level information from water level sensor 1090 includes information (water shortage signal) regarding the water level indicating water shortage of the hypochlorous acid water (mixed water) in mixing tank 1092, outputter 1041e outputs, to outputter 1041e, a signal of water supply request for water supplier 1050. Furthermore, in processing unit 1041d, when the operating time of air purifier 1011 (humidifying motor 1011a) reaches a predetermined time (e.g., 60 minutes) based on the time information from timer 1041c, outputter 1041e outputs, to outputter 1041e, a signal of hypochlorous acid water supply request for hypochlorous acid water generator 1030. Note that in the present exemplary embodiment, the water level at which the hypochlorous acid water (mixed water) in mixing tank 1092 indicates water shortage is set to a water level in a state where the hypochlorous acid water amount has dropped to about 1/3 from the state where the hypochlorous acid water (mixed water) is full in mixing tank 1092.

Then, outputter 1041e outputs the received signals to air purifier 1011, hypochlorous acid water generator 1030 (hypochlorous acid water supplier 1036), and water supplier 1050, respectively.

Then, air purifier 1011 receives a signal from outputter 1041e and executes control of the operation behavior based on the received signal. At this time, hypochlorous acid water generator 1030 (hypochlorous acid water supplier 1036) receives a signal (signal for hypochlorous acid water supply request) from outputter 1041e, and executes behavior (first control) regarding the supply treatment of hypochlorous acid water to air purifier 1011 described above, based on the received signal. Water supplier 1050 receives a signal (signal for water supply request) from outputter 1041e, and executes behavior (second control) regarding the supply treatment of water to air purifier 1011 described above, based on the received signal.

As described above, air purification control unit 1041 executes, as the supply treatment, first control of performing supply of the hypochlorous acid water by hypochlorous acid water generator 1030 (hypochlorous acid water supplier 1036) at predetermined time intervals, and second control of performing supply of water by water supplier 1050 based on information (water shortage information) regarding a water level of mixing tank 1092 from water level sensor 1090, and stores the mixed water in mixing tank 1092. Then, when supplying hypochlorous acid water and water to and storing mixed water in mixing tank 1092, air purification control unit 1041 executes the humidifying purification treatment to the air circulating through space purification device 1010 (air purifier 1011) with supply cycle of the hypochlorous acid water (predetermined time intervals) and supply cycle of the water (every water shortage detection) being made different from each other.

### <Behavior regarding drainage treatment of mixed water of air purifier>

Air purification control unit 1041 executes the following treatment as a behavior regarding the drainage treatment of the mixed water stored in mixing tank 1092 of air purifier 1011.

As a trigger of the drainage treatment of the mixed water stored in mixing tank 1092, air purification control unit 1041 determines the presence or absence of implementation of the drainage treatment based on the information regarding the number of times of execution of the first control in hypochlorous acid water supplier 1036 and the information regarding the number of times of execution of the second control in water supplier 1050. Note that the information regarding the number of times of execution of each control also includes information regarding the time when each control is executed.

Specifically, storage 1041b stores the number of times of execution of the first control in hypochlorous acid water supplier 1036 and the number of times of execution of the second control by water supplier 1050. Here, the number of times of execution is the number of times of each control executed after the start of the humidifying purification treatment behavior (hereinafter, also called "after the operation start") with the initial state (e.g., a state where mixing tank 1092 becomes full with supply of water and supply of hypochlorous acid water executed after the drainage treatment) of mixing tank 1092 as a starting point.

Processing unit 1041d specifies the number of times the first control has been continuously executed (the number of times of continuous execution of the first control) based on the information regarding the number of times of execution of the first control and the information regarding the number of times of execution of the second control, and performs determination as to whether or not the number of times of continuous execution of the first control is the reference number of times.

Here, the reference number of times is set to "5 times" based on the hypochlorous acid concentration of the hypochlorous acid water supplied from hypochlorous acid water supplier 1036 such that the hypochlorous acid water concentration in mixing tank 1092 does not exceed a reference concentration only by continuous supply of the hypochlorous acid water by the first control. The reference concentration is set to such hypochlorous acid concentration that a user in indoor space 1018 does not feel unpleasant due to an odor of air 1009 (air 1009 containing hypochlorous acid) blown into indoor space 1018.

As a result of the determination, when the number of times of continuous execution of the first control is the reference number of times, processing unit 1041d specifies and outputs, to outputter 1041e, control information based on the information regarding time (time information) from timer 1041c and the setting information from storage 1041b. Here, the setting information includes information regarding on/off behavior of electromagnetic valve 1061 of drainage 1060.

Then, outputter 1041e then outputs a signal (control signal) to electromagnetic valve 1061 based on the received control information.

Electromagnetic valve 1061 operates based on a signal from outputter 1041e. Due to this, drainage 1060 starts drainage of the mixed water from mixing tank 1092 to the external drainage pipe through water conveyance pipe 1062.

Thereafter, electromagnetic valve 1061 is stopped after a lapse of a predetermined time (e.g., 1 minute) based on a signal from outputter 1041e having received the time information from timer 1041c. Due to this, mixing tank 1092 is brought into an empty state with the stored mixed water drained entirely.

As described above, air purification control unit 1041 executes drainage treatment of the mixed water from mixing tank 1092 to the outside. Note that the control by which air purification control unit 1041 performs drainage of mixed water by drainage 1060 based on information regarding the number of times of continuous execution of the first control in hypochlorous acid water supplier 1036 is called "third control".

Here, the third control is preferably performed immediately before execution of the first control by hypochlorous acid water supplier 1036. Due to this, for example, since drainage by the third control is no longer performed immediately after the hypochlorous acid water is supplied to mixing tank 1092 by the first control, the mixed water stored in mixing tank 1092 can be continuously used maximally and waste due to the drainage by the third control can be reduced. Note that in the following example, from the viewpoint of reducing waste of hypochlorous acid water as an active ingredient of disinfection, the third control is performed immediately before the execution of the first control.

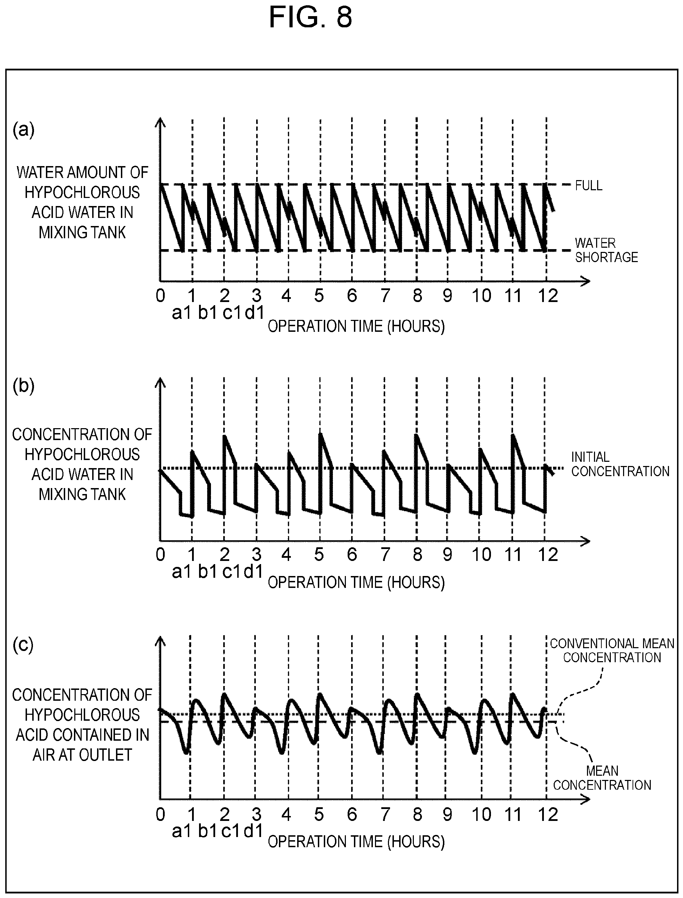

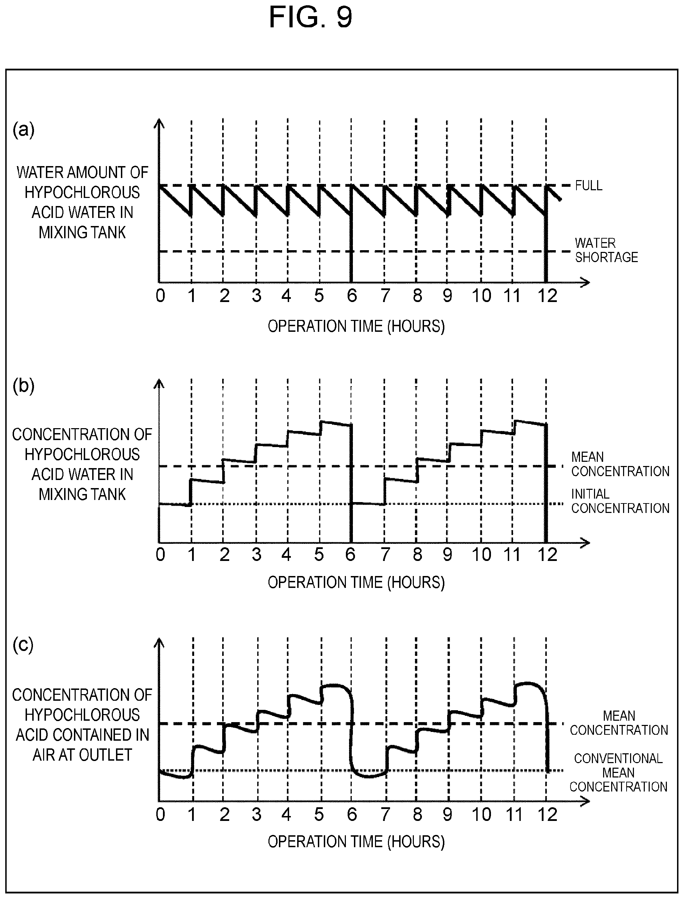

Next, with reference to Figs. 8 and 9, mixed water (mixed water mixed by performing the first control or the second control) in mixing tank 1092 of space purification device 1010 (air purifier 1011) in space purification system 1100 will be described. Fig. 8 is a schematic diagram illustrating temporal changes (winter: first example) of the water amount, the hypochlorous acid water concentration, and the hypochlorous acid concentration in space purification system 1100. More specifically, (a) of Fig. 8 illustrates a temporal change in the water amount of hypochlorous acid water (mixed water) in mixing tank 1092. (b) of Fig. 8 illustrates a history change of the concentration of hypochlorous acid water (mixed water) in mixing tank 1092. (c) of Fig. 8 illustrates a temporal change in the concentration of the hypochlorous acid contained in the air at outlet 1003. Fig. 9 is a schematic diagram illustrating temporal changes (summer: second example) of the water amount, the hypochlorous acid water concentration, and the hypochlorous acid concentration in space purification system 1100. More specifically, (a) of Fig. 9 illustrates a temporal change in the water amount of hypochlorous acid water (mixed water) in mixing tank 1092. (b) of Fig. 9 illustrates a history change of the concentration of hypochlorous acid water (mixed water) in mixing tank 1092. (c) of Fig. 9 illustrates a temporal change in the concentration of the hypochlorous acid contained in the air at outlet 1003.

Here, the supply of the hypochlorous acid water to mixing tank 1092 is executed every predetermined time (1 hour), and the supply of the water to mixing tank 1092 is executed every time water level sensor 1090 detects the water level at which mixing tank 1092 becomes water shortage. The drainage treatment is executed based on a determination result based on the number of times of continuous execution of the first control performed immediately before the execution of the first control. More specifically, the drainage treatment is executed based on whether or not the number of times of continuous execution of the first control has become the reference number of times (5 times) in the drainage determination performed immediately before the supply timing of the hypochlorous acid water.

Note that as described above, even when the hypochlorous acid water (mixed water) in mixing tank 1092 reaches the water level of water shortage, hypochlorous acid water (mixed water) remains in mixing tank 1092 by about 1/3 with respect to that at the time of full. In order to simplify the description, it is assumed that air purifier 1011 behaves with a constant humidification requirement amount during the humidifying purification operating time. Hereinafter, a predetermined amount of hypochlorous acid water supplied to mixing tank 1092 is also called "undiluted hypochlorous acid water".

First, a behavior situation in winter in Japan will be described. Note that in winter in Japan, since the outside air is dry, the humidification requirement amount to air purifier 1011 is large, and supply of water is performed at a shorter interval than supply of hypochlorous acid water. In other words, the water level in mixing tank 1092 becomes water shortage before the supply timing of the hypochlorous acid water.

Therefore, hereinafter, as the first example, the treatment under a humidifying purification condition in which the supply of water (second control) is executed 4 times and the supply of hypochlorous acid water (first control) is executed 3 times in a period in which the operating time after the operation start of air purifier 1011 is up to 3 hours will be described.

Note that the humidifying purification condition described above is a condition set based on controlling air purifier 1011 such that the number of times of performing the first control becomes smaller than the number of times of performing the second control when the humidification requirement amount for air purifier 1011 is greater than or equal to the first reference value. Here, the first reference value is a value set to distinguish between a situation where the humidity of the air is low and the air is dry in winter in Japan and a situation where the humidity of the air is high and the air is moist in summer in Japan.

In the first example, as illustrated in (a) of Fig. 8, the supply of hypochlorous acid water to mixing tank 1092 (first control) is executed at timings of 1 hour, 2 hours, 3 hours..., where the operation start is 0 hours. On the other hand, the supply of water (second control) to mixing tank 1092 is executed at timings of a1 hours, b1 hours, c1 hours, and d1 hours.... At the time point of 0 hours when the operation is started, the supply of the hypochlorous acid water and supply of the water to mixing tank 1092 are executed, and mixing tank 1092 is in a state (initial state) of being full with the hypochlorous acid water (mixed water) having a predetermined concentration.

Since the supply of the hypochlorous acid water (first control) and the supply of water (second control) concur with each other at the timing of 3 hours, the first example can be regarded as the supply of hypochlorous acid water (first control) and the supply of water (second control) by a 3-hour cycle. However, in the supply of water (second control) at this timing, since, in addition to the mixed water remaining in mixing tank 1092 by about 1/3 with respect to the full time, the supply amount of water is reduced by the amount of the supply amount of hypochlorous acid water, the hypochlorous acid water concentration in mixing tank 1092 becomes slightly higher than the initial state at the time point of 0 hours.

In the first example, the supply of hypochlorous acid water becomes 3 times while the supply of water is 4 times in a period in which the operating time after the operation start of air purifier 1011 is up to 3 hours (period in which the operating time is from over 0 hours to less than or equal to 3 hours). Thereafter, the operating time of 3 hours is regarded as the initial state (0 hours), and the same supply behavior is repeated every operating time of 3 hours.

That is, in the first example, it can be deemed that control is performed such that the number of times of performing the first control becomes smaller than the number of times of performing the second control when the humidification requirement amount for air purifier 1011 is greater than or equal to the first reference value.

The drainage determination of the mixed water stored in mixing tank 1092 is performed immediately before the execution of the first control at the timings of 1 hour, 2 hours, 3 hours.... Note that in winter in Japan, the humidification requirement amount to air purifier 1011 is large, and supply of water (second control) is executed at a shorter interval than supply of hypochlorous acid water (first control). Therefore, the first control is not continuously executed, and drainage of the mixed water by the third control is not executed.

This will be described in more detail.

With reference to (a) of Fig. 8, a description will be given focusing on a temporal change in the water level of the hypochlorous acid water (mixed water) in mixing tank 1092.

At the early stage of the operation (0 hours), mixing tank 1092 is filled with mixed water (this is also hypochlorous acid water) of undiluted hypochlorous acid solution and water up to full. The humidifying purification operation reduces the water amount of the mixed water at a constant speed and detects water shortage at a timing at which a1 hours elapses from the operation start, and water is supplied from water supplier 1050 until mixing tank 1092 becomes full. Thereafter, while the water level of the mixed water is reduced at a constant speed by the humidifying purification operation, 1 hour, which is the supply timing of the hypochlorous acid water, elapses, and the drainage determination of the mixed water is executed at this timing of 1 hour. In the humidifying purification operation so far, the number of times of continuous supply of the undiluted hypochlorous acid water by the first control after the supply of water by the second control is 0 times, and it is determined that the number of times of continuous execution of the first control has not reached the reference number of times (5 times). When the supply of water including the second control or the drainage treatment including the third control is executed, the number of times of continuous execution of the first control is reset.

In response to the determination result, the first control is executed without the drainage of the mixed water stored in mixing tank 1092 being executed, and the undiluted hypochlorous acid water is supplied from hypochlorous acid water generator 1030 (hypochlorous acid water supplier 1036) to mixing tank 1092. Due to this, the water level in mixing tank 1092 rises slightly. Then, the number of times of continuous execution of the first control becomes 1 time. Also thereafter, the water level of the mixed water decreases by the humidifying purification operation, the water shortage occurs again at the timing of b1 hours from the operation start, and the water is supplied from water supplier 1050 until mixing tank 1092 becomes full. Then, the number of times of continuous execution of the first control is reset to 0 times.

Thereafter, the drainage determination is performed at a timing when the operating time after the operation start becomes 2 hours. In the humidifying purification operation so far, the number of times of continuous supply of the undiluted hypochlorous acid water by the first control after the supply of water by the second control is 0 times, and it is determined that the number of times of continuous execution of the first control has not reached the reference number of times (5 times).

In response to the determination result, the first control is executed without the drainage of the mixed water stored in mixing tank 1092 being executed, and the undiluted hypochlorous acid water is supplied from hypochlorous acid water generator 1030 (hypochlorous acid water supplier 1036) to mixing tank 1092. Due to this, the water level in mixing tank 1092 rises slightly. Then, the number of times of continuous execution of the first control becomes 1 time. Also thereafter, the water level of the mixed water decreases by the humidifying purification operation, the water shortage occurs again at the timing of c1 hours from the operation start, and the water is supplied from water supplier 1050 until mixing tank 1092 becomes full. Then, the number of times of continuous execution of the first control is reset to 0 times.

Thereafter, the drainage determination is performed at the timing when the operating time after the operation start is 3 hours (d1 hours). In the humidifying purification operation so far, the number of times of continuous supply of the undiluted hypochlorous acid water by the first control after the supply of water by the second control is 0 times, and it is determined that the number of times of continuous execution of the first control has not reached the reference number of times (5 times).

Then, at this timing, since the water shortage and the supply timing of the undiluted hypochlorous acid water concur with each other, the first control and the second control are executed in this order without execution of the drainage of the mixed water stored in mixing tank 1092 in response to the determination result. More specifically, as the first control, the undiluted hypochlorous acid water is first supplied from hypochlorous acid water generator 1030 (hypochlorous acid water supplier 1036) to mixing tank 1092. Thereafter, as the second control, water is supplied from water supplier 1050 until mixing tank 1092 becomes full. Due to this, the undiluted hypochlorous acid water and the water are supplied into mixing tank 1092, and the water level in mixing tank 1092 is brought into a state of being close to that of the initial stage of operation (0 hours). Note that since supply of water is performed, the number of times of continuous execution of the first control is reset to 0 times.

Thereafter, as in the period in which the operating time after the operation start is up to 3 hours, supply of water at the timing when the water shortage occurs, and supply of undiluted hypochlorous acid water at the supply timing of hypochlorous acid water are repeated.

Next, with reference to (b) of Fig. 8, a description will be given focusing on a temporal change in the concentration of hypochlorous acid water (mixed water) in mixing tank 1092.

At the beginning of the operation (0 hours), mixed water of undiluted hypochlorous acid water and water is mixed in mixing tank 1092 so as to have a predetermined concentration (initial concentration). Then, when the humidifying purification operation is started, the concentration of the hypochlorous acid water (mixed water) in mixing tank 1092 decreases with the elapse of time from the operation start up to a1 hours. This is because a constant proportion of hypochlorous acid with respect to the concentration of the hypochlorous acid water vaporizes and is given to the air due to the vapor pressure of hypochlorous acid being higher than that of water. Note that unless the hypochlorous acid vaporizes, the hypochlorous acid contained in the water merely consumed together with the water micronized by air purifier 1011, and therefore, the hypochlorous acid water does not change in terms of the concentration of the hypochlorous acid water in mixing tank 1092 although decreasing at a constant speed depending on the humidification amount. Even at a1 hours, which is the timing at which water level sensor 1090 detects the water shortage, the concentration of the hypochlorous acid water is not zero, which is because the hypochlorous acid water (mixed water) remains in mixing tank 1092 even in a state where a water shortage is detected, as mentioned earlier.

Then, when a1 hours elapses (water shortage detection) from the operation start, the hypochlorous acid water in mixing tank 1092 is diluted with water along with supply of the water from water supplier 1050, and therefore the concentration of the hypochlorous acid water in mixing tank 1092 decreases. Thereafter, the concentration of the hypochlorous acid water (mixed water) slightly decreases by vaporization of the hypochlorous acid until 1 hour, which is the supply timing of the hypochlorous water, elapses.

Then, when 1 hour, which is the supply timing of the hypochlorous acid water, elapses from the operation start, the concentration of the hypochlorous acid water in mixing tank 1092 rises to greater than or equal to the initial concentration along with supply of undiluted hypochlorous acid water from hypochlorous acid water generator 1030 (hypochlorous acid water supplier 1036). This is because a predetermined amount of hypochlorous acid water (undiluted hypochlorous acid water) supplied at the initial stage of operation is supplied to mixed water (water in a state of containing hypochlorous acid) having a water amount smaller than that of water supplied at the initial stage (0 hours) of the operation. Thereafter, the concentration of the hypochlorous acid water (mixed water) decreases by the vaporization of the hypochlorous acid up to b 1 hours (water shortage detection) elapses from the operation start. Note that the decrease speed of the hypochlorous acid is higher than in the initial stage of operation because the content of the hypochlorous acid contained in the mixed water is large and accordingly, the vaporization amount of the hypochlorous acid becomes large.

Then, when b 1 hours elapses (water shortage detection) from the operation start, the hypochlorous acid water in mixing tank 1092 is diluted with water along with supply of the water from water supplier 1050, and therefore the concentration of the hypochlorous acid water in mixing tank 1092 decreases. Thereafter, the concentration of the hypochlorous acid water (mixed water) slightly decreases by vaporization of the hypochlorous acid until 2 hours, which is the supply timing of the hypochlorous acid water, elapses.

Then, when 2 hours, which is the supply timing of the hypochlorous acid water, elapses from the operation start, the concentration of the hypochlorous acid water in mixing tank 1092 rises to greater than or equal to the initial concentration along with supply of undiluted hypochlorous acid water from hypochlorous acid water generator 1030 (hypochlorous acid water supplier 1036). Thereafter, the concentration of the hypochlorous acid water (mixed water) decreases by the vaporization of the hypochlorous acid up to c1 hours (water shortage detection) elapses from the operation start.

Then, when c1 hours elapses (water shortage detection) from the operation start, the hypochlorous acid water in mixing tank 1092 is diluted with water along with supply of the water from water supplier 1050, and therefore the concentration of the hypochlorous acid water in mixing tank 1092 decreases. Thereafter, the concentration of the hypochlorous acid water (mixed water) slightly decreases by vaporization of the hypochlorous acid until 3 hours, which is the supply timing of the hypochlorous acid water, elapses.

When 3 hours (d1 hours), which is the supply timing of the water (and the hypochlorous acid water), elapses from the operation start, the water and the undiluted hypochlorous acid water are supplied into mixing tank 1092, and the concentration of the hypochlorous acid water in mixing tank 1092 is brought into a close state to that in the initial stage of operation (0 hours). Thereafter, the concentration change of hypochlorous acid water (mixed water) is repeated same as ever.

Next, with reference to (c) of Fig. 8, a description will be given focusing on a temporal change in the concentration of the hypochlorous acid contained in air 1009 at outlet 1003.

The concentration of the hypochlorous acid contained in air 1009 released from outlet 1003 is determined by the humidification amount in air purifier 1011 and the concentration of the hypochlorous acid water in mixing tank 1092, but in the first example, since the humidification amount is constant, the concentration of the hypochlorous acid water in mixing tank 1092 is reflected. Therefore, as illustrated in (c) of Fig. 8, the concentration of the hypochlorous acid in air 1009 at outlet 1003 increases or decreases in accordance with an increase or a decrease in the concentration of the hypochlorous acid water in mixing tank 1092 as illustrated in (b) of Fig. 8.

Here, as conventionally, in a case where undiluted hypochlorous acid water and water are supplied to make full every time water level sensor 1090 detects water shortage, the state from the operation start (0 hours) to a1 hours is repeated until the timing of 3 hours (d1 hours). In this case, the mean concentration of the hypochlorous acid contained in air 1009 at outlet 1003 becomes the conventional mean concentration illustrated in (c) of Fig. 8, for example. On the other hand, in the first example, the state from the operation start (0 hours) to a1 hours is the same as the conventional state, but the state in the period from a1 hours to 3 hours is different from the conventional state. More specifically, in the period from a1 hours to 3 hours, as illustrated in (b) of Fig. 8, the period (part of period from 1 hour to b1 hours, period from 2 hours to c1 hours) in which the concentration of the hypochlorous acid water is higher than the initial concentration has become shorter than the period (period from a1 hours to 1 hour, period from b1 hours to 2 hours, period from c1 hours to 3 hours) in which it is smaller than the initial concentration. Therefore, in the period from the operation start (0 hours) to 3 hours, the mean concentration of the hypochlorous acid contained in air 1009 at outlet 1003 becomes a mean concentration lower than the conventional mean concentration.

As described above, as in the first example, when the hypochlorous acid water and water are supplied to mixing tank 1092 and the mixed water is stored, the supply cycle (predetermined time intervals) of the hypochlorous acid water and the supply cycle (every water shortage detection) of the water are made different, whereby the concentration of the hypochlorous acid contained in air 1009 at outlet 1003, i.e., the air blown out into indoor space 1018 can be reduced as compared with the case where the hypochlorous acid water and the water are supplied to mixing tank 1092 in the conventional method.

Next, a behavior situation in summer in Japan will be described. Note that in summer in Japan, since the outside air is moist and damp, the humidification requirement amount to air purifier 1011 is small, and supply of water is performed at a longer interval than supply of the hypochlorous acid water. That is, supply of hypochlorous acid water (first control) is performed many times before supply of water (second control) is performed.

Therefore, hereinafter, as the second example, the treatment under a humidifying purification condition in which the mixed water corresponding to the supply amount of the undiluted hypochlorous acid water is consumed during the period of the operating time of 1 hour and the supply of the hypochlorous acid water (first control) is executed each time will be described. That is, in the second example, supply of hypochlorous acid water (first control) is continuously executed, and supply of water (second control) associated with water shortage detection is not executed.

In the second example, as illustrated in (a) of Fig. 9, the supply of undiluted hypochlorous acid water to mixing tank 1092 (first control) is executed at timings of 1 hour, 2 hours, 3 hours..., where the operation start is 0 hours. On the other hand, the consumption amount associated with the humidifying purification is equivalent to the hypochlorous acid water amount supplied by the first control, and therefore water shortage detection by water level sensor 1090 is not performed and the supply of water (second control) to mixing tank 1092 is not executed. At the time point of 0 hours when the operation is started, the supply of the hypochlorous acid water and supply of the water to mixing tank 1092 are executed, and mixing tank 1092 is in a state (initial state) of being full with the hypochlorous acid water (mixed water) having a predetermined concentration.

The drainage determination of the mixed water stored in mixing tank 1092 is performed immediately before the execution of the first control at the timings of 1 hour, 2 hours, 3 hours....

Specifically, at the timing when the operating time of 1 hour after the operation start elapses, the number of times of supply of the undiluted hypochlorous acid water by the first control is 0 times, and it is determined that the number of times of continuous execution of the first control does not reach the reference number of times (5 times). In response to the determination result, the first control is executed, and mixing tank 1092 is supplied with the undiluted hypochlorous acid water.

Next, the drainage determination of mixed water is executed at the timing when the operating time after the operation start reaches 2 hours. In the humidifying purification operation so far, the number of times of supply of the undiluted hypochlorous acid water by the first control is 1 time, and it is determined that the number of times of execution of the first control has not reached the reference number of times (5 times). In response to the determination result, the first control is executed, and mixing tank 1092 is supplied with the undiluted hypochlorous acid water.

Thereafter, similar control is executed until the timing at which the operating time after the operation start is up to 5 hours.

Subsequently, the drainage determination of mixed water is executed at the timing when the operating time after the operation start reaches 6 hours. In the humidifying purification operation so far, the number of times of continuous supply of the undiluted hypochlorous acid water by the first control is 5 times, and it is determined that the number of times of execution of the first control is the reference number of times (5 times). In response to the determination result, the third control is executed, and the mixed water in mixing tank 1092 is drained entirely. Furthermore, after the third control is executed, the supply of undiluted hypochlorous acid water and the supply of water are newly executed to mixing tank 1092, and mixing tank 1092 is brought into a state of full with the hypochlorous acid water (mixed water) having a predetermined concentration that is the same as the initial state.

Thereafter, the timing of 6 hours is regarded as the initial state (0 hours), and the same supply behavior and drainage operation are repeated every period of 6 hours.

This will be described in more detail.

First, with reference to (a) of Fig. 9, a description will be given focusing on a temporal change in the water level of the hypochlorous acid water (mixed water) in mixing tank 1092.

At the early stage of the operation (0 hours), mixing tank 1092 is filled with mixed water (this is also hypochlorous acid water) of undiluted hypochlorous acid solution and water up to full. Then, the humidifying purification operation reduces the water amount of the mixed water at a constant speed, and 1 hour, which is the supply timing of the hypochlorous acid water, elapses. Then, the drainage determination of the mixed water is executed at this timing of 1 hour.

In the humidifying purification operation so far, since neither the supply of water by the second control nor the supply of undiluted hypochlorous acid water by the first control is executed, the number of times of continuous supply of the undiluted hypochlorous acid water by the first control is 0 times, and it is determined that the number of times of continuous execution of the first control has not reached the reference number of times (5 times). In response to the determination result, the first control is executed without the drainage of the mixed water stored in mixing tank 1092 being executed, and the undiluted hypochlorous acid water is supplied from hypochlorous acid water generator 1030 (hypochlorous acid water supplier 1036) to mixing tank 1092. Due to this, the water level in mixing tank 1092 rises to a full state. Also thereafter, the water level of the mixed water decreases by the humidifying purification operation, 2 hours, which is the supply timing of the hypochlorous acid water, elapses. Then, the drainage determination of the mixed water is executed at this timing of 2 hours.

In the humidifying purification operation so far, since the number of times of continuous supply of the undiluted hypochlorous acid water by the first control is 1 time, it is determined that the number of times of execution of the first control has not reached the reference number of times (5 times). In response to the determination result, the first control is executed without the drainage of the mixed water stored in mixing tank 1092 being executed, and the undiluted hypochlorous acid water is supplied from hypochlorous acid water generator 1030 (hypochlorous acid water supplier 1036) to mixing tank 1092. Due to this, the water level in mixing tank 1092 rises to a full state. Thus, in the second example, although the water amount of the mixed water decreases at a constant speed by the humidifying purification operation, since the consumed amount of the undiluted hypochlorous acid water is supplied, the water amount of the mixed water only increases or decreases between the state of decreasing by the consumption amount and the full state.

Thereafter, 6 hours, which is the supply timing of the undiluted hypochlorous acid water, elapses, and the drainage determination of the mixed water is executed at this timing of 6 hours.

In the humidifying purification operation so far, since the number of times of supply of the undiluted hypochlorous acid water by the first control is 5 times, it is determined that the number of times of execution of the first control has reached the reference number of times (5 times). In response to the determination result, the third control is executed, and the mixed water in mixing tank 1092 is drained. Furthermore, after the execution of drainage of the mixed water by the third control, the supply of undiluted hypochlorous acid water and the supply of water are newly executed to mixing tank 1092, and mixing tank 1092 is brought into a state of full with the hypochlorous acid water (mixed water) having a predetermined concentration that is the same as the initial state (0 hours). Here, the number of times of continuous execution of the first control is reset, and the storage of the number of times of execution of the first control is started again.

Thereafter, the timing of 6 hours is regarded as the initial state (0 hours), and the same supply behavior and drainage operation are repeated every period of 6 hours. More specifically, same as ever, supply of undiluted hypochlorous acid water by the first control at the supply timing of hypochlorous acid water is repeated. Immediately before the execution of the first control, the drainage determination of the mixed water by the third control is performed, and the third control is executed when the condition is satisfied. The water level of the hypochlorous acid water (mixed water) in mixing tank 1092 increases or decreases corresponding to each behavior.

Next, with reference to (b) of Fig. 9, a description will be given focusing on a temporal change in the concentration of hypochlorous acid water (mixed water) in mixing tank 1092.

At the beginning of the operation (0 hours), mixed water of undiluted hypochlorous acid water and water is mixed in mixing tank 1092 so as to have a predetermined concentration (initial concentration). Then, when the humidifying purification operation is started, the concentration of the hypochlorous acid water (mixed water) in mixing tank 1092 decreases with the elapse of time from the operation start up to 1 hour. This is because a constant proportion of hypochlorous acid with respect to the concentration of the hypochlorous acid water vaporizes and is given to the air due to the vapor pressure of hypochlorous acid being higher than that of water, as mentioned above.

Then, when 1 hour, which is the supply timing of the hypochlorous acid water, elapses from the operation start, the concentration of the hypochlorous acid water in mixing tank 1092 rises to greater than or equal to the initial concentration along with supply of undiluted hypochlorous acid water from hypochlorous acid water generator 1030 (hypochlorous acid water supplier 1036). This is because, as described above, a predetermined amount of hypochlorous acid water (undiluted hypochlorous acid water) supplied at the initial stage of operation is supplied to mixed water (water in a state of containing hypochlorous acid) having a water amount smaller than that of the mixed water stored at the initial stage (0 hours) of the operation. Thereafter, the concentration of the hypochlorous acid water (mixed water) slightly decreases by vaporization of the hypochlorous acid until 2 hours elapses from the operation start.

Then, when 2 hours, which is the supply timing of the hypochlorous acid water, elapses from the operation start, the concentration of the hypochlorous acid water in mixing tank 1092 further rises to greater than or equal to the initial concentration along with supply of undiluted hypochlorous acid water from hypochlorous acid water generator 1030 (hypochlorous acid water supplier 1036). Thereafter, the concentration of hypochlorous acid water (mixed water) decreases by the vaporization of the hypochlorous acid until 3 hours elapses from the operation start. Similarly, until the timing of 5 hours thereafter, the concentration change of the hypochlorous acid water (mixed water) is repeated, and the concentration of the hypochlorous acid water (mixed water) gradually rises.

When 6 hours, which is the supply timing of the undiluted hypochlorous acid water, elapses from the operation start, the drainage timing comes based on the drainage determination, therefore, after the hypochlorous acid water (mixed water) in mixing tank 1092 is drained entirely, water and undiluted hypochlorous acid water are supplied into mixing tank 1092, and the concentration of the hypochlorous acid water in mixing tank 1092 is brought into a similar state to that in the initial stage of operation (0 hours). Thereafter, the concentration change of hypochlorous acid water (mixed water) is repeated in the same manner as ever.

Next, with reference to (c) of Fig. 9, a description will be given focusing on a temporal change in the concentration of the hypochlorous acid contained in air 1009 at outlet 1003.

Since the concentration of the hypochlorous acid contained in air 1009 released from outlet 1003 is determined by the humidification amount in air purifier 1011 and the concentration of the hypochlorous acid water in mixing tank 1092 as in winter in Japan, as illustrated in (c) of Fig. 9, the concentration of the hypochlorous acid contained in air 1009 at outlet 1003 increases or decreases in accordance with an increase or a decrease in the concentration of the hypochlorous acid water in mixing tank 1092 illustrated in (b) of Fig. 9.

Here, as conventionally, in a case where undiluted hypochlorous acid water and water are supplied to make full every time water level sensor 1090 detects water shortage, the concentration of the hypochlorous acid water continues to decrease from the operation start (0 hours) to 6 hours. Strictly speaking, the concentration of the hypochlorous acid water continues to decrease in the period, within 6 hours, from the full state to when water shortage is detected. In this case, the mean concentration of the hypochlorous acid contained in air 1009 at outlet 1003 becomes the conventional mean concentration illustrated in (c) of Fig. 9, for example.

On the other hand, in the second example, the state from the operation start (0 hours) to 1 hour is the same as the conventional state, but the state in the period from 1 hour to 6 hours is different from the conventional state. More specifically, in the period from 1 hour to 6 hours, as illustrated in (b) of Fig. 9, the period in which the concentration of the hypochlorous acid water is higher than the initial concentration is far longer than the period in which it is lower than the initial concentration. Therefore, in the period from the operation start (0 hours) to 6 hours, the mean concentration of the hypochlorous acid contained in air 1009 at outlet 1003 becomes a mean concentration higher than the conventional mean concentration.

Then, also for 6 hours and thereafter, the concentration change of the mixed water is repeated every 6 hours with 6 hours as 1 cycle, and it is therefore possible to keep adjusting the concentration of the hypochlorous acid water within a range of less than or equal to a certain concentration without the concentration of the hypochlorous acid water keeping rising. That is, when the humidifying purification operation is continued, the concentration of the hypochlorous acid water in mixing tank 1092 may excessively rise, however, by providing control of drainage determination in accordance with the number of times of continuous supply of the undiluted hypochlorous acid water by the first control, it is possible to reset the concentration of the hypochlorous acid water in mixing tank 1092 at regular intervals, and eventually the addition amount of the hypochlorous acid contained in air 1009 at outlet 1003, and it is possible to control the supply amount of a hypochlorous acid gas to indoor space 1018.

As described above, space purification system 1100 supplies hypochlorous acid water into mixing tank 1092 every preset time (e.g., 1 hour) as the first control, executes the treatment of supplying water based on the water level information (water shortage signal) from water level sensor 1090 as the second control, and drains the mixed water in mixing tank 1092 based on the number of times of continuous execution of the first control as the third control. Furthermore, air purification control unit 1041 of space purification system 1100 makes the number of times of performing the first control within a predetermined period different from the number of times of performing the second control within a predetermined period based on the humidification requirement amount (a humidification requirement amount corresponding to winter in Japan or a humidification requirement amount corresponding to summer in Japan) required to air purifier 1011. Due to this, in a state where the humidification requirement amount is high as in winter in Japan, air 1009 in a state where the content of the hypochlorous acid amount is small can be released to indoor space 1018 as compared with the conventional method, and in a state where the humidification requirement amount is low as in summer in Japan, air 1009 in a state where the content of the hypochlorous acid amount is large can be released to indoor space 1018 as compared with the conventional method. Furthermore, when the humidifying purification operation is continued for a long time, an excessive increase in the hypochlorous acid concentration released to indoor space 1018 can be suppressed.

That is, by operating the supply of hypochlorous acid water, the supply of water, and the drainage of mixed water by triggers separate from one another, it is possible to adjust the concentration of the hypochlorous acid water in mixing tank 1092 (concentration of the hypochlorous acid contained in air 1009 blown out to indoor space 1018) by simple control (first control, second control, and third control).

As described above, space purification system 1100 according to the present second exemplary embodiment can achieve the following effects.
(1) Space purification system 1100 includes hypochlorous acid water generator 1030 that generates hypochlorous acid water, hypochlorous acid water supplier 1036 that supplies hypochlorous acid water from hypochlorous acid water generator 1030 to mixing tank 1092, water supplier 1050 that supplies water to mixing tank 1092, water level sensor 1090 for detecting a water level of mixing tank 1092, air purifier 1011 that micronizes and releases, into air, mixed water of the hypochlorous acid water and the water stored in mixing tank 1092, and air purification control unit 1041 that controls supply treatment in hypochlorous acid water supplier 1036 and water supplier 1050 and drainage treatment of the mixed water stored in mixing tank 1092. Air purification control unit 1041 (i) executes, as the control of the supply treatment, first control of causing the hypochlorous acid water supplier 1036 to perform supply of the hypochlorous acid water at predetermined time intervals (e.g., 60 minutes), and second control of causing the water supplier 1050 to perform supply of the water based on information (water shortage information) regarding the water level of mixing tank 1092 from water level sensor 1090, and (ii) executes, as the control of the drainage treatment, third control of causing the mixing tank 1092 to drain the mixed water stored in mixing tank 1092 when the first control is continuously executed a predetermined number of times.

Due to this, when air having high relative humidity is ventilated as in summer in Japan, since the consumption amount of the mixed water stored in mixing tank 1092 is small, the supply frequency (the number of times of performing the first control) of hypochlorous acid water to mixing tank 1092 increases, and the mixed water is micronized and released into the air in a state where the hypochlorous acid concentration of the mixed water in mixing tank 1092 is high. At this time, by executing the third control when continuously executing the first control a predetermined number of times (e.g., 5 times), draining the mixed water stored in mixing tank 1092, and resetting the mixed water in mixing tank 1092, it is possible to suppress an excessive increase in the hypochlorous acid concentration in mixing tank 1092. As a result, even in a situation where the micronized hypochlorous acid water hardly vaporize, hypochlorous acid raised to a predetermined concentration contained in the air can be released into indoor space 1018.

On the other hand, when air having low relative humidity is ventilated as in winter in Japan, since the consumption amount of the mixed water stored in mixing tank 1092 is large, the supply frequency of water to mixing tank 1092 (the number of times of performing the second control) increases, and the mixed water is micronized and released into the air in a state where the hypochlorous acid concentration of the mixed water in mixing tank 1092 is low. As a result, even in a situation where the micronized hypochlorous acid water easily vaporizes, hypochlorous acid attenuated to a predetermined concentration contained in the air can be released into indoor space 1018.

That is, space purification system 1100 can easily adjust the amount of hypochlorous acid to be released into the air.

(2) In space purification system 1100, air purification control unit 1041 executes the third control immediately before executing the first control after continuously executing the first control a predetermined number of times. Due to this, in space purification system 1100, since drainage by the third control is no longer performed immediately after the hypochlorous acid is supplied to mixing tank 1092 by the first control, the hypochlorous acid water supplied by the first control can be continuously used maximally and waste due to the drainage by the third control can be reduced.

Space purification system 1100 can return the state in mixing tank 1092 to the operation initial state before the hypochlorous acid water concentration in mixing tank 1092 excessively increases even when a long time operation (e.g., 24 hours) is performed. That is, space purification system 1100 can easily adjust the amount of hypochlorous acid to be released into the air.

(3) In space purification system 1100, in the supply treatment, air purification control unit 1041 performs control such that the number of times of performing the first control becomes smaller than the number of times of performing the second control when the humidification requirement amount required for air purifier 1011 is greater than or equal to a first reference value, and performs control such that the number of times of performing the first control becomes larger than the number of times of performing the second control when the humidification requirement amount is less than the first reference value. This enables space purification system 1100 can micronize and release, into the air, the mixed water in a state where the hypochlorous acid concentration in mixing tank 1092 is high when the humidification requirement amount is less than the first reference value in the supply treatment. On the other hand, the mixed water can be micronized and released into the air in a state where the hypochlorous acid concentration in mixing tank 1092 is low when the humidification requirement amount is greater than or equal to the first reference value. In other words, based on the humidification requirement amount, space purification system 1100 can give hypochlorous acid to air 1009 released from air purifier 1011 under a condition suitable for the environment of indoor space 1018.

The present disclosure has been described above based on the exemplary embodiments. It is understood by those skilled in the art that these exemplary embodiments are merely examples, that the components or the treatment processes of those can be combined as various modifications, and that such modifications also fall within the scope of the present disclosure.

In the first example and the second example in space purification system 1100 according to the present second exemplary embodiment, it has been described that air purifier 1011 behaves at a constant humidification requirement amount during the humidifying purification operating time, but actually, air purifier 1011 behaves at a humidification requirement amount specified based on a humidity difference between the target humidity and the humidity of the air in indoor space 1018 every constant time.

In space purification system 1100 according to the present second exemplary embodiment, it is preferable to set the predetermined number of times in the third control based on the concentration of the hypochlorous acid water supplied by the first control. Due to this, for example, in space purification system 1100, when the concentration of the hypochlorous acid water supplied by the first control is high, the increase in the concentration of the hypochlorous acid water in mixing tank 1092 becomes fast in a case where the first control is continuously executed a predetermined number of times, and therefore it is possible to more reliably suppress the excessive increase in the concentration of the hypochlorous acid water in mixing tank 1092 by setting the predetermined number of times to be small.

### (Third exemplary embodiment)

As a conventional space purification device, an air-conditioning system that disinfects a space by bringing air to be supplied indoors into contact with a gas-liquid contact member containing a purification component and releasing the air is known (see, for example, PTL 1).

In such conventional space purification device, generally, in addition to release of micronized water, water stored in the device (water containing a purification component) contains a part of the purification component along with micronization behavior, and the water and the purification component are vaporized and released into a space.

With the conventional space purification device, however, in a situation where a humidification amount required for an indoor space is small, for example, in summer season in Japan (rainy season in particular), when air dehumidified by an air-conditioner or the like and having high relative humidity (e.g., 12 °C 95%) is ventilated, water (hypochlorous acid water) containing a micronized purification component is hardly vaporized, therefore the purification component (hypochlorous acid) is not vaporized, and the purification component is hardly released into the indoor space. On the other hand, in a situation where the required humidification amount is large, for example, in winter in Japan, when heated air having low relative humidity (e.g., 20 °C 30%) is ventilated, water containing the micronized purification component is easily vaporized, and therefore a large amount of purification component is released into the indoor space. In other words, the conventional space purification device has a problem that it is not easy to adjust the amount of the purification component released into the indoor space (into the air).

Therefore, the present disclosure has been made to solve the above-described conventional problems, and an object of the present disclosure is to provide a technique that can easily adjust the amount of a purification component released into air.

In order to achieve this object, a space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water suppier to perform supply of the water based on information regarding the water level of the mixing tank from the water level sensor, and (ii) executes, as the control of the drainage treatment, third control of causing the mixing tank to drain the mixed water stored in the mixing tank when the second control is not executed for a predetermined period since the supply of the water by the water supplier is performed, thereby achieving the intended object.

The space purification system according to the present disclosure can easily adjust the amount of the purification component released into air.

Again, a space purification system according to the present disclosure includes: a hypochlorous acid water generator that generates hypochlorous acid water; a hypochlorous acid water supplier that supplies the hypochlorous acid water from the hypochlorous acid water generator to a mixing tank; a water supplier that supplies water to the mixing tank; a water level sensor for detecting a water level of the mixing tank; a humidifying purifier that micronizes and releases, into air, mixed water of the hypochlorous acid water and the water stored in the mixing tank; and a control unit that controls supply treatment in the hypochlorous acid water supplier and the water supplier and drainage treatment of the mixed water stored in the mixing tank. The control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank when the second control is not executed for a predetermined period since the supply of the water by the water supplier is performed.

By doing this, when air having high relative humidity is ventilated as in summer in Japan, since the consumption amount of mixed water stored in the mixing tank is small, the supply frequency (the number of times of performing the first control) of the hypochlorous acid water to the mixing tank increases, and the mixed water is micronized and released into the air in a state where the hypochlorous acid concentration of the mixed water in the mixing tank is high. At this time, by executing the third control when having not executed the second control for a predetermined period since performing supply of water by the water supplier, draining the mixed water stored in the mixing tank, and resetting the mixed water in the mixing tank, it is possible to suppress an excessive increase in the hypochlorous acid concentration in the mixing tank. As a result, even in a situation where the micronized hypochlorous acid water hardly vaporize, hypochlorous acid raised to a predetermined concentration contained in the air can be released into the indoor space. On the other hand, when air having low relative humidity is ventilated as in winter in Japan, since the consumption amount of the mixed water stored in the mixing tank is large, the supply frequency of water to the mixing tank (the number of times of performing the second control) increases, and the mixed water is micronized and released into the air in a state where the hypochlorous acid concentration of the mixed water in the mixing tank is low. As a result, even in a situation where the micronized hypochlorous acid water easily vaporizes, hypochlorous acid attenuated to a predetermined concentration contained in the air can be released into the indoor space. That is, the space purification system can easily adjust the amount of hypochlorous acid to be released into the air.

In the space purification system according to the present disclosure, the control unit preferably executes the third control immediately before executing the first control. Due to this, in the space purification system, since drainage by the third control is no longer performed immediately after the hypochlorous acid is supplied to the mixing tank by the first control, the hypochlorous acid water supplied by the first control can be continuously used maximally and waste due to the drainage by the third control can be reduced.

In the space purification system according to the present disclosure, the predetermined period is preferably set based on the concentration of the hypochlorous acid water supplied by the first control. For example, in the space purification system, when the concentration of the hypochlorous acid water supplied by the first control is high, unless the supply of water by the second control is executed, the increase in the concentration of the hypochlorous acid water in the mixing tank becomes fast. Therefore, by setting the predetermined period to be short, it is possible to more reliably suppress the excessive increase in the concentration of the hypochlorous acid water in the mixing tank.

Modes for carrying out the present disclosure will now be described with reference to the accompanying drawings. Note that the following exemplary embodiments are examples embodying the present disclosure, and do not limit the technical scope of the present disclosure in any way. Throughout the drawings, identical parts are denoted by the identical reference marks, and descriptions thereof will be omitted. Furthermore, details of each part not directly related to the present disclosure are not described for each drawing to avoid redundancy.

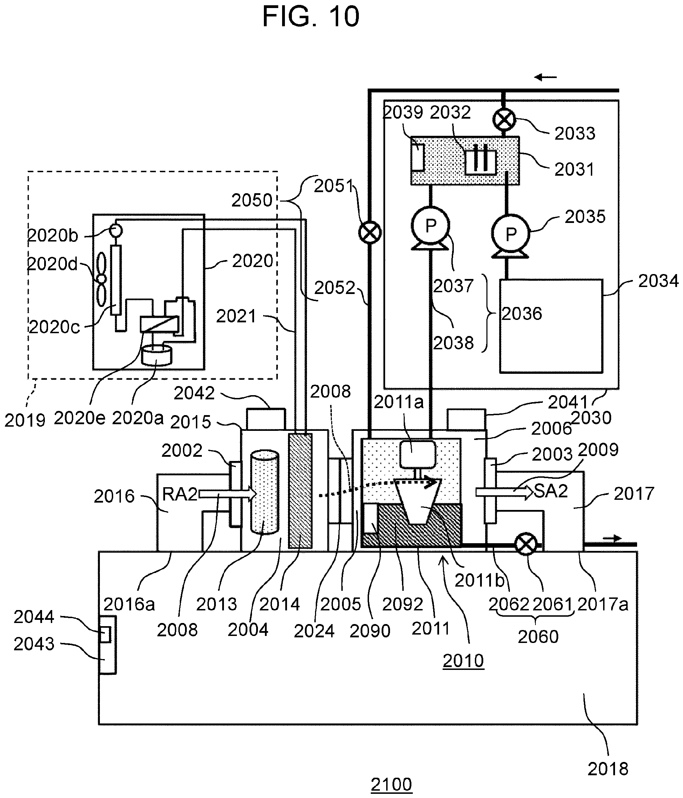

Fig. 10 is a view illustrating the configuration of space purification system 2100 according to the third exemplary embodiment of the present disclosure. Space purification system 2100 is a device that performs cooling treatment (dehumidification treatment) or heating treatment on air 2008 (RA2) from indoor space 2018 as necessary when circulating the air of indoor space 2018, and causes air 2008 flowing inside to contain a component (hereinafter, simply called "air purification component") that purifies air together with micronized water. Space purification system 2100 performs sterilization and deodorization of indoor space 2018 by supplying indoor space 2018 with air 2009 (SA2) having circulated inside. Here, hypochlorous acid is used as the air purification component, and the water containing the air purification component is hypochlorous acid water.

As illustrated in Fig. 10, space purification system 2100 is mainly configured to include space purification device 2010, air-conditioning device 2015, and hypochlorous acid water generator 2030.

Space purification device 2010 includes outlet 2003, air purifier 2011, and air purification control unit 2041. Air-conditioning device 2015 includes inlet 2002, air blower 2013, refrigerant coil 2014, and air-conditioning control unit 2042. Each of space purification device 2010 and air-conditioning device 2015 has a housing constituting an outer frame of the respective device, and space purification device 2010 and air-conditioning device 2015 are connected via duct 2024. Inlet 2002 is formed on a side surface of air-conditioning device 2015, and outlet 2003 is formed on a side surface of space purification device 2010.

Inlet 2002 is an intake port for taking in air 2008 from indoor space 2018 into air-conditioning device 2015. Inlet 2002 is communicated via duct 2016 with indoor inlet 2016a provided on a ceiling or the like of indoor space 2018. Thus, inlet 2002 enables the air of indoor space 2018 to be suctioned into air-conditioning device 2015 via indoor inlet 2016a.

Outlet 2003 is a discharge port through which air 2009 (SA2) having circulated in space purification device 2010 is discharged to indoor space 2018. Outlet 2003 is communicated via duct 2017 with indoor outlet 2017a provided on a ceiling or the like of indoor space 2018. Thus, outlet 2003 enables air 2009 having circulated in space purification device 2010 to be blown out toward indoor space 2018 from indoor outlet 2017a.

Inside air-conditioning device 2015 and space purification device 2010 is provided with an air passage (prior stage air passage 2004, middle stage air passage 2005, and subsequent stage air passage 2006) causing inlet 2002 and outlet 2003 to be communicated each other via duct 2024. Prior stage air passage 2004 is an air passage adjacent to inlet 2002. Prior stage air passage 2004 is provided with air blower 2013 and refrigerant coil 2014.

Middle stage air passage 2005 is an air passage through which air 2008 having circulated through prior stage air passage 2004 circulates, at a position adjacent to prior stage air passage 2004 (duct 2024). Inside middle stage air passage 2005 is provided with air purifier 2011.

Subsequent stage air passage 2006 is an air passage adjacent to outlet 2003, and in subsequent stage air passage 2006, air 2008 having circulated through middle stage air passage 2005 circulates through air purifier 2011 and becomes air 2009 containing hypochlorous acid together with micronized water.

In air-conditioning device 2015 and space purification device 2010, air 2008 having been suctioned in from inlet 2002 circulates through prior stage air passage 2004, circulates through middle stage air passage 2005 and subsequent stage air passage 2006, and is blown out from outlet 2003 as air 2009.

Air blower 2013 of air-conditioning device 2015 is a device for conveying air 2008 (RA2) in indoor space 2018 from inlet 2002 into air-conditioning device 2015. Air blower 2013 is installed upstream of refrigerant coil 2014 in prior stage air passage 2004. In air blower 2013, on/off of operation behavior is controlled in accordance with blower output information from air-conditioning control unit 2042. Due to operation behavior of air blower 2013, air 2008 in indoor space 2018 is taken in to air-conditioning device 2015, and directed to refrigerant coil 2014.

Refrigerant coil 2014 is a member that is disposed downstream of air blower 2013 in prior stage air passage 2004 and cools or heats air 2008 to be introduced. Refrigerant coil 2014 changes an output state (cooling, heating, or off) in response to an output signal from air-conditioning control unit 2042, and adjusts the cooling capacity (cooling amount) or the heating capacity (heating amount) for air 2008 to be introduced. When refrigerant coil 2014 cools air 2008 to be introduced, air 2008 having been introduced is dehumidified, and therefore, the cooling capacity (cooling amount) for air 2008 can be deemed also as the dehumidifying capacity for air 2008 (dehumidification amount).

Refrigerant coil 2014 functions as a heat absorber or a heat radiator in a refrigeration cycle configured to include a compressor, a heat radiator, an expander, and a heat absorber, and is configured to absorb heat (cooling) or radiate heat (heating) when the refrigerant introduced from condenser unit 2020 circulates inside. More specifically, refrigerant coil 2014 is connected to condenser unit 2020 via refrigerant circuit 2021 through which a refrigerant flows. Condenser unit 2020 is an outdoor unit installed in outdoor space 2019, and includes compressor 2020a, expander 2020b, outdoor heat exchanger 2020c, blower fan 2020d, and four-way valve 2020e. Since condenser unit 2020 with a general configuration is used, detailed descriptions of these devices (compressor 2020a, expander 2020b, outdoor heat exchanger 2020c, blower fan 2020d, and four-way valve 2020e) will be omitted.

Since four-way valve 2020e is connected to the refrigeration cycle including refrigerant coil 2014, air-conditioning device 2015 can switch between a state of a cooling mode (dehumidification mode) in which the refrigerant circulates in a first direction by four-way valve 2020e and cools and dehumidifies the air (air 2008) and a state of a heating mode in which the refrigerant circulates in a second direction by four-way valve 2020e and heats the air (air 2008).

Here, the first direction is a direction in which the refrigerant circulates through compressor 2020a, outdoor heat exchanger 2020c, expander 2020b, and refrigerant coil 2014 in this order. The second direction is a direction in which the refrigerant circulates through compressor 2020a, refrigerant coil 2014, expander 2020b, and outdoor heat exchanger 2020c in this order. Refrigerant coil 2014 can cool or heat the air (air 2008) to be introduced.

Air purifier 2011 of space purification device 2010 is a unit for humidifying air 2008 taken therein, and causes hypochlorous acid together with micronized water to be contained the air during humidification. More specifically, air purifier 2011 includes mixing tank 2092, water level sensor 2090, humidifying motor 2011a, and humidifying nozzle 2011b. Air purifier 2011 has a centrifugal crushing type configuration in which humidifying nozzle 2011b is rotated using humidifying motor 2011a, the hypochlorous acid water stored in mixing tank 2092 of air purifier 2011 is suctioned by a centrifugal force, scattered, collided, and crushed in the surroundings (centrifugal direction), and moisture is contained in the passing air. Air purifier 2011 adjusts the humidifying capacity (humidification amount) by changing the rotation speed (hereinafter, rotation output value) of humidifying motor 2011a in response to an output signal from air purification control unit 2041. The humidification amount can be deemed also to be an addition amount by which the hypochlorous acid is added to the air. Note that air purifier 2011 corresponds to the "humidifying purifier" in the claims.

Water level sensor 2090 measures the water level of the hypochlorous acid water stored in mixing tank 2092, and outputs a measurement value to air purification control unit 2041. More specifically, water level sensor 2090 measures, as the water level of the hypochlorous acid water stored in mixing tank 2092, each of the water level at which mixing tank 2092 is brought into a water shortage state and the water level at which mixing tank 2092 is brought into a full state, and outputs the measurement values to air purification control unit 2041 as water level information. Note that in the present exemplary embodiment, the water level at which mixing tank 2092 is brought into a water shortage state is set to a water level in a state where the hypochlorous acid water amount has dropped to about 1/3 from the state where the hypochlorous acid water is full in mixing tank 2092.

Mixing tank 2092 is a tank in which hypochlorous acid water is stored in air purifier 2011, and can be deemed also to be a water storage. In mixing tank 2092, hypochlorous acid water with a predetermined concentration supplied from hypochlorous acid water supplier 2036 described later is mixed with the water supplied from water supplier 2050 described later, and is stored as mixed water including diluted hypochlorous acid water. Note that the hypochlorous acid water (mixed water) stored in mixing tank 2092 can be drained from mixing tank 2092 to the outside by drainage 2060 that behaves in response to an output signal from air purification control unit 2041.

Hypochlorous acid water generator 2030 includes electrolytic tank 2031, electrodes 2032, electromagnetic valve 2033, brine tank 2034, brine conveyance pump 2035, water level sensor 2039, and hypochlorous acid water supplier 2036.

Brine tank 2034 stores brine, and supplies the brine to electrolytic tank 2031 via brine conveyance pump 2035 in response to an output signal from air purification control unit 2041. Electrolytic tank 2031 stores the brine that is an electrolysis target supplied from brine tank 2034. Electrolytic tank 2031 is also supplied with tap water from a water supply pipe such as water supply via electromagnetic valve 2033 in response to an output signal from air purification control unit 2041, the supplied tap water and brine are mixed, and brine having a predetermined concentration is stored therein. Electrodes 2032 are disposed in electrolytic tank 2031, performs electrolysis of brine for a predetermined time by energization in response to an output signal from air purification control unit 2041, and generates hypochlorous acid water having a predetermined concentration. That is, electrolytic tank 2031 generates hypochlorous acid water by performing electrolysis of an aqueous chloride solution (e.g., aqueous sodium chloride solution) as an electrolyte between a pair of electrodes. Since a general device is used as electrolytic tank 2031, a detailed description will be omitted. Here, the electrolyte is an electrolyte from which hypochlorous acid water can be generated, and is not particularly limited as long as it contains chloride ions even in a small amount, and examples of the electrolyte include an aqueous solution in which sodium chloride, calcium chloride, magnesium chloride, or the like is dissolved as a solute. Hydrochloric acid is also acceptable. In the present exemplary embodiment, an aqueous sodium chloride solution (brine) in which sodium chloride is added to water is used as the electrolyte.

Water level sensor 2039 measures the water level in electrolytic tank 2031, and outputs the measurement value to air purification control unit 2041.

Hypochlorous acid water supplier 2036 supplies hypochlorous acid water from electrolytic tank 2031 into mixing tank 2092 of air purifier 2011 in response to an output signal from air purification control unit 2041. Hypochlorous acid water supplier 2036 includes hypochlorous acid water conveyance pump 2037 and water conveyance pipe 2038. Hypochlorous acid water conveyance pump 2037 feeds the hypochlorous acid water in electrolytic tank 2031 to water conveyance pipe 2038 in response to an output signal from air purification control unit 2041. Water conveyance pipe 2038 is connected between hypochlorous acid water conveyance pump 2037 and mixing tank 2092, and conveys the hypochlorous acid water to mixing tank 2092.

Water supplier 2050 supplies water to mixing tank 2092 in response to an output signal from air purification control unit 2041. Water supplier 2050 includes electromagnetic valve 2051 and water conveyance pipe 2052. Electromagnetic valve 2051 controls as to whether or not to flow, to water conveyance pipe 2052, the water supplied from a water supply pipe external of space purification device 2010 in response to an output signal from air purification control unit 2041. Water conveyance pipe 2052 is connected between electromagnetic valve 2051 and mixing tank 2092, through which water is conveyed to mixing tank 2092.

Drainage 2060 is connected to a bottom of mixing tank 2092, and drains the mixed water stored in mixing tank 2092 to the outside in response to an output signal from air purification control unit 2041. Drainage 2060 includes electromagnetic valve 2061 and water conveyance pipe 2062. Electromagnetic valve 2061 controls whether or not to flow the mixed water stored in mixing tank 2092 to the external drainage pipe in response to an output signal from air purification control unit 2041. Water conveyance pipe 2062 is connected between mixing tank 2092 and electromagnetic valve 2061, and supplies mixed water to the external drainage pipe.

In air purifier 2011, the hypochlorous acid water from hypochlorous acid water supplier 2036 and the water from water supplier 2050 are supplied to mixing tank 2092. The hypochlorous acid water and the water are mixed in mixing tank 2092 of air purifier 2011. Mixed water of hypochlorous acid water and water can also be called hypochlorous acid water. More specifically, in mixing tank 2092 of air purifier 2011, the hypochlorous acid water from hypochlorous acid water supplier 2036 or the water from water supplier 2050 is supplied to and mixed with the hypochlorous acid water remaining in mixing tank 2092. Air purifier 2011 releases the hypochlorous acid water to indoor space 2018 by centrifugally crushing the mixed water of the hypochlorous acid water and the water stored in mixing tank 2092. The micronized hypochlorous acid water is released to indoor space 2018 in a state where the liquid component is evaporated.

Operation device 2043 is installed on a wall surface of indoor space 2018. Operation device 2043 includes a user interface that a user can operate, and receives a temperature setting value and a humidity setting value from the user. Operation device 2043 includes temperature and humidity sensor 2044, and temperature and humidity sensor 2044 measures the temperature and humidity of air in indoor space 2018. It is sufficient to use a known technology for temperature and humidity sensor 2044 to measure the temperature and humidity, and therefore, a description is omitted here.

Operation device 2043 is connected to air purification control unit 2041 and air-conditioning control unit 2042 in a wired or wireless manner, and transmits a temperature setting value, a humidity setting value, a temperature measurement value, and a humidity measurement value to air purification control unit 2041 and air-conditioning control unit 2042. These pieces of information may all be transmitted together, any two or more may be transmitted together, or each may be transmitted. Operation device 2043 may transmit information to air purification control unit 2041, and air purification control unit 2041 may transfer information to air-conditioning control unit 2042.

Air-conditioning control unit 2042 of air-conditioning device 2015 receives the temperature setting value and the temperature measurement value, and controls refrigerant coil 2014 and condenser unit 2020 so that the temperature measurement value approaches the temperature setting value. Air-conditioning control unit 2042 increases the degree of heating as a difference between the temperature measurement value and the temperature setting value increases when the temperature measurement value is lower than the temperature setting value in the heating mode.

Next, air purification control unit 2041 of space purification device 2010 will be explained.

As treatment operations of hypochlorous acid water generator 2030 and space purification device 2010, air purification control unit 2041 controls each of a behavior regarding the electrolysis treatment in electrolytic tank 2031, a behavior regarding the supply treatment of hypochlorous acid water to air purifier 2011, a behavior regarding the supply treatment of water to air purifier 2011, a behavior regarding the humidifying purification treatment in air purifier 2011, and a behavior regarding the drainage treatment of the mixed water in air purifier 2011. Note that air purification control unit 2041 includes a computer system including a processor and a memory. The computer system functions as a control unit by the processor executing a program stored in the memory. The program to be executed by the processor is assumed here to be recorded in advance in the memory of the computer system, but may be provided in a manner being recorded in a non-transitory recording medium such as a memory card, or provided through a telecommunication line such as the Internet. Air purification control unit 2041 corresponds to the "control unit" in the claims.

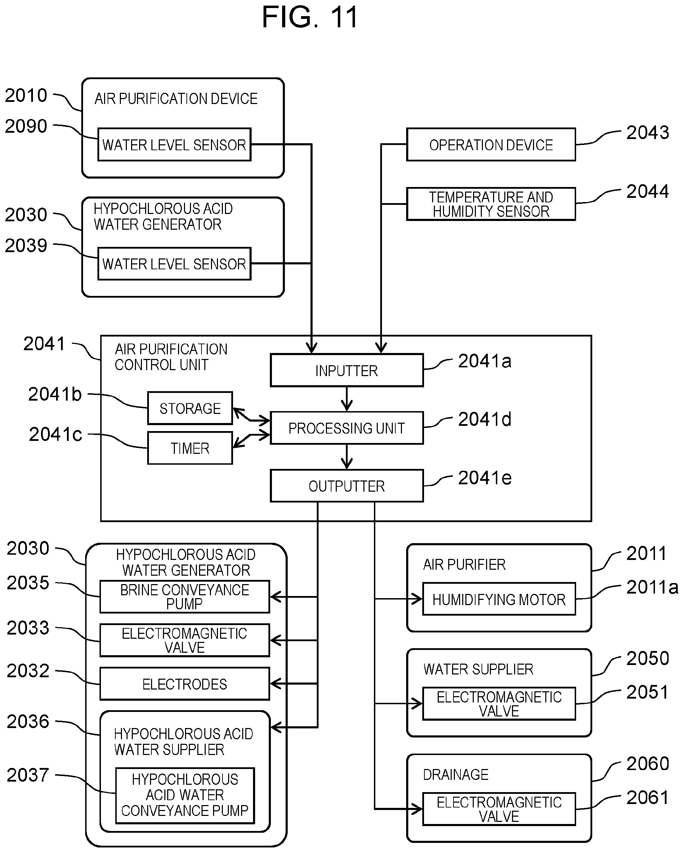

Fig. 11 is a block diagram illustrating the configuration of air purification control unit 2041 of space purification system 2100 according to the third exemplary embodiment. Specifically, as illustrated in Fig. 11, air purification control unit 2041 includes inputter 2041a, storage 2041b, timer 2041c, processing unit 2041d, and outputter 2041e.

### <Behavior regarding electrolysis treatment in electrolytic tank>

Air purification control unit 2041 executes the following treatment as a behavior regarding the electrolysis treatment in electrolytic tank 2031.

As a trigger of the electrolysis treatment of electrolytic tank 2031, air purification control unit 2041 receives and outputs, to processing unit 2041d, water level information (water shortage signal) from water level sensor 2039 and information regarding time (time information) from timer 2041c.

Processing unit 2041d specifies and outputs, to outputter 2041e, control information based on the water level information from water level sensor 2039, the time information from timer 2041c, and the setting information from storage 2041b. Here, the setting information includes information regarding start time or end time of hypochlorous acid water generation, information regarding a supply amount of tap water to be introduced into electrolytic tank 2031, information regarding an input amount of a liquid containing chloride ions in brine conveyance pump 2035, information regarding electrolysis conditions (such as time, current value, and voltage) in electrodes 2032, information regarding opening/closing timing of electromagnetic valve 2033, and information regarding on/off behavior of hypochlorous acid water conveyance pump 2037.

The electrolysis conditions in electrodes 2032 can be determined based on the water amount of tap water in electrolytic tank 2031, the chloride ion concentration, the electrolysis time, and a deterioration degree of electrodes 2032, are set with an algorithm created, and stored in storage 2041b.

Outputter 2041e outputs a signal (control signal) to each device (brine conveyance pump 2035, electromagnetic valve 2033, and hypochlorous acid water conveyance pump 2037) based on the received control information.

More specifically, brine conveyance pump 2035 first maintains a state of stopping based on a signal from outputter 2041e, and hypochlorous acid water conveyance pump 2037 maintains a state of stopping based on a signal from outputter 2041e.

Then, electromagnetic valve 2033 is opened based on a signal from outputter 2041e. Due to this, supply of tap water from the water supply pipe to electrolytic tank 2031 starts. Thereafter, electromagnetic valve 2033 is closed based on a signal from outputter 2041e having received the water level information (full) from water level sensor 2039. Due to this, electrolytic tank 2031 is brought into a state where tap water is supplied with a set supply amount.

Next, brine conveyance pump 2035 starts behavior based on a signal from outputter 2041e, conveys, to electrolytic tank 2031, a liquid containing a predetermined amount of chloride ions, and is stopped. Due to this, chloride ions dissolve in the tap water, and electrolytic tank 2031 is brought into a state where an aqueous solution (aqueous chloride solution) containing a predetermined amount of chloride ions is generated.

Then, electrodes 2032 start electrolysis of the aqueous chloride solution based on a signal from outputter 2041e, generates the hypochlorous acid water with the set conditions, and is stopped. The hypochlorous acid water generated by electrodes 2032 is brought into a state where, for example, the hypochlorous acid concentration is 100 ppm to 150 ppm (e.g., 120 ppm) and pH is 7.0 to 8.5 (e.g., 8.0).

As described above, air purification control unit 2041 executes the electrolysis treatment in electrolytic tank 2031, and hypochlorous acid water of a predetermined concentration and amount is generated.

### <Behavior regarding supply treatment of hypochlorous acid water to air purifier>

Air purification control unit 2041 executes the following treatment as a behavior regarding the supply treatment of hypochlorous acid water to air purifier 2011.

As a trigger of the supply treatment of hypochlorous acid water to air purifier 2011, air purification control unit 2041 outputs a hypochlorous acid water supply request to hypochlorous acid water generator 2030 (hypochlorous acid water supplier 2036) every time timer 2041c measures the operating time of humidifying motor 2011a and the operating time elapses a predetermined time (e.g., 60 minutes). Here, the predetermined time is time estimated in advance through an experimental evaluation, given the fact that hypochlorous acid in hypochlorous acid water vaporizes and decreases over time.

Specifically, processing unit 2041d specifies and outputs, to outputter 2041e, control information based on the information regarding time (time information) from timer 2041c and the setting information from storage 2041b. Here, the setting information includes information regarding the supply interval of the hypochlorous acid water (e.g., 60 minutes) and information regarding on/off behavior of hypochlorous acid water conveyance pump 2037.

Then, outputter 2041e outputs a signal (control signal) to hypochlorous acid water conveyance pump 2037 of hypochlorous acid water supplier 2036 based on the received control information.

Hypochlorous acid water conveyance pump 2037 operates based on a signal from outputter 2041e. Due to this, hypochlorous acid water generator 2030 starts supply of the hypochlorous acid water from electrolytic tank 2031 to air purifier 2011 (mixing tank 2092). Note that in order to ensure the concentration of the hypochlorous acid water stored in electrolytic tank 2031, when hypochlorous acid water is supplied from hypochlorous acid water generator 2030 to mixing tank 2092, the entire amount of hypochlorous acid water generated in electrolytic tank 2031 is supplied. Therefore, after the hypochlorous acid water is supplied, electrolytic tank 2031 is in an empty state, and generation of hypochlorous acid water is not started from a state where the hypochlorous acid water remains in electrolytic tank 2031. When the hypochlorous acid water in electrolytic tank 2031 is brought into a state where the entire amount has been supplied, water level sensor 2039 outputs a water shortage signal as water level information.

Thereafter, hypochlorous acid water conveyance pump 2037 is stopped based on a signal from outputter 2041e having received the information regarding the time (time required for supplying a prescribed amount) from timer 2041c. Due to this, hypochlorous acid water generator 2030 supplies the hypochlorous acid water from electrolytic tank 2031 to air purifier 2011 (mixing tank 2092) at a set supply amount.

As described above, air purification control unit 2041 executes the supply treatment of hypochlorous acid water from hypochlorous acid water generator 2030 (electrolytic tank 2031) to air purifier 2011. Note that the control by which air purification control unit 2041 performs supply of the hypochlorous acid water by hypochlorous acid water supplier 2036 at predetermined time intervals is "first control".

### <Behavior regarding supply treatment of water to air purifier>

Air purification control unit 2041 executes the following treatment as a behavior regarding the supply treatment of water to air purifier 2011.

As a trigger of the supply treatment of water to air purifier 2011, air purification control unit 2041 receives water level information (water shortage signal) from water level sensor 2090 of space purification device 2010, and outputs a water supply request to water supplier 2050.

Specifically, inputter 2041a receives and outputs, to processing unit 2041d, water level information (water shortage signal) from water level sensor 2090 of space purification device 2010.

Processing unit 2041d specifies and outputs, to outputter 2041e, control information based on the water level information (water shortage signal) from inputter 2041a, the information regarding time (time information) from timer 2041c, and the setting information from storage 2041b. Here, the setting information includes information regarding on/off behavior of electromagnetic valve 2051 of water supplier 2050.

Then, outputter 2041e then outputs a signal (control signal) to electromagnetic valve 2051 based on the received control information.

Electromagnetic valve 2051 operates based on a signal from outputter 2041e. Due to this, water supplier 2050 starts supply of water from an external water supply pipe to air purifier 2011 (mixing tank 2092), via water conveyance pipe 2052.

Thereafter, electromagnetic valve 2051 is stopped based on a signal from outputter 2041e having received the water level information (full signal) from water level sensor 2090 of space purification device 2010. Due to this, water supplier 2050 supplies water from the external water supply pipe to air purifier 2011 (mixing tank 2092) until the water reaches the set amount.

As described above, air purification control unit 2041 executes the supply treatment of water from water supplier 2050 to air purifier 2011. Note that the control by which air purification control unit 2041 supplies water by water supplier 2050 based on information (water shortage information) regarding the water level of mixing tank 2092 from water level sensor 2090 is "second control".

### <Behavior regarding humidifying purification treatment in air purifier>

Next, the behavior regarding the humidifying purification treatment in air purifier 2011 of air purification control unit 2041 will be explained.

Inputter 2041a receives user input information from operation device 2043, temperature and humidity information of the air of indoor space 2018 from temperature and humidity sensor 2044, and water level information of the hypochlorous acid water (mixed water) in mixing tank 2092 from water level sensor 2090. Inputter 2041a outputs each piece of the received information to processing unit 2041d.

Here, operation device 2043 is a terminal to which user input information (e.g., air volume, target temperature, target humidity, presence or absence of addition of hypochlorous acid, target supply amount level of hypochlorous acid, and the like) regarding space purification device 2010 is input, and is communicably connected to air purification control unit 2041 in a wireless or wired manner.

Temperature and humidity sensor 2044 is a sensor provided in target indoor space 2018, and senses the temperature and humidity of the air in indoor space 2018.

Storage 2041b stores the user input information received by inputter 2041a, and the supply setting information in supply behavior of hypochlorous acid to the air being circulated in the device. Storage 2041b outputs, to processing unit 2041d, the supply setting information having been stored. Note that the supply setting information in supply behavior of hypochlorous acid can also be deemed as humidification setting information in humidifying purification operation of air purifier 2011.

Timer 2041c outputs time information regarding current time to processing unit 2041d.

Processing unit 2041d receives various types of information (user input information, temperature and humidity information, and water level information) from inputter 2041a, the time information from timer 2041c, and the supply setting information from storage 2041b. Using the received user input information, time information, and supply setting information, processing unit 2041d specifies control information regarding humidifying purification operation behavior.

Specifically, processing unit 2041d specifies the humidification requirement amount necessary for indoor space 2018 based on a humidity difference between the target humidity stored in storage 2041b and the temperature and humidity information of the air in indoor space 2018 from temperature and humidity sensor 2044 at a regular interval by time information from timer 2041c. Then, processing unit 2041d specifies the control information regarding the humidifying purification operation behavior based on the specified humidification requirement amount and the supply setting information stored in storage 2041b. Then, processing unit 2041d outputs the specified control information to outputter 2041e.

In processing unit 2041d, when the water level information from water level sensor 2090 includes information (water shortage signal) regarding the water level indicating water shortage of the hypochlorous acid water (mixed water) in mixing tank 2092, outputter 2041e outputs, to outputter 2041e, a signal of water supply request for water supplier 2050. Furthermore, in processing unit 2041d, when the operating time of air purifier 2011 (humidifying motor 2011a) reaches a predetermined time (e.g., 60 minutes) based on the time information from timer 2041c, outputter 2041e outputs, to outputter 2041e, a signal of hypochlorous acid water supply request for hypochlorous acid water generator 2030. Note that in the present exemplary embodiment, the water level at which the hypochlorous acid water (mixed water) in mixing tank 2092 indicates water shortage is set to a water level in a state where the hypochlorous acid water amount has dropped to about 1/3 from the state where the hypochlorous acid water (mixed water) is full in mixing tank 2092.

Then, outputter 2041e outputs the received signals to air purifier 2011, hypochlorous acid water generator 2030 (hypochlorous acid water supplier 2036), and water supplier 2050, respectively.

Then, air purifier 2011 receives a signal from outputter 2041e and executes control of the operation behavior based on the received signal. At this time, hypochlorous acid water generator 2030 (hypochlorous acid water supplier 2036) receives a signal (signal for hypochlorous acid water supply request) from outputter 2041e, and executes behavior (first control) regarding the supply treatment of hypochlorous acid water to air purifier 2011 described above, based on the received signal. Water supplier 2050 receives a signal (signal for water supply request) from outputter 2041e, and executes behavior (second control) regarding the supply treatment of water to air purifier 2011 described above, based on the received signal.

As described above, air purification control unit 2041 executes, as the supply treatment, first control of performing supply of the hypochlorous acid water by hypochlorous acid water generator 2030 (hypochlorous acid water supplier 2036) at predetermined time intervals, and second control of performing supply of water by water supplier 2050 based on information (water shortage information) regarding a water level of mixing tank 2092 from water level sensor 2090, and stores the mixed water in mixing tank 2092. Then, when supplying hypochlorous acid water and water to and storing mixed water in mixing tank 2092, air purification control unit 2041 executes the humidifying purification treatment to the air circulating through space purification device 2010 (air purifier 2011) with supply cycle of the hypochlorous acid water (predetermined time intervals) and supply cycle of the water (every water shortage detection) being made different from each other.

### <Behavior regarding drainage treatment of mixed water of air purifier>

Air purification control unit 2041 executes the following treatment as a behavior regarding the drainage treatment of the mixed water stored in mixing tank 2092 of air purifier 2011.

As a trigger of the drainage treatment of the mixed water stored in mixing tank 2092, air purification control unit 2041 determines the presence or absence of implementation of the drainage treatment based on the information regarding the execution time (execution time information) of the second control in water supplier 2050.

Specifically, storage 2041b stores execution time information of the second control. Here, the execution time is the execution time of the second control executed after the start of the humidifying purification treatment behavior (hereinafter, also called "after the operation start") with the initial state (e.g., a state where mixing tank 2092 becomes full with supply of water and supply of hypochlorous acid water executed after the drainage treatment) of mixing tank 2092 as a starting point. The execution time of the second control is stored in storage 2041b each time the second control is executed. Note that storage 2041b also stores the time when the humidifying purification treatment behavior is started included in the execution time information of the second control.

Processing unit 2041d specifies a period in which the second control is not executed (non-execution period of the second control) based on the execution time information of the second control from storage 2041b and the information regarding the time (time information) from timer 2041c. Then, processing unit 2041d determines whether or not the specified non-execution period of the second control is greater than or equal to a reference time.

Here, the reference time is set to "6 hours" based on the hypochlorous acid concentration of the hypochlorous acid water supplied from hypochlorous acid water supplier 2036 such that the hypochlorous acid water concentration in mixing tank 2092 does not exceed a reference concentration only by continuous supply of the hypochlorous acid water by the first control. The reference concentration is set to such hypochlorous acid concentration that a user in indoor space 2018 does not feel unpleasant due to an odor of air 2009 (air 2009 containing hypochlorous acid) blown into indoor space 2018. Note that the reference time corresponds to the "predetermined period" in the claims.

As a result of the determination, when the non-execution period of the second control is greater than or equal to the reference time, processing unit 2041d specifies and outputs, to outputter 2041e, control information based on the time information from timer 2041c and the setting information from storage 2041b. Here, the setting information includes information regarding on/off behavior of electromagnetic valve 2061 of drainage 2060.

Then, outputter 2041e then outputs a signal (control signal) to electromagnetic valve 2061 based on the received control information.

Electromagnetic valve 2061 operates based on a signal from outputter 2041e. Due to this, drainage 2060 starts drainage of the mixed water from mixing tank 2092 to the external drainage pipe through water conveyance pipe 2062.

Thereafter, electromagnetic valve 2061 is stopped after a lapse of a predetermined time (e.g., 1 minute) based on a signal from outputter 2041e having received the time information from timer 2041c. Due to this, mixing tank 2092 is brought into an empty state with the stored mixed water drained entirely.

As described above, air purification control unit 2041 executes drainage treatment of the mixed water from mixing tank 2092 to the outside. Note that the control by which air purification control unit 2041 performs drainage of mixed water by drainage 2060 based on information (non-execution period of second control) regarding the execution time of the second control in water supplier 2050 is "third control".

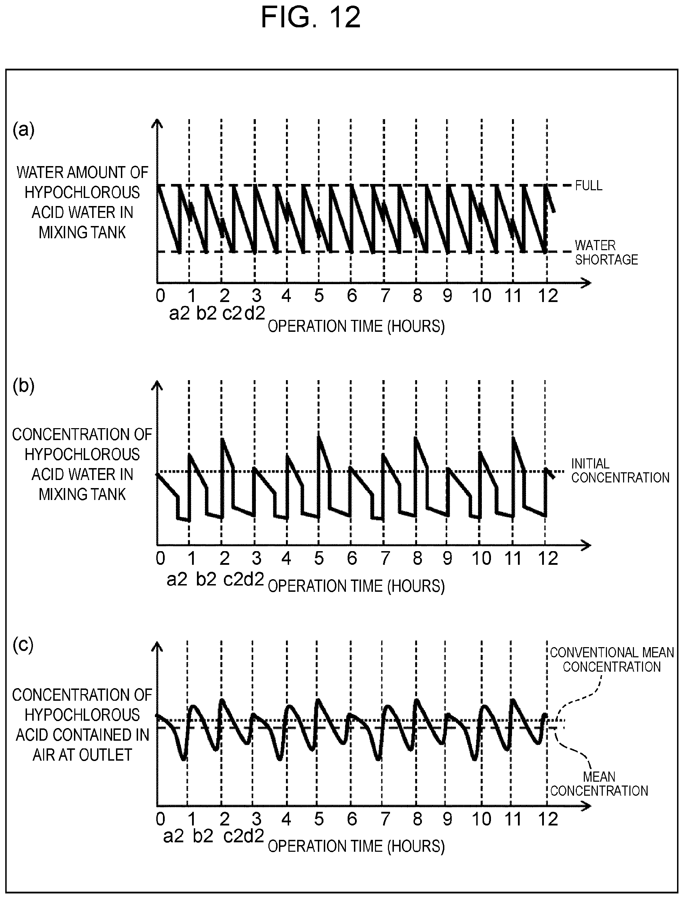

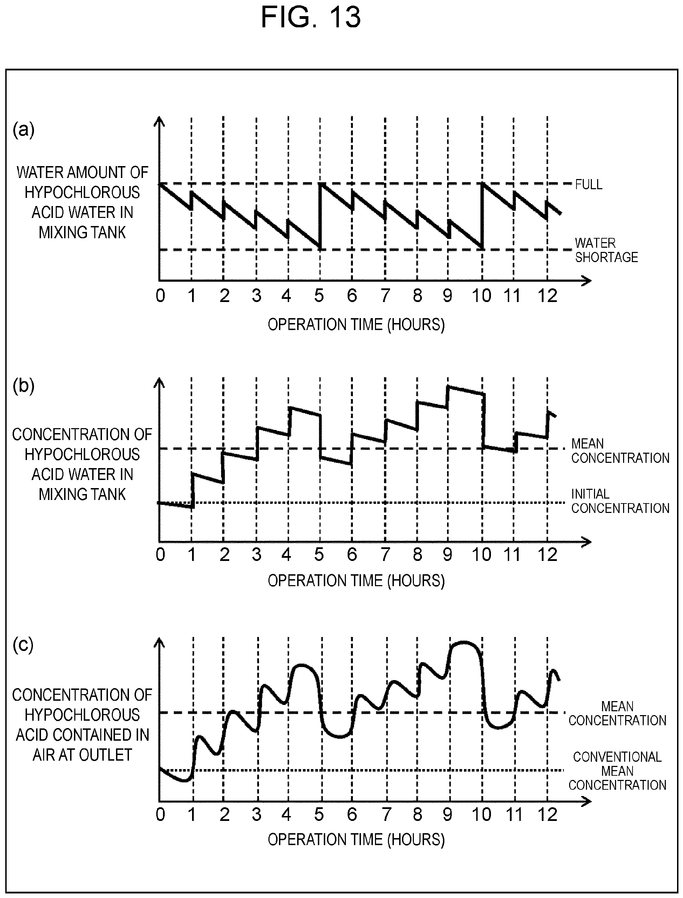

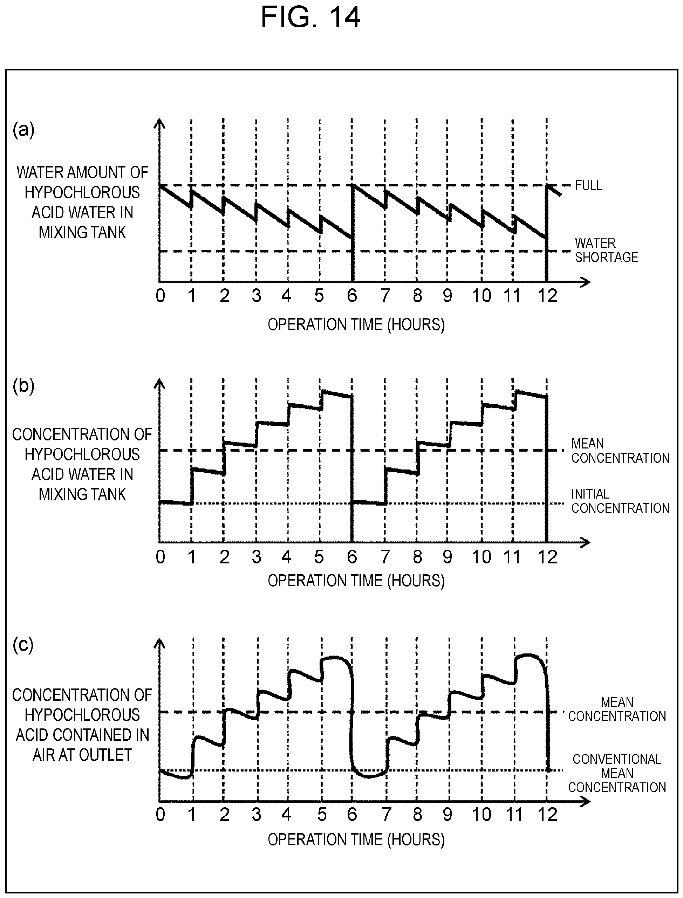

Next, with reference to Figs. 12 to 14, mixed water (mixed water mixed by performing the first control or the second control) in mixing tank 2092 of space purification device 2010 (air purifier 2011) in space purification system 2100 will be described. Fig. 12 is a schematic diagram illustrating temporal changes (winter: first example) of the water amount, the hypochlorous acid water concentration, and the hypochlorous acid concentration in space purification system 2100. More specifically, (a) of Fig. 12 illustrates a temporal change in the water amount of hypochlorous acid water (mixed water) in mixing tank 2092. (b) of Fig. 12 illustrates a history change of the concentration of hypochlorous acid water (mixed water) in mixing tank 2092. (c) of Fig. 12 illustrates a temporal change in the concentration of the hypochlorous acid contained in the air at outlet 2003. Fig. 13 is a schematic diagram illustrating temporal changes (summer: second example) of the water amount, the hypochlorous acid water concentration, and the hypochlorous acid concentration in space purification system 2100. More specifically, (a) of Fig. 13 illustrates a temporal change in the water amount of hypochlorous acid water (mixed water) in mixing tank 2092. (b) of Fig. 13 illustrates a history change of the concentration of hypochlorous acid water (mixed water) in mixing tank 2092. (c) of Fig. 13 illustrates a temporal change in the concentration of the hypochlorous acid contained in the air at outlet 2003. Fig. 14 is a schematic diagram illustrating temporal changes (summer: third example) of the water amount, the hypochlorous acid water concentration, and the hypochlorous acid concentration in space purification system 2100. More specifically, (a) of Fig. 14 illustrates a temporal change in the water amount of hypochlorous acid water (mixed water) in mixing tank 2092. (b) of Fig. 14 illustrates a history change of the concentration of hypochlorous acid water (mixed water) in mixing tank 2092. (c) of Fig. 14 illustrates a temporal change in the concentration of the hypochlorous acid contained in the air at outlet 2003.

Here, the supply of the hypochlorous acid water to mixing tank 2092 is executed every predetermined time (1 hour), and the supply of the water to mixing tank 2092 is executed every time water level sensor 2090 detects the water level at which mixing tank 2092 becomes water shortage.

Note that as described above, even when the hypochlorous acid water (mixed water) in mixing tank 2092 reaches the water level of water shortage, hypochlorous acid water (mixed water) remains in mixing tank 2092 by about 1/3 with respect to that at the time of full. In order to simplify the description, it is assumed that air purifier 2011 behaves with a constant humidification requirement amount during the humidifying purification operating time. Hereinafter, a predetermined amount of hypochlorous acid water supplied to mixing tank 2092 is also called "undiluted hypochlorous acid water".

First, a behavior situation in winter in Japan will be described. Note that in winter in Japan, since the outside air is dry, the humidification requirement amount to air purifier 2011 is large, and supply of water is performed at a shorter interval than supply of hypochlorous acid water. In other words, the water level in mixing tank 2092 becomes water shortage before the supply timing of the hypochlorous acid water.

Therefore, hereinafter, as the first example, the treatment under a humidifying purification condition in which the supply of water (second control) is executed 4 times and the supply of hypochlorous acid water (first control) is executed 3 times in a period in which the operating time after the operation start of air purifier 2011 is up to 3 hours will be described.

Note that the humidifying purification condition described above is a condition set based on controlling air purifier 2011 such that the number of times of performing the first control becomes smaller than the number of times of performing the second control when the humidification requirement amount for air purifier 2011 is greater than or equal to the first reference value. Here, the first reference value is a value set to distinguish between a situation where the humidity of the air is low and the air is dry in winter in Japan and a situation where the humidity of the air is high and the air is moist in summer in Japan.

In the first example, as illustrated in (a) of Fig. 12, the supply of hypochlorous acid water to mixing tank 2092 (first control) is executed at timings of 1 hour, 2 hours, 3 hours..., where the operation start is 0 hours. On the other hand, the supply of water (second control) to mixing tank 2092 is executed at timings of a2 hours, b2 hours, c2 hours, and d2 hours.... At the time point of 0 hours when the operation is started, the supply of the hypochlorous acid water and supply of the water to mixing tank 2092 are executed, and mixing tank 2092 is in a state (initial state) of being full with the hypochlorous acid water (mixed water) having a predetermined concentration.

Since the supply of the hypochlorous acid water (first control) and the supply of water (second control) concur with each other at the timing of 3 hours, the first example can be regarded as the supply of hypochlorous acid water (first control) and the supply of water (second control) by a 3-hour cycle. However, in the supply of water (second control) at this timing, since, in addition to the mixed water remaining in mixing tank 2092 by about 1/3 with respect to the full time, the supply amount of water is reduced by the amount of the supply amount of hypochlorous acid water, the hypochlorous acid water concentration in mixing tank 2092 becomes slightly higher than the initial state at the time point of 0 hours.

In the first example, the supply of hypochlorous acid water becomes 3 times while the supply of water is 4 times in a period in which the operating time after the operation start of air purifier 2011 is up to 3 hours (period in which the operating time is from over 0 hours to less than or equal to 3 hours). Thereafter, the operating time of 3 hours is regarded as the initial state (0 hours), and the same supply behavior is repeated every operating time of 3 hours.

That is, in the first example, it can be deemed that control is performed such that the number of times of performing the first control becomes smaller than the number of times of performing the second control when the humidification requirement amount for air purifier 2011 is greater than or equal to the first reference value.

Note that in winter in Japan, the humidification requirement amount to air purifier 2011 is large, and supply of water (second control) is executed at a shorter interval than the reference time (6 hours). Therefore, the second control is frequently executed, and drainage of the mixed water by the third control is not executed.

This will be described in more detail.

With reference to (a) of Fig. 12, a description will be given focusing on a temporal change in the water level of the hypochlorous acid water (mixed water) in mixing tank 2092.

At the early stage of the operation (0 hours), mixing tank 2092 is filled with mixed water (this is also hypochlorous acid water) of undiluted hypochlorous acid solution and water up to full. The humidifying purification operation reduces the water amount of the mixed water at a constant speed and detects water shortage at a timing at which a2 hours elapses from the operation start, and water is supplied from water supplier 2050 until mixing tank 2092 becomes full. Thereafter, while the water level of the mixed water is reduced at a constant speed by the humidifying purification operation, 1 hour, which is the supply timing of the hypochlorous acid water, elapses, and the first control is executed. That is, the undiluted hypochlorous acid water is supplied from hypochlorous acid water generator 2030 (hypochlorous acid water supplier 2036) to mixing tank 2092. Due to this, the water level in mixing tank 2092 rises slightly. Also thereafter, the water level of the mixed water decreases by the humidifying purification operation, the water shortage occurs again at the timing of b2 hours from the operation start, and the water is supplied from water supplier 2050 until mixing tank 2092 becomes full.

Thereafter, the first control is executed at a timing when the operating time after the operation start becomes 2 hours, and the undiluted hypochlorous acid water is supplied from hypochlorous acid water generator 2030 (hypochlorous acid water supplier 2036) to mixing tank 2092. Due to this, the water level in mixing tank 2092 rises slightly. Also thereafter, the water level of the mixed water decreases by the humidifying purification operation, the water shortage occurs again at the timing of c2 hours from the operation start, and the water is supplied from water supplier 2050 until mixing tank 2092 becomes full.

Thereafter, the operating time after the operation start reaches a timing of 3 hours (d2 hours). At this timing, since the water shortage detection and the supply timing of the undiluted hypochlorous acid water concur with each other, the first control and the second control are executed in this order. More specifically, as the first control, the undiluted hypochlorous acid water is first supplied from hypochlorous acid water generator 2030 (hypochlorous acid water supplier 2036) to mixing tank 2092. Thereafter, as the second control, water is supplied from water supplier 2050 until mixing tank 2092 becomes full. Due to this, the undiluted hypochlorous acid water and the water are supplied into mixing tank 2092, and the water level in mixing tank 2092 is brought into a state of being the same as that of the initial stage of operation (0 hours).

Thereafter, as in the period in which the operating time after the operation start is up to 3 hours, supply of water at the timing when the water shortage occurs, and supply of undiluted hypochlorous acid water at the supply timing of hypochlorous acid water are repeated.

Next, with reference to (b) of Fig. 12, a description will be given focusing on a temporal change in the concentration of hypochlorous acid water (mixed water) in mixing tank 2092.

At the beginning of the operation (0 hours), mixed water of undiluted hypochlorous acid water and water is mixed in mixing tank 2092 so as to have a predetermined concentration (initial concentration). Then, when the humidifying purification operation is started, the concentration of the hypochlorous acid water (mixed water) in mixing tank 2092 decreases with the elapse of time from the operation start up to a2 hours. This is because a constant proportion of hypochlorous acid with respect to the concentration of the hypochlorous acid water vaporizes and is given to the air due to the vapor pressure of hypochlorous acid being higher than that of water. Note that unless the hypochlorous acid vaporizes, the hypochlorous acid contained in the water merely consumed together with the water micronized by air purifier 2011, and therefore, the hypochlorous acid water does not change in terms of the concentration of the hypochlorous acid water in mixing tank 2092 although decreasing at a constant speed depending on the humidification amount. Even at a2 hours, which is the timing at which water level sensor 2090 detects the water shortage, the concentration of the hypochlorous acid water is not zero, which is because the hypochlorous acid water (mixed water) remains in mixing tank 2092 even in a state where a water shortage is detected, as mentioned earlier.

Then, when a2 hours elapses (water shortage detection) from the operation start, the hypochlorous acid water in mixing tank 2092 is diluted with water along with supply of the water from water supplier 2050, and therefore the concentration of the hypochlorous acid water in mixing tank 2092 decreases. Thereafter, the concentration of the hypochlorous acid water (mixed water) slightly decreases by vaporization of the hypochlorous acid until 1 hour, which is the supply timing of the hypochlorous water, elapses.

Then, when 1 hour, which is the supply timing of the hypochlorous acid water, elapses from the operation start, the concentration of the hypochlorous acid water in mixing tank 2092 rises to greater than or equal to the initial concentration along with supply of undiluted hypochlorous acid water from hypochlorous acid water generator 2030 (hypochlorous acid water supplier 2036). This is because a predetermined amount of hypochlorous acid water (undiluted hypochlorous acid water) supplied at the initial stage of operation is supplied to mixed water (water in a state of containing hypochlorous acid) having a water amount smaller than that of water supplied at the initial stage (0 hours) of the operation. Thereafter, the concentration of the hypochlorous acid water (mixed water) decreases by the vaporization of the hypochlorous acid up to b2 hours (water shortage detection) elapses from the operation start. Note that the decrease speed of the hypochlorous acid is higher than in the initial stage of operation because the content of the hypochlorous acid contained in the mixed water is large and accordingly, the vaporization amount of the hypochlorous acid becomes large.

Then, when b2 hours elapses (water shortage detection) from the operation start, the hypochlorous acid water in mixing tank 2092 is diluted with water along with supply of the water from water supplier 2050, and therefore the concentration of the hypochlorous acid water in mixing tank 2092 decreases. Thereafter, the concentration of the hypochlorous acid water (mixed water) slightly decreases by vaporization of the hypochlorous acid until 2 hours, which is the supply timing of the hypochlorous acid water, elapses.

Then, when 2 hours, which is the supply timing of the hypochlorous acid water from the operation start, elapses, the concentration of the hypochlorous acid water in mixing tank 2092 rises to greater than or equal to the initial concentration along with supply of undiluted hypochlorous acid water from hypochlorous acid water generator 2030 (hypochlorous acid water supplier 2036). Thereafter, the concentration of the hypochlorous acid water (mixed water) decreases by the vaporization of the hypochlorous acid up to c2 hours (water shortage detection) elapses from the operation start.

Then, when c2 hours elapses (water shortage detection) from the operation start, the hypochlorous acid water in mixing tank 2092 is diluted with water along with supply of the water from water supplier 2050, and therefore the concentration of the hypochlorous acid water in mixing tank 2092 decreases. Thereafter, the concentration of the hypochlorous acid water (mixed water) slightly decreases by vaporization of the hypochlorous acid until 3 hours, which is the supply timing of the hypochlorous acid water, elapses.

When 3 hours (d2 hours), which is the supply timing of the water (and the hypochlorous acid water), elapses from the operation start, the water and the undiluted hypochlorous acid water are supplied into mixing tank 2092, and the concentration of the hypochlorous acid water in mixing tank 2092 is brought into a close state to that in the initial stage of operation (0 hours). Thereafter, the concentration change of hypochlorous acid water (mixed water) is repeated same as ever.

Next, with reference to I of Fig. 12, a description will be given focusing on a temporal change in the concentration of the hypochlorous acid contained in air 2009 at outlet 2003.

The concentration of the hypochlorous acid contained in air 2009 released from outlet 2003 is determined by the humidification amount in air purifier 2011 and the concentration of the hypochlorous acid water in mixing tank 2092, but in the first example, since the humidification amount is constant, the concentration of the hypochlorous acid water in mixing tank 2092 is reflected. Therefore, as illustrated I(c) of Fig. 12, the concentration of the hypochlorous acid in air 2009 at outlet 2003 increases or decreases in accordance with an increase or a decrease in the concentration of the hypochlorous acid water in mixing tank 2092 as illustrated in (b) of Fig. 12.

Here, as conventionally, in a case where undiluted hypochlorous acid water and water are supplied to make full every time water level sensor 2090 detects water shortage, the state from the operation start (0 hours) to a2 hours is repeated until the timing of 3 hours (d2 hours). In this case, the mean concentration of the hypochlorous acid contained in air 2009 at outlet 2003 becomes the conventional mean concentration illustratIin (c) of Fig. 12, for example. On the other hand, in the first example, the state from the operation start (0 hours) to a2 hours is the same as the conventional state, but the state in the period from a2 hours to 3 hours is different from the conventional state. More specifically, in the period from a2 hours to 3 hours, as illustrated in (b) of Fig. 12, the period (part of period from 1 hour to b2 hours, period from 2 hours to c2 hours) in which the concentration of the hypochlorous acid water is higher than the initial concentration has become shorter than the period (period from a2 hours to 1 hour, period from b2 hour to 2 hours, period from c2 hours to 3 hours) in which it is smaller than the initial concentration. Therefore, in the period from the operation start (0 hours) to 3 hours, the mean concentration of the hypochlorous acid contained in air 2009 at outlet 2003 becomes a mean concentration lower than the conventional mean concentration.

As described above, as in the first example, when the hypochlorous acid water and water are supplied to mixing tank 2092 and the mixed water is stored, the supply cycle (predetermined time intervals) of the hypochlorous acid water and the supply cycle (every water shortage detection) of the water are made different, whereby the concentration of the hypochlorous acid contained in air 2009 at outlet 2003, i.e., the air blown out into indoor space 2018 can be reduced as compared with the case where the hypochlorous acid water and the water are supplied to mixing tank 2092 in the conventional method.

Next, a behavior situation in summer in Japan will be described. Note that in summer in Japan, since the outside air is moist and damp, the humidification requirement amount to air purifier 2011 is small, and supply of water is performed at a longer interval than supply of the hypochlorous acid water. That is, supply of hypochlorous acid water (first control) is performed many times before supply of water (second control) is performed.

Therefore, hereinafter, as the second example, the treatment under a humidifying purification condition in which the supply of water (second control) is executed 1 time and the supply of hypochlorous acid water (first control) is executed 5 times in a period in which the operating time after the operation start of air purifier 2011 is up to 5 hours will be described.

In the second example, as illustrated in (a) of Fig. 13, the supply of undiluted hypochlorous acid water to mixing tank 2092 (first control) is executed at timings of 1 hour, 2 hours, 3 hours, 4 hours, and 5 hours where the operation start is 0 hours. On the other hand, the supply of water (second control) to mixing tank 2092 is executed at timing in which water shortage detection by water level sensor 2090 is performed at the timing of 5 hours. At the time point of 0 hours when the operation is started, the supply of the hypochlorous acid water and supply of the water to mixing tank 2092 are executed, and mixing tank 2092 is in a state (initial state) of being full with the hypochlorous acid water (mixed water) having a predetermined concentration.

Specifically, at the timing when the operating time after the operation start reaches 1 hour, the first control is executed, and the undiluted hypochlorous acid water is supplied to mixing tank 2092. Thereafter, similar control is executed until the timing at which the operating time after the operation start is up to 4 hours.

Subsequently, the operating time after the operation start reaches a timing of 5 hours. At this timing, since the water shortage detection and the supply timing of the undiluted hypochlorous acid water concur with each other, the first control and the second control are executed in this order. More specifically, as the first control, the undiluted hypochlorous acid water is supplied from hypochlorous acid water generator 2030 (hypochlorous acid water supplier 2036) to mixing tank 2092. Subsequently, as the second control, water is supplied from water supplier 2050 until mixing tank 2092 becomes full. Due to this, the undiluted hypochlorous acid water and the water are supplied into mixing tank 2092, and the water level in mixing tank 2092 is brought into a state of being close to that of the initial stage of operation (0 hours).

Thereafter, the timing when the operating time is 5 hours is regarded as the initial state (0 hours), and the same supply behavior and are repeated every period of 5 hours.

This will be described in more detail.

First, with reference to (a) of Fig. 13, a description will be given focusing on a temporal change in the water level of the hypochlorous acid water (mixed water) in mixing tank 2092.

At the early stage of the operation (0 hours), mixing tank 2092 is filled with mixed water (this is also hypochlorous acid water) of undiluted hypochlorous acid solution and water up to full. Then, when the humidifying purification operation is started, the humidifying purification operation reduces the water amount of the mixed water at a constant speed, and 1 hour, which is the supply timing of the hypochlorous acid water, elapses.

Then, in the humidifying purification operation in the period in which the operating time is up to 1 hour from the operation start, since supply of water by the second control has not been executed, the non-execution period (about 1 hour) of the second control has not reaches the reference time (6 hours). Therefore, the first control is executed without the drainage of the mixed water stored in mixing tank 2092 being executed, and the undiluted hypochlorous acid water is supplied from hypochlorous acid water generator 2030 (hypochlorous acid water supplier 2036) to mixing tank 2092. Due to this, the water level in mixing tank 2092 slightly rises. Also thereafter, the water level of the mixed water decreases by the humidifying purification operation, 2 hours, which is the supply timing of the hypochlorous acid water, elapses.

In the humidifying purification operation in the period in which the operating time is up to 2 hours from the operation start, the non-execution period (about 2 hours) of the second control has not reached the reference time (6 hours). Therefore, the first control is executed without the drainage of the mixed water stored in mixing tank 2092 being executed, and the undiluted hypochlorous acid water is supplied from hypochlorous acid water generator 2030 (hypochlorous acid water supplier 2036) to mixing tank 2092. Due to this, the water level in mixing tank 2092 slightly rises. Thereafter, similar control is executed until the timing at which the operating time after the operation start is up to 4 hours. Thus, in the second example, while the water amount of the mixed water decreases at a constant speed by the humidifying purification operation, since the undiluted hypochlorous acid water is supplied, the water amount of the mixed water decreases in accordance with the difference between the humidification amount and the supply amount while the water amount of the mixed water increases.

Subsequently, the operating time after the operation start reaches a timing of 5 hours. In the humidifying purification operation in the period in which the operating time is up to 5 hours from the operation start, the non-execution period (about 5 hours) of the second control has not reached the reference time (6 hours). At this timing, since the water shortage detection and the supply timing of the undiluted hypochlorous acid water concur with each other, the first control and the second control are executed in this order without execution of the drainage of the mixed water stored in mixing tank 2092. As described above, as the first control, the undiluted hypochlorous acid water is first supplied from hypochlorous acid water generator 2030 (hypochlorous acid water supplier 2036) to mixing tank 2092. Thereafter, as the second control, water is supplied from water supplier 2050 until mixing tank 2092 becomes full. Due to this, the undiluted hypochlorous acid water and the water are supplied into mixing tank 2092, and the water level in mixing tank 2092 is brought into a state of being close to that of the initial stage of operation (0 hours). Note that since supply of water by the water supplier 2050 has been performed, the non-execution period of the second control is specified again with this timing as a starting point.

Thereafter, the timing when the operating time is 5 hours is regarded as the initial state (0 hours), and the same supply behavior and drainage operation are repeated every period of 5 hours. More specifically, same as ever, supply of undiluted hypochlorous acid water by the first control at the supply timing of hypochlorous acid water and supply of water by the second control at the supply timing of water are repeated. The water level of the hypochlorous acid water (mixed water) in mixing tank 2092 increases or decreases corresponding to each behavior.

Next, with reference to (b) of Fig. 13, a description will be given focusing on a temporal change in the concentration of hypochlorous acid water (mixed water) in mixing tank 2092.

At the beginning of the operation (0 hours), mixed water of undiluted hypochlorous acid water and water is mixed in mixing tank 2092 so as to have a predetermined concentration (initial concentration). Then, when the humidifying purification operation is started, the concentration of the hypochlorous acid water (mixed water) in mixing tank 2092 decreases with the elapse of time from the operation start up to 1 hour. This is because a constant proportion of hypochlorous acid with respect to the concentration of the hypochlorous acid water vaporizes and is given to the air due to the vapor pressure of hypochlorous acid being higher than that of water, as mentioned above.

Then, when 1 hour, which is the supply timing of the hypochlorous acid water, elapses from the operation start, the concentration of the hypochlorous acid water in mixing tank 2092 rises to greater than or equal to the initial concentration along with supply of undiluted hypochlorous acid water from hypochlorous acid water generator 2030 (hypochlorous acid water supplier 2036). This is because, as described above, a predetermined amount of hypochlorous acid water (undiluted hypochlorous acid water) supplied at the initial stage of operation is supplied to mixed water (water in a state of containing hypochlorous acid) having a water amount smaller than that of the mixed water stored at the initial stage (0 hours) of the operation. Thereafter, the concentration of the hypochlorous acid water (mixed water) slightly decreases by vaporization of the hypochlorous acid until 2 hours elapses from the operation start.

Then, when 2 hours, which is the supply timing of the hypochlorous acid water, elapses from the operation start, the concentration of the hypochlorous acid water in mixing tank 2092 further rises along with supply of undiluted hypochlorous acid water from hypochlorous acid water generator 2030 (hypochlorous acid water supplier 2036). Similarly, until the timing of 4 hours thereafter, the concentration change of the hypochlorous acid water (mixed water) is repeated, and the concentration of the hypochlorous acid water (mixed water) gradually rises.

Then, when 5 hours, which is the supply timing of water and undiluted hypochlorous acid water, elapses from the operation start, the water and the undiluted hypochlorous acid water are supplied into mixing tank 2092, and the concentration of the hypochlorous acid water in mixing tank 2092 decreases because the hypochlorous acid water in mixing tank 2092 is diluted along with supply of water from water supplier 2050. However, since the hypochlorous acid water and the water are supplied in a state where about 1/3 of the hypochlorous acid water remains, the concentration of the hypochlorous acid water in mixing tank 2092 is not diluted to the initial concentration in the initial state. Thereafter, the concentration of the hypochlorous acid water tends to rise overall along with the lapse of time, but basically, the concentration change of hypochlorous acid water (mixed water) is repeated same as ever.

Next, with reference to (c) of Fig. 13, a description will be given focusing on a temporal change in the concentration of the hypochlorous acid contained in air 2009 at outlet 2003.

Since the concentration of the hypochlorous acid contained in air 2009 released from outlet 2003 is determined by the humidification amount in air purifier 2011 and the concentration of the hypochlorous acid water in mixing tank 2092 as in winter in Japan, as illustrated in (c) of Fig. 13, the concentration of the hypochlorous acid contained in air 2009 at outlet 2003 increases or decreases in accordance with an increase or a decrease in the concentration of the hypochlorous acid water in mixing tank 2092 illustrated in (b) of Fig. 13.

Here, as conventionally, in a case where undiluted hypochlorous acid water and water are supplied to make full every time water level sensor 2090 detects water shortage, the concentration of the hypochlorous acid water continues to decrease from the operation start (0 hours) to 5 hours. Strictly speaking, the concentration of the hypochlorous acid water continues to decrease in the period, within 5 hours, from the full state to when water shortage is detected. In this case, the mean concentration of the hypochlorous acid contained in air 2009 at outlet 2003 becomes the conventional mean concentration illustrated in (c) of Fig. 13, for example.

On the other hand, in the second example, the state from the operation start (0 hours) to 1 hour is the same as the conventional state, but the state in the period in which the operating time is from 1 hour to 5 hours is different from the conventional state. More specifically, in the period in which the operating time is from 1 hour to 5 hours, as illustrated in (b) of Fig. 13, the period in which the concentration of the hypochlorous acid water is higher than the initial concentration is far longer than the period in which it is lower than the initial concentration. Therefore, in the period from the operation start (0 hours) to 5 hours, the mean concentration of the hypochlorous acid contained in air 2009 at outlet 2003 becomes a mean concentration higher than the conventional mean concentration.

Then, also when the operating time is 5 hours and thereafter, the concentration change of the mixed water is repeated every 5 hours with 5 hours as 1 cycle, and it is therefore possible to keep adjusting the concentration of the hypochlorous acid water within a range of less than or equal to a certain concentration without the concentration of the hypochlorous acid water keeping rising.

Next, as the third example, the treatment under a humidifying purification condition in which the supply of water (second control) is not executed in a period in which the operating time after the operation start of air purifier 2011 is up to 6 hours will be described. That is, the third example becomes treatment under a condition of being less likely to be humidified than the second example in summer in Japan.

In the third example, as illustrated in (a) of Fig. 14, the supply of undiluted hypochlorous acid water to mixing tank 2092 (first control) is executed at timings of 1 hour, 2 hours, 3 hours..., where the operation start is 0 hours. On the other hand, the supply of water (second control) to mixing tank 2092 is not executed because the consumption amount associated with the humidifying purification is smaller than that in the second example and therefore the water shortage detection by water level sensor 2090 is not performed. At the time point of 0 hours when the operation is started, the supply of the hypochlorous acid water and supply of the water to mixing tank 2092 are executed, and mixing tank 2092 is in a state (initial state) of being full with the hypochlorous acid water (mixed water) having a predetermined concentration.

Specifically, at the timing when the operating time after the operation start reaches 1 hour, the first control is executed, and the undiluted hypochlorous acid water is supplied to mixing tank 2092. Thereafter, similar control is executed until the timing at which the operating time after the operation start is up to 5 hours.

Subsequently, the operating time after the operation start reaches a timing of 6 hours. At this timing, since the non-execution period of the second control is 6 hours, it is determined that the non-execution period of the second control is greater than or equal to the reference time (6 hours). In response to the determination result, the third control is executed, and the mixed water in mixing tank 2092 is drained entirely. Furthermore, after the third control is executed, the supply of undiluted hypochlorous acid water and the supply of water are newly executed to mixing tank 2092, and mixing tank 2092 is brought into a state of full with the hypochlorous acid water (mixed water) having a predetermined concentration that is the same as the initial state.

Thereafter, the timing of 6 hours is regarded as the initial state (0 hours), and the same supply behavior and drainage operation are repeated every period of 6 hours.

This will be described in more detail.

First, with reference to (a) of Fig. 14, a description will be given focusing on a temporal change in the water level of the hypochlorous acid water (mixed water) in mixing tank 2092.

At the early stage of the operation (0 hours), mixing tank 2092 is filled with mixed water (this is also hypochlorous acid water) of undiluted hypochlorous acid solution and water up to full. Then, the humidifying purification operation reduces the water amount of the mixed water at a constant speed, and 1 hour, which is the supply timing of the hypochlorous acid water, elapses. Then, the drainage determination of the mixed water is executed at this timing of 1 hour.

At the early stage of the operation (0 hours), mixing tank 2092 is filled with mixed water (this is also hypochlorous acid water) of undiluted hypochlorous acid solution and water up to full. Then, when the humidifying purification operation is started, the humidifying purification operation reduces the water amount of the mixed water at a constant speed, and 1 hour, which is the supply timing of the hypochlorous acid water, elapses.

Then, in the humidifying purification operation in the period in which the operating time is up to 1 hour from the operation start, since supply of water by the second control has not been executed, the non-execution period (about 1 hour) of the second control has not reaches the reference time (6 hours). Therefore, the first control is executed without the drainage of the mixed water stored in mixing tank 2092 being executed, and the undiluted hypochlorous acid water is supplied from hypochlorous acid water generator 2030 (hypochlorous acid water supplier 2036) to mixing tank 2092. Due to this, the water level in mixing tank 2092 slightly rises. Also thereafter, the water level of the mixed water decreases by the humidifying purification operation, 2 hours, which is the supply timing of the hypochlorous acid water, elapses.

In the humidifying purification operation in the period in which the operating time is up to 2 hours from the operation start, the non-execution period (about 2 hours) of the second control has not reached the reference time (6 hours). Therefore, the first control is executed without the drainage of the mixed water stored in mixing tank 2092 being executed, and the undiluted hypochlorous acid water is supplied from hypochlorous acid water generator 2030 (hypochlorous acid water supplier 2036) to mixing tank 2092. Due to this, the water level in mixing tank 2092 slightly rises. Thereafter, similar control is executed until the timing at which the operating time after the operation start is up to 5 hours. Thus, in the third example, while the water amount of the mixed water decreases at a constant speed by the humidifying purification operation, since the undiluted hypochlorous acid water is supplied, the water amount of the mixed water decreases in accordance with the difference between the humidification amount and the supply amount while the water amount of the mixed water increases.

Subsequently, the operating time after the operation start reaches a timing of 6 hours. In the humidifying purification operation in the period in which the operating time is up to 6 hours from the operation start, the non-execution period (about 6 hours) of the second control becomes greater than or equal to the reference time (6 hours), and is determined to have reached the reference time. In response to the determination result, the third control is executed, and the mixed water in mixing tank 2092 is drained. Furthermore, after the execution of drainage of the mixed water by the third control, the supply of undiluted hypochlorous acid water and the supply of water are newly executed to mixing tank 2092, and mixing tank 2092 is brought into a state of full with the hypochlorous acid water (mixed water) having a predetermined concentration that is the same as the initial state (0 hours). Note that since supply of water by the water supplier 2050 has been performed, the non-execution time of the second control is specified again with this timing as a starting point.

Thereafter, the timing when the operating time is 6 hours is regarded as the initial state (0 hours), and the same supply behavior and drainage operation are repeated every period of 6 hours. More specifically, same as ever, supply of undiluted hypochlorous acid water by the first control at the supply timing of hypochlorous acid water and supply of water by the second control at the supply timing of water are repeated. The water level of the hypochlorous acid water (mixed water) in mixing tank 2092 increases or decreases corresponding to each behavior.

Next, with reference to (b) of Fig. 14, a description will be given focusing on a temporal change in the concentration of hypochlorous acid water (mixed water) in mixing tank 2092.

At the beginning of the operation (0 hours), mixed water of undiluted hypochlorous acid water and water is mixed in mixing tank 2092 so as to have a predetermined concentration (initial concentration). Then, when the humidifying purification operation is started, the concentration of the hypochlorous acid water (mixed water) in mixing tank 2092 decreases with the elapse of time from the operation start up to 1 hour. This is because a constant proportion of hypochlorous acid with respect to the concentration of the hypochlorous acid water vaporizes and is given to the air due to the vapor pressure of hypochlorous acid being higher than that of water, as mentioned above.

Then, when 1 hour, which is the supply timing of the hypochlorous acid water, elapses from the operation start, the concentration of the hypochlorous acid water in mixing tank 2092 rises to greater than or equal to the initial concentration along with supply of undiluted hypochlorous acid water from hypochlorous acid water generator 2030 (hypochlorous acid water supplier 2036). This is because, as described above, a predetermined amount of hypochlorous acid water (undiluted hypochlorous acid water) supplied at the initial stage of operation is supplied to mixed water (water in a state of containing hypochlorous acid) having a water amount smaller than that of the mixed water stored at the initial stage (0 hours) of the operation. Thereafter, the concentration of the hypochlorous acid water (mixed water) slightly decreases by vaporization of the hypochlorous acid until 2 hours elapses from the operation start.

Then, when 2 hours, which is the supply timing of the hypochlorous acid water, elapses from the operation start, the concentration of the hypochlorous acid water in mixing tank 2092 further rises along with supply of undiluted hypochlorous acid water from hypochlorous acid water generator 2030 (hypochlorous acid water supplier 2036). Similarly, until the timing of 5 hours thereafter, the concentration change of the hypochlorous acid water (mixed water) is repeated, and the concentration of the hypochlorous acid water (mixed water) gradually rises.

When 6 hours, which is the supply timing of the undiluted hypochlorous acid water, elapses from the operation start, the drainage timing comes based on the drainage determination, therefore, after the hypochlorous acid water (mixed water) in mixing tank 2092 is drained entirely, water and undiluted hypochlorous acid water are supplied into mixing tank 2092, and the concentration of the hypochlorous acid water in mixing tank 2092 is brought into a similar state to that in the initial stage of operation (0 hours). Thereafter, the concentration change of hypochlorous acid water (mixed water) is repeated in the same manner as ever.

Next, with reference to (c) of Fig. 14, a description will be given focusing on a temporal change in the concentration of the hypochlorous acid contained in air 2009 at outlet 2003.

Since the concentration of the hypochlorous acid contained in air 2009 released from outlet 2003 is determined by the humidification amount in air purifier 2011 and the concentration of the hypochlorous acid water in mixing tank 2092 as in the second example, as illustrated in (c) of Fig. 14, the concentration of the hypochlorous acid contained in air 2009 at outlet 2003 increases or decreases in accordance with an increase or a decrease in the concentration of the hypochlorous acid water in mixing tank 2092 illustrated in (b) of Fig. 14.

Here, as conventionally, in a case where undiluted hypochlorous acid water and water are supplied to make full every time water level sensor 2090 detects water shortage, the concentration of the hypochlorous acid water continues to decrease from the operation start (0 hours) to 6 hours. Strictly speaking, the concentration of the hypochlorous acid water continues to decrease in the period, within 6 hours, from the full state to when water shortage is detected. In this case, the mean concentration of the hypochlorous acid contained in air 2009 at outlet 2003 becomes the conventional mean concentration illustrated in (c) of Fig. 14, for example.

On the other hand, in the third example, the state from the operation start (0 hours) to 1 hour is the same as the conventional state, but the state in the period from 1 hour to 6 hours is different from the conventional state. More specifically, in the period from 1 hour to 6 hours, as illustrated in (b) of Fig. 14, the period in which the concentration of the hypochlorous acid water is higher than the initial concentration is far longer than the period in which it is lower than the initial concentration. Therefore, in the period from the operation start (0 hours) to 6 hours, the mean concentration of the hypochlorous acid contained in air 2009 at outlet 2003 becomes a mean concentration higher than the conventional mean concentration.

Then, also for 6 hours and thereafter, the concentration change of the mixed water is repeated every 6 hours with 6 hours as 1 cycle, and it is therefore possible to keep adjusting the concentration of the hypochlorous acid water within a range of less than or equal to a certain concentration without the concentration of the hypochlorous acid water keeping rising. That is, when the humidifying purification operation is continued, the concentration of the hypochlorous acid water in mixing tank 2092 may excessively rise, however, by providing control of drainage determination in accordance with the non-execution period by the second control, it is possible to reset the concentration of the hypochlorous acid water in mixing tank 2092 at regular intervals, and eventually the addition amount of hypochlorous acid contained in air 2009 at outlet 2003, and it is possible to control the supply amount of a hypochlorous acid gas to indoor space 2018.

As described above, space purification system 2100 supplies hypochlorous acid water into mixing tank 2092 every preset time (e.g., 1 hour) as the first control, executes the treatment of supplying water based on the water level information (water shortage signal) from water level sensor 2090 as the second control, and drains the mixed water in mixing tank 2092 based on the non-execution period of the second control as the third control. Furthermore, air purification control unit 2041 of space purification system 2100 makes the number of times of performing the first control within a predetermined period different from the number of times of performing the second control within a predetermined period based on the humidification requirement amount (a humidification requirement amount corresponding to winter in Japan or a humidification requirement amount corresponding to summer in Japan) required to air purifier 2011. Due to this, in a state where the humidification requirement amount is high as in winter in Japan, air 2009 in a state where the content of the hypochlorous acid amount is small can be released to indoor space 2018 as compared with the conventional method, and in a state where the humidification requirement amount is low as in summer in Japan, air 2009 in a state where the content of the hypochlorous acid amount is large can be released to indoor space 2018 as compared with the conventional method. Furthermore, when the humidifying purification operation is continued for a long time, an excessive increase in the hypochlorous acid concentration released to indoor space 2018 can be suppressed.

That is, by operating the supply of hypochlorous acid water, the supply of water, and the drainage of mixed water by triggers separate from one another, it is possible to adjust the concentration of the hypochlorous acid water in mixing tank 2092 (concentration of the hypochlorous acid contained in air 2009 blown out to indoor space 2018) by simple control (first control, second control, and third control).

As described above, space purification system 2100 according to the present third exemplary embodiment can achieve the following effects.
(1) Space purification system 2100 includes hypochlorous acid water generator 2030 that generates hypochlorous acid water, hypochlorous acid water supplier 2036 that supplies hypochlorous acid water from hypochlorous acid water generator 2030 to mixing tank 2092, water supplier 2050 that supplies water to mixing tank 2092, water level sensor 2090 for detecting a water level of mixing tank 2092, air purifier 2011 that micronizes and releases, into air, mixed water of the hypochlorous acid water and the water stored in mixing tank 2092, and air purification control unit 2041 that controls supply treatment in hypochlorous acid water supplier 2036 and water supplier 2050 and drainage treatment of the mixed water stored in mixing tank 2092. Air purification control unit 2041 (i) executes, as the control of the supply treatment, first control of causing the hypochlorous acid water supplier 2036 to perform supply of the hypochlorous acid water at predetermined time intervals (e.g., 60 minutes), and second control of causing the water supplier 2050 to perform supply of the water based on information (water shortage information) regarding the water level of mixing tank 2092 from water level sensor 2090, and (ii) executes, as the control of the drainage treatment, third control of causing the mixing tank to drain the mixed water stored in mixing tank 2092 when the second control is not executed for a predetermined period (e.g., 6 hours) since the supply of the water by water supplier 2050 is performed.

Due to this, when air having high relative humidity is ventilated as in summer in Japan, since the consumption amount of the mixed water stored in mixing tank 2092 is small, the supply frequency (the number of times of performing the first control) of hypochlorous acid water to mixing tank 2092 increases, and the mixed water is micronized and released into the air in a state where the hypochlorous acid concentration of the mixed water in mixing tank 2092 is high. At this time, by executing the third control when having not executed the second control for a predetermined period (e.g., 6 hours) since performing supply of water by water supplier 2050, draining the mixed water stored in mixing tank 2092, and resetting the mixed water in mixing tank 2092, it is possible to suppress an excessive increase in the hypochlorous acid concentration in mixing tank 2092. As a result, even in a situation where the micronized hypochlorous acid water hardly vaporize, hypochlorous acid raised to a predetermined concentration contained in the air can be released into indoor space 2018.

On the other hand, when air having low relative humidity is ventilated as in winter in Japan, since the consumption amount of the mixed water stored in mixing tank 2092 is large, the supply frequency of water to mixing tank 2092 (the number of times of performing the second control) increases, and the mixed water is micronized and released into the air in a state where the hypochlorous acid concentration of the mixed water in mixing tank 2092 is low. As a result, even in a situation where the micronized hypochlorous acid water easily vaporizes, hypochlorous acid attenuated to a predetermined concentration contained in the air can be released into indoor space 2018.

That is, space purification system 2100 can easily adjust the amount of hypochlorous acid to be released into the air.

(2) Space purification system 2100 can return the state in mixing tank 2092 to the operation initial state before the hypochlorous acid water concentration in mixing tank 2092 excessively increases even when a long time operation (e.g., 24 hours) is performed. That is, space purification system 2100 can easily adjust the amount of hypochlorous acid to be released into the air.

(3) In space purification system 2100, in the supply treatment, air purification control unit 2041 performs control such that the number of times of performing the first control becomes smaller than the number of times of performing the second control when the humidification requirement amount required for air purifier 2011 is greater than or equal to a first reference value, and performs control such that the number of times of performing the first control becomes larger than the number of times of performing the second control when the humidification requirement amount is less than the first reference value. This enables space purification system 2100 can micronize and release, into the air, the mixed water in a state where the hypochlorous acid concentration in mixing tank 2092 is high when the humidification requirement amount is less than the first reference value in the supply treatment. On the other hand, the mixed water can be micronized and released into the air in a state where the hypochlorous acid concentration in mixing tank 2092 is low when the humidification requirement amount is greater than or equal to the first reference value. In other words, based on the humidification requirement amount, space purification system 2100 can give hypochlorous acid to air 2009 released from air purifier 2011 under a condition suitable for the environment of indoor space 2018.

The present disclosure has been described above based on the exemplary embodiments. It is understood by those skilled in the art that these exemplary embodiments are merely examples, that the components or the treatment processes of those can be combined as various modifications, and that such modifications also fall within the scope of the present disclosure.

In the first example, the second example, and the third example in space purification system 2100 according to the present third exemplary embodiment, it has been described that air purifier 2011 behaves at a constant humidification requirement amount during the humidifying purification operating time, but actually, air purifier 2011 behaves at a humidification requirement amount specified based on a humidity difference between the target humidity and the humidity of the air in indoor space 2018 every constant time.

In the first example, the second example, and the third example in space purification system 2100 according to the present third exemplary embodiment, the description has been given with a situation where the water shortage detection and the supply timing of the undiluted hypochlorous acid water concur with each other, but actually, in most cases, the water shortage detection and the supply timing of the undiluted hypochlorous acid water are different from each other. In such situation, it is preferable that air purification control unit 2041 not execute the third control immediately when the non-execution period of the second control becomes the reference time (6 hours) but execute the third control immediately before executing the first control. Due to this, in space purification system 2100, since drainage by the third control is no longer performed immediately after the hypochlorous acid is supplied to mixing tank 2092 by the first control, the hypochlorous acid water supplied by the first control can be continuously used maximally and waste due to the drainage by the third control can be reduced.

In space purification system 2100 according to the present third exemplary embodiment, the predetermined period is preferably set based on the concentration of the hypochlorous acid water supplied by the first control. For example, in space purification system 2100, when the concentration of the hypochlorous acid water supplied by the first control is high, unless the supply of water by the second control is executed, the increase in the concentration of the hypochlorous acid water in mixing tank 2092 becomes fast. Therefore, by setting the predetermined period to be short, it is possible to more reliably suppress the excessive increase in the concentration of the hypochlorous acid water in mixing tank 2092. On the other hand, in space purification system 2100, when the concentration of the hypochlorous acid water supplied by the first control is low, wasteful drainage of mixed water by the third control can be reduced by setting the predetermined period to be long.

In space purification system 2100 according to the present third exemplary embodiment, the mixed water may be drained when drainage of the mixed water is not executed even once within 24 hours thereafter since drainage of the mixed water in mixing tank 2092 is performed by any drainage control (e.g., the third control). By doing this, the mixed water in mixing tank 2092 is reset, and an excessive increase in the hypochlorous acid concentration in mixing tank 2092 can be suppressed.

### INDUSTRIAL APPLICABILITY

The space purification system according to the present disclosure can easily adjust the amount of hypochlorous acid released into the air when hypochlorous acid water is micronized and the hypochlorous acid is released into the air, and is useful as a system that sterilizes or deodorizes the air in a target space.

### REFERENCE MARKS IN THE DRAWINGS

2: inlet
3: outlet
4: prior stage air passage
5: middle stage air passage
6: subsequent stage air passage
8: air
9: air
10: space purification device
11: air purifier
11a: humidifying motor
11b: humidifying nozzle
13: air blower
14: refrigerant coil
15: air-conditioning device
16: duct
16a: indoor inlet
17: duct
17a: indoor outlet
18: indoor space
20: condenser unit
20a: compressor
20b: expander
20c: outdoor heat exchanger
20d: blower fan
20e: four-way valve
21: refrigerant circuit
24: duct
30: hypochlorous acid water generator
31: electrolytic tank
32: electrodes
33: electromagnetic valve
34: brine tank
35: brine conveyance pump
36: hypochlorous acid water supplier
37: hypochlorous acid water conveyance pump
38: water conveyance pipe
39: water level sensor
41: air purification control unit
41a: inputter
41b: storage
41c: timer
41d: processing unit
41e: outputter
42: air-conditioning control unit
43: operation device
44: temperature and humidity sensor
50: water supplier
51: electromagnetic valve
52: water conveyance pipe
60: drainage
61: electromagnetic valve
62: water conveyance pipe
90: water level sensor
92: mixing tank
100: space purification system
1002: inlet
1003: outlet
1004: prior stage air passage
1005: middle stage air passage
1006: subsequent stage air passage
1008: air
1009: air
1010: space purification device
1011: air purifier
1011a: humidifying motor
1011b: humidifying nozzle
1013: air blower
1014: refrigerant coil
1015: air-conditioning device
1016: duct
1016a: indoor inlet
1017: duct
1017a: indoor outlet
1018: indoor space
1020: condenser unit
1020a: compressor
1020b: expander
1020c: outdoor heat exchanger
1020d: blower fan
1020e: four-way valve
1021: refrigerant circuit
1024: duct
1030: hypochlorous acid water generator
1031: electrolytic tank
1032: electrodes
1033: electromagnetic valve
1034: brine tank
1035: brine conveyance pump
1036: hypochlorous acid water supplier
1037: hypochlorous acid water conveyance pump
1038: water conveyance pipe
1039: water level sensor
1041: air purification control unit
1041a: inputter
1041b: storage
1041c: timer
1041d: processing unit
1041e: outputter
1042: air-conditioning control unit
1043: operation device
1044: temperature and humidity sensor
1050: water supplier
1051: electromagnetic valve
1052: water conveyance pipe
1060: drainage
1061: electromagnetic valve
1062: water conveyance pipe
1090: water level sensor
1092: mixing tank
1100: space purification system
2002: inlet
2003: outlet
2004: prior stage air passage
2005: middle stage air passage
2006: subsequent stage air passage
2008: air
2009: air
2010: space purification device
2011: air purifier
2011a: humidifying motor
2011b: humidifying nozzle
2013: air blower
2014: refrigerant coil
2015: air-conditioning device
2016: duct
2016a: indoor inlet
2017: duct
2017a: indoor outlet
2018: indoor space
2020: condenser unit
2020a: compressor
2020b: expander
2020c: outdoor heat exchanger
2020d: blower fan
2020e: four-way valve
2021: refrigerant circuit
2024: duct
2030: hypochlorous acid water generator
2031: electrolytic tank
2032: electrodes
2033: electromagnetic valve
2034: brine tank
2035: brine conveyance pump
2036: hypochlorous acid water supplier
2037: hypochlorous acid water conveyance pump
2038: water conveyance pipe
2039: water level sensor
2041: air purification control unit
2041a: inputter
2041b: storage
2041c: timer
2041d: processing unit
2041e: outputter
2042: air-conditioning control unit
2043: operation device
2044: temperature and humidity sensor
2050: water supplier
2051: electromagnetic valve
2052: water conveyance pipe
2060: drainage
2061: electromagnetic valve
2062: water conveyance pipe
2090: water level sensor
2092: mixing tank
2100: space purification system

## Claims

1. A space purification system comprising:
a hypochlorous acid water generator that generates hypochlorous acid water;
a hypochlorous acid water supplier that supplies the hypochlorous acid water from the hypochlorous acid water generator to a mixing tank;
a water supplier that supplies water to the mixing tank;
a water level sensor for detecting a water level of the mixing tank;
a humidifying purifier that micronizes and releases, into air, mixed water of the hypochlorous acid water and the water stored in the mixing tank; and
a control unit that controls supply treatment in the hypochlorous acid water supplier and the water supplier and drainage treatment of the mixed water stored in the mixing tank,
wherein the control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank based on an integrated humidification amount in the humidifying purifier.

2. The space purification system according to Claim 1, wherein the control unit executes the third control when the integrated humidification amount becomes greater than or equal to a reference amount.

3. The space purification system according to Claim 2, wherein the integrated humidification amount is calculated based on a number of times of execution of the first control and the second control.

4. The space purification system according to any one of Claims 1 to 3, wherein the control unit executes the third control when a number of times of performing the first control becomes a reference number of times.

5. The space purification system according to any one of Claims 1 to 4, wherein the control unit executes the third control immediately before executing the first control or the second control.

6. The space purification system according to any one of Claims 1 to 5, wherein in the control of the supply treatment, the control unit (i) performs the first control and the second control to cause a number of times of performing the first control to become smaller than a number of times of performing the second control when a humidification requirement amount required for the humidifying purifier is greater than or equal to a first reference value, and (ii) performs the first control and the second control to cause the number of times of performing the first control to become larger than the number of times of performing the second control when the humidification requirement amount is less than the first reference value.

7. A space purification system comprising:
a hypochlorous acid water generator that generates hypochlorous acid water;
a hypochlorous acid water supplier that supplies the hypochlorous acid water from the hypochlorous acid water generator to a mixing tank;
a water supplier that supplies water to the mixing tank;
a water level sensor for detecting a water level of the mixing tank;
a humidifying purifier that micronizes and releases, into air, mixed water of the hypochlorous acid water and the water stored in the mixing tank; and
a control unit that controls supply treatment in the hypochlorous acid water supplier and the water supplier and drainage treatment of the mixed water stored in the mixing tank,
wherein the control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank when the first control is continuously executed a predetermined number of times.

8. The space purification system according to Claim 7, wherein the control unit executes the third control immediately before executing the first control after continuously executing the first control the predetermined number of times.

9. The space purification system according to Claim 7 or 8, wherein the predetermined number of times in the third control is set based on a concentration of the hypochlorous acid water supplied by the first control.

10. A space purification system comprising:
a hypochlorous acid water generator that generates hypochlorous acid water;
a hypochlorous acid water supplier that supplies the hypochlorous acid water from the hypochlorous acid water generator to a mixing tank;
a water supplier that supplies water to the mixing tank;
a water level sensor for detecting a water level of the mixing tank;
a humidifying purifier that micronizes and releases, into air, mixed water of the hypochlorous acid water and the water stored in the mixing tank; and
a control unit that controls supply treatment in the hypochlorous acid water supplier and the water supplier and drainage treatment of the mixed water stored in the mixing tank,
wherein the control unit (i) executes, as the control of the supply treatment, first control of causing the hypochlorous acid water supplier to perform supply of the hypochlorous acid water at predetermined time intervals, and second control of causing the water supplier to perform supply of the water based on information regarding the water level of the mixing tank from the water level sensor, and (ii) executes, as the control of the drainage treatment, third control of causing the mixing tank to drain the mixed water stored in the mixing tank when the second control is not executed for a predetermined period since the supply of the water by the water supplier is performed.

11. The space purification system according to Claim 10, wherein the control unit executes the third control immediately before executing the first control.

12. The space purification system according to Claim 10 or 11, wherein the predetermined period is set based on a concentration of the hypochlorous acid water supplied by the first control.
